# EUROPEAN PATENT APPLICATION

(11) **EP 2 463 382 A1**
(43) Date of publication of application: **13.06.2012**
(21) Application number: 11009657.5
(22) Date of filing: 07.12.2011
(51) Int. Cl.: C12Q 1/68

(54) **Method for identifying E. Coli M-17**

(30) Priority: 07.12.2010 US 420344 P
(71) Applicant: Enterologics, Inc., Saint Paul, MN 55104 (US)
(72) Inventor: Hoerr, Robert A., Shoreview, Minnesota 55126 (US); Bostwick, Eileen F., Dayton, Minnesota 55327 (US); Testa, Douglas R., Lebanon, New Jersey 08833 (US)
(74) Representative: Schmidt, Karsten

(57) **Abstract**

A method of identifying an M17 strain of *E*. *coli* in a human biological sample is provided. The method comprises analyzing products of an amplification reaction using DNA extracted from the human biological sample and a primer pair which amplifies an M17 specific nucleic acid sequence of an M17 nucleic acid sequence, wherein the primer pair is selected from the group consisting of SEQ ID NOs: 37 and 38; SEQ ID NO: 39 and 40; and SEQ ID NOs: 45 and 46, wherein a product of the amplification reaction is indicative of an M17 strain of *E.coli.* Additional primers and kits comprising same are also provided.

SEQUENCE LISTING

<110>  BioBalance LLC
       Hoerr, Robert A.
       Bostwick, Eileen F.
       Testa, Douglas R.

<120>  METHOD FOR IDENTIFYING E. COLI M-17

<130>  49419

<160>  647

<170>  PatentIn version 3.5

<210>  1
<211>  1181
<212>  DNA
<213>  Escherichia coli strain M17

<400>  1
cccccagctc atcgtgtttt ccgttgacga cggttgaaat acgcttatcc gggtctgatt      60

aatgaatcag gctgattaac cctatcggtc aggctctctc aaatgaacat aaatatgttg     120

ctatactcat tctgacttaa gcggctgtga tatattcagt ataaaataag tcacttataa     180

cagttcgata aataaaattc gcagcttccg ggttgataac catcatatat actatggcaa     240

ggatattaat atggcgacac ttgagcaact tatcattttc tttcttctta ttggcgttgg     300

tgtcattgct cgtaaagtgg gagttattac tccaggaaac acgccccaac tcacatctct     360

tgtttttaac tttgcgatgc cagccattat actttccggt attacaactg aacagccaca     420

tatttccgga aaagatcttt ccatcgtact tacatccgca ttcactacgc ttattttact     480

gattatttgc agtcgtatgc tggcaagaat tttacgctat gaaagagaat actatggtgt     540

gattactgtt atgacaacat ttactaatgt ttccatgatg gggatcccaa tgatatattc     600

tttatacggc tccgaagcca tgatttatat cactgtcttt ttactcccat ataacttatt     660

gttcttttca tatggttatt actgcatgaa ggaccagagt ggtaatactg aaagtcttaa     720

caccatttat cttcccggaa aatgagatgc ctgcaccggt gaagacgaca tttgccccct     780

tgagtgcttc cgcaatgggt gttcccatga atgaatttct caaactcacg cgaatcccaa     840

tcgttatcta ttatggcgat tttattgctg aaaaaccgga tactgccgtt ggtccggata     900

aatggagaag tgaatatgaa atggcaaagc aatttgtcat gacagtcaat cgtcacggtg     960

gagacgccac ccttgtccat cttcctgata ttggtatcaa aggcaattct catttcctga    1020

tggcggaaaa gaataatcag gagattgccg ggatactggc atcgtggtta caagataaag    1080

gactcgataa ataactcagc gccctaaaag cattgatgga gaggaatgaa tattatgcgc    1140

cttttactta tagaagatga agagaaaacc tcgacgtata t                        1181

<210>  2
<211>  170
<212>  DNA
<213>  Escherichia coli strain M17

<400>  2

gttttttctg atggagattc agagcctcga agatgtttgc tctgttcact gctgtatcaa 60

gctttcaacg ttactgaagc gctggccttc gccgtattaa aacgctcact ggcctacagg 120

cgtgcggtga aaaaggtcat tatgcccgcg caaggtccac agcaaaatgt 170

<210> 3
<211> 1681
<212> DNA
<213> Escherichia coli strain M17

<400> 3
gtagtatgta aagaattacc tttggttgat ttgtaatagc taccgattac gttatcggta 60

gctataagaa atttttatgt ctttactgtg tttatttatt caatactagt tggactatag 120

caatgaaaga taacagacaa aaagtcgttc attataaaaa agcagtaatc ccacacagca 180

aatcatcatt acaaaacata ctacttagta ttataggtga gggcggcgtt gcagaaaaag 240

tacttagtag acaagaaaaa ataacacctt cagatgaaaa ttcggggtat cgtttcttaa 300

ataaaagcga cacatacaaa actatacttt ttgggcaact ggttttattt gaaaaagaca 360

aaagccagtc tctattagag cttagtgata atgttaaatt ttatgacatt aactccatca 420

cgtctaatga tataattatt gatggagata aacgtagtga caaaaaaaga gagtttgtag 480

attcaatttt atattttggt gtactaaaca accatgtcat gatcgttcaa tccacatcgc 540

ttcgagcaag ggagcttgag gcgcatttga attggctaat taatacattc acagatgaaa 600

aagaatctgt gttaatgctt caagataagc catccgaaga aacgatgaaa aaaatggaga 660

aagcaccagc caagagcata aaaattggtg gattacccat tacttcagaa gttaaaaatt 720

cagagaacca aacaccctcc ttttcaaatg gttcggtgaa gacaataaaa taccgcccaa 780

tagggaaagg aggtgcgcta ctaaaagcat tttttggcga taactgggca aatgatataa 840

acattaaaga ttcattagat gaggccaatt tacaagtaag cctcgagatt acatacttca 900

gaaaaacaaa tgatagcggt caagctgtta tagacagtat tgctacctca ctacgacatc 960

tagatgagga tgatattagc atcaaacttc aaggcggtgg ggaaataacg ggaaaagaga 1020

taaaattatc cggaaaaatt agcgttgaat ataacaatgg cattattgat gaaaatgatt 1080

tattcttaaa aatgcataag tggctgttct caaaacttga tgccggagaa ttggttgcaa 1140

aataacctac caaaaggagg gcgataatga aatcgaacat catccacctt attggtatgt 1200

tggtcttcta ttttgctggt tactttttgt tcaagtcagc attgggctat attacaccgg 1260

gtgttgtcct aactgctccg tgggctgtgg ttggactcat gcaattgcca ttatcatatt 1320

gtatacaagc catttttaag gcaaatgagg ccaatgacca ctcgtcttta acccctagtg 1380

aggttaggcg attagtgcca atagtcaaat ccaagagact gaaattggtt ttattattag 1440

ctttctatgt attttcaact ttgtttgtaa tcttggggtt aatagcaagc acaaatgatc 1500

aagccctact cttcagggtc ttaaaaattt caggtggatt actctttatt tctctctata 1560

ccagtgtatt cgttcataaa ataatggttg agcttcaaga gtttaaagcg ttattaaacc 1620

gaagagaatc agagaagaag gaaagagagt cactccttga tagattaaaa tgacccccct 1680

c                                                                 1681

<210>  4
<211>  189
<212>  DNA
<213>  Escherichia coli strain M17

<400>  4
taacgtaggt aaaggtctgg atggtagtgc acgtgtcaca ctgaaaaact ccagctggga      60

tggtgatgtc aataacgcca aaactgctgt ttttctgacg gatggttctg tgtggaatgg     120

tgctgtttcc attaacgcgc cagtggaccg cgcatcagta ccaatgggct cggttcaacc     180

aaataaaag                                                             189

<210>  5
<211>  416
<212>  DNA
<213>  Escherichia coli strain M17

<400>  5
tattcttttg cttcatcatc gccattaatc agatacaccg gatatttagg aatgacatcc      60

acaagaatct gaatggctct ctccgtgacg ctgccgttaa tatcgcgcgt ccatccttta     120

taatcgctct gaaattctcg ggctgcctct cgcaactggt actgagcgtt cgtcgggtca     180

aggataggta tgccctgatt actttcatca agtgtgccat tgcctttctg cttgttcacc     240

cgctcttttt cgatattttc catttgttct tcgcgcgctt gggcctcttt atcaagtcca     300

tctttatcca gttgtggcgc cttaatataa aaaggttgta caagaagact gttattcgca     360

tagcgaccga tacgccaggc ggtgatccag ccgatctggt cactaattgc gctttc         416

<210>  6
<211>  555
<212>  DNA
<213>  Escherichia coli strain M17

<400>  6
gatgggtatt agctcatatc agaactaata cccatcaatg acacagagcc aactacccac      60

cctgtgtcag gggctaactt ttaacaccat aattcaccgc tagataaaaa ctgtattgca     120

ttgctcgagc ggcgcagcat tatcagttag agtgtcttta ccttgttagg ctccacagtt     180

cgcactgtcc aacgcctgtc actttcacac gctgcgcctt cgagaatatt catttaatcg     240

ctgataacga acatcttgct ccagttgtgg agcagcttga acaatttgaa gcaagcattg     300

ccccaactct cgaggccccc agtgttgttc ggcaatcacg gcattcagca gggatagagc     360

gtccgcagga tatcggctgc aaatatctga ttttagcaaa aggcgaacgt cgtagctaag     420

gtgttcgagc ggttgcagcc agtcctgcac cactgccaaa gccgccggaa attcacctcg     480

ggcagcaatc accatacgag tcaacgattc ggatatgcgt ggggtggcca agttgcggga     540

ctttggccaa acctg                                                      555

<210>  7

<211>  1357
<212>  DNA
<213>  Escherichia coli strain M17

<400>  7
aagtagcgaa ttgctgccgg tgctcgccaa gatcagaata gtgattggcg ctctccaaaa      60

aatctgactt gaaagctatc agcaacggtt catacaggcg tggcgaccag aggaagcctt     120

cccacacagc ttttgcttcg acgggattac tccaagcaaa caagggcaat agatactgtt     180

cggtccaagg tcgatctacg cgaaaaaatg cgatcagccg cgaccccagc aacacccgac     240

catggcggaa tagctctatc tgtacattac acaatttggt gaaaagtgtt ttcaattcaa     300

caggaagcaa atcattgtca ttcgggttct gtttgaacca tagggtgatc agtgattgcg     360

tgacatgccc aatgggatga ttgatcgccg tagaaacagg atcataggtc tcaattccgt     420

ttcgaatggt gctagactct gggcttgttt ctatcatcag aacccgacga caaagggcca     480

gtagaatctc ctcgtggcag aggatggtct tcgaagcctc ctccatccac caagtgacag     540

cgtgggaaat ctcctgaagt actgcgtcag gcatgtcaag caccaacggt gcggcgtacc     600

gccacgaacg caaaatcatc cctggttcag cccaagtctg cagagcttca cgccaccgac     660

caacaggcca cacatcatct tgtgatagtt tacgtaacgc atacagactg tgaaaaaagc     720

gcgtacggca aacatcactc caagtgtcct catagaaagg cagtctttct ggcataggct     780

ttgcgagcca ttgcactaat tcctgccact tacggggcgc aatgtcgaca tctatactct     840

cctcgaagcc tggatcaccg gttccgctca tccagtgaga gaattcatca cgctcgttgg     900

ttgccagttg ccattttggg tatgctgtgg atatttccgt caaacgtgta gccgcagact     960

ctcccaaaac aaggcccgct cccctgagct tcgctagaca caaccagacg gaatgagcca    1020

ccaaataatg ccacctgtct gcttccaaat tatcctcgta catctcgcgc ggaggccctg    1080

ccaagatagc agtttccaga cgctcttgtg caattcctgt cagatgtcgt ccctgcaaaa    1140

caaacagtct gaatacctct cgcccagtat ccgtggccca caaccaccat gaaccgtcct    1200

ctaacaacca attaacccac cgctcaggtg gtatgcagtt gtcttggctt gcggcaaaca    1260

gtgccagacg tttgaaggtg ggatatggca actcaaacca attctgagca atgcgcgtgg    1320

cctgatcgct gtctttggct cgaacggcta accatga                             1357

<210>  8
<211>  795
<212>  DNA
<213>  Escherichia coli strain M17

<400>  8
atcttccaac aggtatggca aggacgattt ccatgactcg tcagcaaggt cgaacagggt      60

tgaacgtacg taatcagcag tcagcaccag ctcccaatcc accaattgct tgattcgcaa     120

gggttcatca gtgctgctcg aatcgtcttc actatagcga aacggccgcc tcaacataac     180

cttgggagaa agcaactcgc gtaactccaa gcgcaatgta gtcgtcaagc cttcattctt     240

taagcggttt tgccaacgat acaaatccag gttctgcaat ggcgatttca cacgaccact     300

aagcagaagc cgccataaag tagacatagg tggaccagga atagccaggg gggaatgcaa 360

gagaatttca tctaactcag aagtcttacg ctcccgcatc agtgctgcta agcgatctag 420

ttcgctctca atcagaaaca tccaacggtc gtgtatttgt ccgccgcgtt cagcaatcca 480

tatgatcaac ctagggtcgc ccagataacg aactagccaa cgggctatat gggacattac 540

gtcatcccat ttgctagcac tgacacatcc agaaaccagc gacatctgcg gggccagctc 600

atagggcgca ggacgctgaa ccagactgaa tcggagtttc gggtcaattt cgacatgcgg 660

agatacacaa aagcgtggca gatcgctgta tttaaatcgt tcgtccgaga aagctttcaa 720

taaccaatcc agcggcggag caggattgag ttccgcaaag cgttttgctg gtaaacctga 780

tttatctgac aaggc 795

<210> 9
<211> 337
<212> DNA
<213> Escherichia coli strain M17

<400> 9
tacataggcc tgaaaagcct ggcctgtacg tttagctgcc acatgaaaga gacggtcaaa 60

gttggtagtg actaatcgaa gggcaccctc gcggctacgg gctaaacgta acagcgccgc 120

ctgagtatca atagcgcccc tacgacggag ctttggctta agggcttttt ccaacgcgcg 180

tcggacggct atacgctgcc ctggtaagcg ccgttccagc aaatctaatg tgccgtcaaa 240

ttgcccacgc tcgaaaacct cacgctcaat ttctgaaagt gttgttccgt tcctctggta 300

aattagttct accaaccctt tgaaaccagg taaacca 337

<210> 10
<211> 526
<212> DNA
<213> Escherichia coli strain M17

<400> 10
ctttgatttt cagttgttag aaaatgcagg gatccagcta attgatggtg acagaggcaa 60

aaattatcct aaacataatg attttatgga gaatggctat tgtcttttct tatccgctaa 120

aaatgtcact aaatctggtt ttcaatttca agaaacatta tttatcaatg aaactaaaga 180

tcgtgaacta agagcaggaa agcttaagta cggtgacatt gttcttacta cccgtggcac 240

agtaggtaat gttgcgtact atgacaataa taatccatat aaacatatta gaatcaactc 300

aggaatgatt atcattcgag ctgataataa attatggaac cctaaatttc tatatttcat 360

attaaaatca gaactattaa aagaacagat cattaatctt atctctggct ctgcagtgcc 420

tcaactaccc gcgagagata ttaggaaatt tatattacca gttattaata gaagccttca 480

aaataaaatt actaatatta tatcagatat taatgataag gttaat 526

<210> 11
<211> 464
<212> DNA
<213> Escherichia coli strain M17

<400>  11
ggaggctggg aaacaaaccg actggaaaat attttagagc tggcttatgg gaaagcactc      60

aaaaaaacag aaagaatcga aggagattat cccgtttatg gttctggtgg ggtggatggt     120

tcccataatg agtttcttgt taaaggacct ggtattattg ttggaaggaa aggaactgtt     180

ggttcattgt attgggagaa caaagatttt tacccaattg atacagtatt ttatgtaaaa     240

cctaaaaagt acttctcgct tgtttactgc tatcagctct tgaaaacact aggattagaa     300

aatatgaata ctgatgcggc agtccctggg ttgaacagaa acaatgctta tcgtcttgac     360

gtcataacac caacacaaac aatcattgcc caatatacaa atatagtaca aacgttcagg     420

tataaaatgg attcaaataa taatgagatt gacaatctaa ccaa                      464

<210>  12
<211>  479
<212>  DNA
<213>  Escherichia coli strain M17

<400>  12
aaatgagcga gttgagttat ctggaaaagc tactcaaggg gtgtgatgtc gaatggattt      60

ctctggggaa tattggcaaa ttcatccgtg gcaatgggct acagaaaaaa gatttagttg     120

atagtggttt tccagcaata cattatggcc agatttatac aagatatggg ttatcggctg     180

atagaacatt taattacgta tcgccagagc tagccaataa gctaagaaag gcacaaaaga     240

atgacttact gctagcgaca acatcagaaa acgatgagga tgtcgtaaaa cctttagctt     300

ggctcggtga taaagttgct atttccggag atatgatgtt atttcgccat gagcaaaacg     360

ctaaatatct cgcccacttt ttccagtcaa aaatatttca agcgcaaaaa atgaaatata     420

ttactggggc taaagtccgt cgggtttcca gtggcgatct agctaaaatt actattcca      479

<210>  13
<211>  396
<212>  DNA
<213>  Escherichia coli strain M17

<400>  13
caatactcga aaacaagaat tagtttcgaa ctacttgatg aaaagaatta tgttggcgta      60

gataatctgc tgcaaaacag agcaggaaaa tcattatcta atcatgtacc gacatcaggt     120

aaattaacgg agtttatacc taacgatata ttgattggga atatccgccc atatttgaaa     180

aaaatttggc aagcagattg tatcggtgga acaaatggcg atgtattggt tattcgttgc     240

atagatagct caataaatac cagatatcta tatcagattt tggcagatga caaattcttt     300

gaatacaaca tgcagcatgc taaaggagca aaaatgccgc gtggcagtaa agaggatata     360

atgaaatacc caatccctgt accgccactc acctgg                               396

<210>  14
<211>  673
<212>  DNA
<213>  Escherichia coli strain M17

<400>  14

aacgcctctt atagggcaag ctgcatcaaa taaattaagc taccttggta ttgggaaaaa 60

gatatccttt gatggtgatt tttatactgt agatggaatg aaattcagca aaagttatta 120

tgagaaatta tgggagcaag gcagacctgc gccattcgtg caagcaagag aagttttgaa 180

tagcaatcca aaaattgagc cggatcctag aggcgcacct ggctacttaa gatatgaagg 240

tgcaggtctt gaaatgatct ataacccgaa aacgggacaa gttggtcata ttcaaccagt 300

caaggttaaa taaaaagagg aagtaaatgg atatttggcc tgaatttcaa cgagatcttg 360

agatgtatcg agatgttgtt ttatcaataa aacgtaacct tcggttgtat gaggagtgta 420

ttgaatcgtt agttcatcag atcggaagca ccaattttga taatgcgcag cctctatttg 480

atgacctctt tcgtatgcaa tctgaacttg ctactatgtt atacaaatat gaatataagc 540

caggcaaaag gatacaagat ttaatatatc atctggacag ggatgatttt tactcaagaa 600

aatattggca taaaaaattc agtgatggat tggcatggcc tgaataatag aagtagaaat 660

cccgccctca gtt 673

<210> 15
<211> 806
<212> DNA
<213> Escherichia coli strain M17

<400> 15
   tggaataccg ggggggatgg ggaccggata atgaagaaaa agcccgtcat caggaggttc 60
   agcaacaacg atttgatgaa ctgagtaaaa tatatgataa aagtcacccg gccggagagt 120
   taacggtgga tgggcagact atcaggcaga gttcggtaag taaccgttat ggtacgacga 180
   aagtgtttga aagccagact ttgacggata aacagatcca caattacgca cagcaactgg 240
   caggcgatac accgctgaaa gaggtaaaat cgggaatcta cacgtctaaa ctatcagatg 300
   gttcggtaat aacacttaga aacatatcta catcagaagg tcagacaggc gctcgttgga 360
   ctatagatat tcgtaataac caaaagctta ctgaactggg gaataaatat tcacgtgtgg 420
   agattaagtt caaatgatta ctgagttaag tgaaagagaa tacaaggagt tgacagacgc 480
   tgcagaggga cagactcttg atgctatttt gtgttattca gttcctgata ttcaccctgc 540
   agagtttagt tttgcagata gaagagaaat ctttttttgg gcattaaccc gtcttctgaa 600
   agaagggcgt atcaaactcg ccaagcacgg taagtttttg gaaggaagtg ttgacgagca 660
   ggttgagcgt tttcgacagg catttccgaa aacagaagaa gagatggaag acggtatctg 720
   gttttttgat gagagttgtc cggggggggc tgtttgggtg cttgaagatg gctctctcga 780
   atggacctga taaccccttc acagcg 806

<210> 16
<211> 348
<212> DNA
<213> Escherichia coli strain M17

<400> 16
aacagggggc tataacgtac ctggaggcat aaacccatgc tgttcaagta tacctctggc 60

aacaggtgaa actaaaaagt cggcaagtgg cagggcctta tcggtcagac aggcaaatcc 120

atagatagca atcggattat aaggttccgg tatttctata acttttactg aatcaattga 180

ttgcaagcca ggaacataat tggcataacc gatgaacaga tcagtataat cattttcaat 240

taaccactgc gctgctatcg catttcctgg aagaggcaac gaatgcctcc ccccaactaa 300

tgctacagcc cgttgtctta ttttttcacc gacactgccc atattttc 348

<210> 17
<211> 286
<212> DNA
<213> Escherichia coli strain M17

<400> 17
tgttccgatt cgcaatgttt cccgatttaa taaagaatac cagtcgtcat tctcattgaa 60

tttattcgcc cgaacataca aacacaaacg attgctggca aaaggtttaa tacacaatgc 120

tcgtcctgcg gacattaata tctgtgggtg agtcatattg gctgatgcaa aaaaatcgca 180

tgcctcaccc gcctcaatac gctcccttaa tatacccgct ggtccaaaat cacactgtat 240

attatcggcc tgatactctg acacaagtga tttccatacc cggcgt 286

<210> 18
<211> 506
<212> DNA
<213> Escherichia coli strain M17

<400> 18
ggtgaaagtt cgttcagctt gcgtagtaaa tgataaagaa agcaaaaaaa tcagtaataa 60

aaacaactta ttatttattt tcatattcca tcacactaca aatcggtata taaataacta 120

tatatcgaaa tggtagcacc agtcaggctg tgggaaaagc gattccggta tatttttgat 180

gtgaccgtat gcagagaatg tgacgtttat gtgttcagca gaggttttca attccgttat 240

ctgtaaccct gaatacgcat gatcataata gcaaagacaa aggagtcgtt acgttgccgc 300

accctatcca ttcaccatcg cctgagacga taaaatatca gtaccatcag cttgtacata 360

aacggattat actgatttca ataataacgc tggcgatgct ctcctcacta tttctggaca 420

tcatactggg ctcttccaat ttgccagtca gtgagctatg gcatgcttta gctcatccgt 480

ttagtcgtga taccagcgtc catgtc 506

<210> 19
<211> 163
<212> DNA
<213> Escherichia coli strain M17

<400> 19
attttatcag gcgtatttat cattgttttt ttatggtcat tttcccgctc atggcaactt 60

acggcattac gcatgggaga agaaagagcg ttaagttttg gtatcaatgc acagcaactg 120

cgtctttcct ccctgttacg tataagtttg cttgcagcat tat 163

<210> 20
<211> 402

<212>  DNA
<213>  Escherichia coli strain M17

<400>  20
attagtatta ttctgcgcta tcgcaggagt ttgttatgaa aggtctttca ctgcataaat      60

taaatgcagg ttatcagaaa aaaaacgtaa tatgtgatct cagtttacct gaactgcaac     120

gaggaaaaat aacaatcatt ctgggaccaa atggttgcgg taaatcgaca ttgttacgtg     180

cactggcggg actgaataag gccaacggta aggtatttct tgatggtgtc aacctaatgc     240

aaatgccatt ttctgcgcgt gccgacagta caatattttt gccacagttt ttaccacatg     300

atgtgcatct gaatgtgttg gaatctattc tggtttccct acgcgcgtca aagatgggcg     360

ggaaacatga aaaaacacaa tcaatagcta ttctcaaaca ac                        402

<210>  21
<211>  1092
<212>  DNA
<213>  Escherichia coli strain M17

<400>  21
tttagtaaaa agtgagacac gtaatcgcaa tatagtcacc attatggtag ttcacgactt      60

aaatattgca ctacgatatg gtgattatgt cgttatgtta aaaaaaggca aacttattgc     120

cagtggaaaa cccaaagagg tcattacgcc agagaatctg gctaatgttt atcgcatccg     180

tggacgaata gagcattgtt ctcatggctt gttacaactt atcgttgatg gtgtcattcc     240

ggattaacaa aatggatgga agatttctct cccggaactt ctcatcactt tttgacacca     300

tcatcaacga tgattaacta aaacagtgtt ccctttgatg atgagtttgt ttatatgcag     360

catgaggatt agctcgtgct ttatatcaga actcacctca tataaaaacg agtaattttc     420

aacttttcaa ggtccaatgc aaggtcaatt tacaatgaat gccctgttgg cctaaaatac     480

acccatcaaa tactattttt gatcacttgt ataacggttt cgatacgctc ttttatttcc     540

tctagttgct gctgacgacc ttcaggtgtt aatgtctgat cttcgttcag atccaaaacc     600

catcgacgtg gaatcaatgc accaaaaaca atatccatta acatccccac acaattcccc     660

gtatttttaa cagtaatgcg tccttgagct tgctgaatat ccaaccactc aattaacagt     720

tctcttgagc gaagtgtttt agttgtatat aaatagtctg aaagatctgg aaagagaata     780

gactcacgcg tcattaattt taataacgct tctctctctt tagcgtcttc ctgacttata     840

tctaatcgga atatgcgaaa cagtgcttca ttaagaggga gctcttcatt ttccggtctc     900

ggaagatcga gaaacagtgc tttatattcc ttaataactt cagagaaaat ttctttttta     960

ttgctgaaca cttcataaat cgttcttttt gatacttttg cccggcgggc aatttctgtg    1020

gttgaggttt gagcaaaacc aagctcaatg aaggcactgt acgccccaat aactattgct    1080

tttttacgct ca                                                        1092

<210>  22
<211>  561
<212>  DNA
<213>  Escherichia coli strain M17

<400> 22
ccttacactt gtataactgg tgattcttct tgagagtatc gactggtggc atctggagat 60
acgctttcgt tggcaggtta tgacgcaact taagtgggag aaaggcagtc atgctgcagc 120
attgacgatt tgttatcact gaatacttgg ggttgtacta tagtgcttag acgatattac 180
agattaattt acggacataa atatctcaaa atgacaattt aataaaactc aggtaaaaaa 240
tgcattgcgg ttttaatctt ttttgatata taagcacatg cgttataaat ataaaagatt 300
acactttgtt attgaaataa attggcttgg ctattcaata tgttaatttt cattataggg 360
atgtttaaaa ataaatgtat tctgagtagt catgtaatgt atgtgagatg attttaatga 420
atgcaaatgt gtaaataata agaaaatgct ttttgatgat ggaaacatca caaaacacaa 480
ctagttaaat tgctacattc cccgtttggg tgataatgtc tgatttttat tagcatcttc 540
aacttattaa gttgatctgt g 561

<210> 23
<211> 279
<212> DNA
<213> Escherichia coli strain M17

<400> 23
ggttatccgt caggaagagg aaaaaatgtt ctgcctattc tctgctaaat ttattccaca 60
ggttcctagt ggaacgccgt ttacatgaaa aggttgcttg acgctctgaa gcgtaatgtc 120
ttgaaaatta atgttcaact attatttcat ttatcactgt taggttggga atgaatcaaa 180
ctgacaggga attatatgga aaatcaaccc aataccggct tcggataagt atcggttttt 240
aataccagcg gaaataggta aggtcaaaaa acagcctta 279

<210> 24
<211> 1192
<212> DNA
<213> Escherichia coli strain M17

<400> 24
gccttgctta gacctgtgtc caaattacgt gggttggatc attgttccac gacgatttga 60
aagtatatta atgaacaata aatacacatt tctgtatttt cgtatatttt acaatagtga 120
atatacaaaa tctaatatat tctatatttt tttgttctaa ccttttgaaa tgaagtgact 180
ttttattctg tcttatgggt gtttttttct gctgcaagca cttccagtat tatttccgaa 240
tttacattat aaacgcctca tgaaattata ctttcatagg aagtggaggt ggttatatga 300
aaagtcaaag tgctttatat aaactaaacc tttctgataa acgggccatt ttaaatataa 360
atagattaga aatcagggat gttcactttt ttgaatgtac aattatttat ctctatgatg 420
cacgaattgt attaaaaaat agcaatggta aaaagacaat tattttccct cagtcaattt 480
gttatattga aaaaaataca actgtagata taattatatg tgataaaaaa agggaaggcc 540
ttatgatgtc tataatgtag atggtgatat actcaataat atttgtgaga taatgagtcc 600
tcttctttcc cattttggaa atgtaaatta tccatatgct aaggtgttct catttacagc 660

agatgatgtt gataaaaaaa tctttcatct tttaatgaaa aaggaaacgc catattacag 720

gaagttatat aaacttacat accttttatc aaagtgtgat aatattgaga cattaattta 780

ttcattatca aaatcaaaaa ataaaacaat tactgaaaga ctaaaggata taattgagtc 840

agacctatct aaaacatggc aaataagtga ttttgcaaaa attctacata tgtcagagtc 900

attaattaga aaaaggctca aaggtgaaaa tataaactat aataccctga ttgttgatat 960

tagaatgaac tatgcattta acatgttaat ggcaacggaa aaaaacataa atataatatc 1020

aagagaggtt ggttatgtca gtacatcata ttttattagt aaattcagaa attacttcgg 1080

aattacacca aaacagtttt caataaaagt aaagaataaa ataaggagtt agtttctata 1140

agacatgcac ttaaatgtat ccattaataa aaatggtaat atgttcaatc ag 1192

<210> 25
<211> 777
<212> DNA
<213> Escherichia coli strain M17

<400> 25
acgtggttta aaagttatca tgaagaaaat ccctgattag aaaggtgctc ttgaacatca 60

tgaatgtaaa agatttatca attacaacgg ttctcttggc cgccctttat tttatggctt 120

tttcttttta caaaggatac agctcatttt atggattccc tgtaagtttt atttctattg 180

ggattgagga aatagttaag ttttctgtta ttgcattagg acttttgtgc accttagtcg 240

ctttgctgca tattgatact gaagagaaga atattccatg gggggtgtat ttatttttct 300

tcattttggc ctctttgctt tcatactgga caatgtattt atatggcggt agctcatacc 360

tttatgaaaa atccataagg gatgtggcta cccaagcaat actattggga tttattagcc 420

ctatcagtgt cagggcagta tctgttttta ttaggaatga atttagaata aaaaacaaaa 480

ctcatggggt tatgtttttt atatccatag caatgcttcc ttcagtcctt gggtgggcat 540

gggcatactt atcagatgag cctctttttt attctaaaaa atatgatgct tatattatcg 600

aaagctataa cggaaaattt gtcttgggga actgtaaaaa aaaatagctc tgaatttatg 660

agtgtcgaag gaattgaggg gaaattaatt cctgtaagca taaaagaaac acaacaacta 720

aaagtatgtt ttttaaaagc ttctaagctc aagaaaaatc gctcagaatg attgtct 777

<210> 26
<211> 1437
<212> DNA
<213> Escherichia coli strain M17

<400> 26
aaaatatcct catatggtat atggttaaat gctagatatc atgaattaaa ggagacaact 60

aatgactgtg ggtaataaga gacagcctaa tggattacct gaatattatt gtcaccgtgg 120

taacgacata aaagatgcga tagttcggga ttttattatt cagcctgttg cacacctgaa 180

acttattact ggacaaacta aaaccagtta ttccggttat ccgttaaccg atcagtacta 240

ttgtttttca tttaaaaaac gctctgatga aaattgtaca ggggcttttt tctgtggtag 300

agatgttgca aaggatttca ttcggcgtat cggttgtaca gagctgccgt tatttaatcc 360

tatgaaatcg gataccagtg cagcaataga acgttccggt tctggctcag gtagttctgg 420

tattactgtc aagccaaatg ctgacaaaaa gctatttata gacgcagctc gtctgttaat 480

gacgctgtgg gattcaacaa gtaagggaca aaccccactg gcgtcggttc ttaatgaagc 540

aatttctggt ggtatagata aacctccaga aattagacat gtagtggcgg ttaatacaat 600

gttgggtaac acaaaaaata ctttaactgg tttgctggat aaagcaagag cgaagtatcc 660

tgatgtgtta ttcagagatt ttgatttcac gcctcttaac gagattgtaa aacgttcaaa 720

atcatataat aagcatcccc cttgttattg agtatcagac actgtattgg taggaaggcc 780

tatcatttca tttaatgtct ctcgatgtac tgctggtgct aaagggactg gcggtgcgtc 840

tgttggaagc agttctttag cttcgggcca tacatccagt aatcgtttta ctgtccgaac 900

tttatttaga gccgcgctga cgttctgtat gatttcggtt tcatcacttt taatttgtgt 960

gtagagagca tcaaagctat aaaactccgt taccagagga tcttcgccaa gtagagtaaa 1020

ttctgcaggt gctattttat taatatgctc gggggctcct gattcataac ctctgtagtt 1080

gccattgaaa taaatgtttg ctctggctcc agccagattg agatatatgc agcaatcttt 1140

tcgaattaaa gaactattgg tttttaatga atcagggatt tttgttacca tggattcaat 1200

cttttttata ttttttaata tttcagcttc tttctctacg cccccgatag ctgcaagccg 1260

aacacgttcg gcccattcaa cacgagcagc tcgtagtgct gatttacgtt tggggatacc 1320

cgctttctca agtgcgttat tgatgatttg agttttaagg tgattagtta atacgggctg 1380

tgacattatt ctttccctca tgagataccc cagcacgtag ctgggggatg aatattt 1437

<210> 27
<211> 418
<212> DNA
<213> Escherichia coli strain M17

<400> 27
tggctttggt gtctgaagct gtgtttggca agcagggcat tcacctttga gcggtttata 60

cgttttcacg ccgtttcctc attcagttca ctttggtggt tcggcagctt cgacttgtat 120

cagtagacag tgcttcgccg caccccaaag ggaacttact gacccgtatt gccgacatcc 180

tgtcccgcca cggtcccgac gcatggttta gagtcgcgcc gttcgacttg tggctaaaga 240

tacaatttga gttgtattgt gtaaaccaca aatgtggtat tttgtggggt gtgaatacta 300

ctttattgat atttaagtga atttaatttg taagacttta caaatggggt aagggaggct 360

gttacgcctc ccgatattta cggtgggaat actaacgttt acggcggtaa atacggtg 418

<210> 28
<211> 719
<212> DNA
<213> Escherichia coli strain M17

<400> 28
tgtctccttc tttaaactct ggttgcatgc tgtcgccatc gatacgtaat gcgaagcatg 60

tttctggaat gtcagagtct gccaaaatat attcaaattc ccctgtcaga tctgttatgt 120

ctcttgcttc tgtaagctct cctgcctgaa cgtaacttaa tataggtatg cgtctggtac 180

ttatctccgc gagaggcata atatttttac cgttcagtag ccagtctggg ctacatttca 240

aggccttagc cagatccaga agattgcgtg gctttctggt tcgtccactt tcgatggatt 300

ctatggattg ttggctaacc cctgcagaat tcgctacttc cacttgagtc attcctaatt 360

ccaaacgacg tgctttaaaa cgggctgcga gagacatggg attagtcctt ctgggttgta 420

ttaatgtgta tctccacatt aaaaacaatt tttgttgtat ttgacaaacc tcgtttgttg 480

ttgctaaata ccactaaaat tgtatgaggt gaaagcaatg accttagcga cccgagtaaa 540

agaacgacgt aaagaactca aaatgacgca agttacgttg gctgagctca caggagtgag 600

ccagcaggca ataaacagaa ttgaaagtgg tgttattgct cggccacgtt atcttcttga 660

aatgtctata gcattggatt gcgaccctaa ttggctgttg tatggctcac aaaacgata 719

<210> 29
<211> 234
<212> DNA
<213> Escherichia coli strain M17

<400> 29
gcggatctgt atacgcaatc ggctttgcaa aggggatgag caatgagggg tattattttt 60

ctgttagctg ccttttctgc gtgcagcgcg tgggcggatg gcttcacggt taaatgcggt 120

ggctacacta tggttgcaaa ccagggcgag ttatcgacta tcaacggtga aagagttacc 180

tctcaaaaaa tcaccgaact gggtaccaat ggtttgaaag tagacatggg gctt 234

<210> 30
<211> 937
<212> DNA
<213> Escherichia coli strain M17

<400> 30
ggctgggcga tgatttttga cgtccataat attatggatg aactgttgcc aattgcgcta 60

aatttaacaa aataatactg gttaacgcat tttttgaacc atctgtacac agtagttgtc 120

tatactttac cttaaaggta aactctgcgc tccactgatg ttcactatgg gaggggatat 180

gaggaaaatc gttaatcgtt acttagccgc tgccggtagt gttatggaca ttatgccgac 240

tacagattac tggcaatatg ctgcttctgg atctgattgt cgggcaataa gaagtgatgt 300

taaagtcatt gcaagtgaca tgaacagagt tatcaggtat gagttcaaaa ccccgcaagc 360

aggcggccgt tctcaaaaag catcaggaaa acaaagcgtc gcaaaaagcc gttcctgatt 420

cggatggtat cgacgaagat attgattcct cggaatatga tgagtctcag gaagtagtgg 480

aaaagctgtc tgcagttgcc aaccttgatc ctgagctaaa atcgaagtta acacctgaac 540

ttcttgagag agtttctaag aacccgaagg ttattgccat gtttcagcag gagtcttttc 600

agggaccact cccacctcct gctatgctct tacaatataa tcaaatatta cccggcggcg 660

ctgagagaat tttcaggcta accgaaaaag agcaagagca taggcataag acgcaaagtg 720

atgcattact tggtgcggtt tcacgcgaca aacgtgggca atggatggga ttttgcattg 780

caatggtagt gcttataatg gctgcgtttt ttgctaatcg cggtaacact tggttcgcag 840

gtactcttgc tactttagat ctcgtaggat taactacagt gtttgtttat ggtaggaaga 900

aacctaaata atccatacat cctttcaaac ccgcttc 937

<210> 31
<211> 888
<212> DNA
<213> Escherichia coli strain M17

<400> 31
gttggtactt tcattcatat acatgaccgt tgtatacggt gaaagagtgg cattgataaa 60

cctagcattt tcttcaacac gtctttgttc aactgtataa taataatgtt tttcaaggat 120

actctcggct acttctcccc accatttatt aataggttca ttattggtta aattggggct 180

gcttaatgag gagaaatttg cgtaacgtcc ccgtcgggca tcagcaaagg aatctaaaca 240

ttcaagtatt ttgtaggtaa ttttatgtgt aggacgtgaa tatctcagag atagttgcat 300

ttttttagaa atactatcaa cctcgttggt taattcaatg agcttgtgtc caaattttct 360

tacaatattc tcgtcaggca tttttccttt gtgttcaatg gcatagtgtg caacaagtat 420

tagtttagca agtctctcaa aacctacaga aagcccaaaa aatgctgtgt aatattcacc 480

tattttgtct gcataattgg ctttacctat ggctgttgcc ccagaaccaa tgagctgtgt 540

tactaaagat gcttctcggc ccagagcgtg ccatgctgga atatcccagg gatttttatt 600

cactttatta ttttcctttg ctcataaatt taaattgttt gagtatctca taaaatataa 660

ggaggtggtt aaggttgttg atcgcatata atagctattg aaagtcaaga actcggtttt 720

atgttctacc actaattaat atattgtgag tctgacaata tattaattag tatgatcaaa 780

ccagccatca actgattcct tgtatctttc agagtctcca gaacaaaagc cttaacgaat 840

tccttatttg tgagtgcatt acaaaaagcc attgttgcag ggaacgag 888

<210> 32
<211> 185
<212> DNA
<213> Escherichia coli strain M17

<400> 32
tatcagagta aaaatgtaac gctgattttt ttgttaaatg atgcgcatta tatattcaat 60

gattaaaata ctcttgatca aataaaacta tctaattaat aatgtaaaat ttcattatgc 120

tatttaagat gtgaaaaata acttaaataa gggatgggat atatttatat tatcccggtt 180

ataga 185

<210> 33
<211> 2424
<212> DNA
<213> Escherichia coli strain M17

<400> 33
atagctattt taagaaaaga aatgatgtaa atatgcatct ggtttttctt tataatgtaa 60

ggatgtccga tgaaacatca ccatcgtgaa tgttatctgg ttaataattt tcgtatgtaa 120
tattatctat gccatctggc catgttttca attgttttag ttctcgacaa ctgtttttat 180
atccagtatg attttactcg tatccggcag aatattttta gatgctgttt catgaactcc 240
ggttgaaagg tttattctct acttttggga ataatagtta aaatggagat aagtgtaatg 300
acgtttagta atgaaattga agttttcata aaagattttg ttaaagatct tgaaaatgat 360
tgtgctgcta ttttttctgg cgcaggaatg tctaaaagtc aaggttatgt tgattggaaa 420
gaattactta gcgatatagc agaagagctt agtttagaca ttaataaaga agacgatcta 480
atatcgcttg ctcaatttca tgttaatgaa aagaaagggg ctacaaaact cacaaggaag 540
ataatcgaag agttttctga gcaagccgag ccatcagagg ttcataatat aattgctcgt 600
ttaccgatta agacattttg gacaacaaac tatgacactt taattgaaga tagtctgcgg 660
aaagtttaca aaattgcaga tgtaaaaaga aacatagatg atttaaatac aacacgacct 720
aagcgagacg cgatagtata taaaatgcat ggtgatattt cgtccccatc caaggctatt 780
ttatataaag agcagtatga aaaatactac agaacacacg cttcatttat caccgcactt 840
agcggagatt taatatcaaa aacctttcta ttcatagggt ttagttttac cgacccaaat 900
ctggattaca ttttgagtcg tttgtatact cattctgggg acaagaggac acactattgc 960
tttatgaaga atgaggctaa taattcccta gatgatgaag aaacagtaaa ttataagatc 1020
agaaaacaaa aactgcgaat cgatgatctc aagagatttg gtatacaagt attattaata 1080
gatgaatatt ctgacatccc taaaattcta gctaagatag agcaatcatt taaaaagaaa 1140
acgatattta tttctggtag tgcagaaaaa tatggggatc ttgaaagacc taaagcatta 1200
gattttattc acacattagc atatgagatt ataaagaata attataggat agtgaatgga 1260
tttggatggg gcgtcggaag ttctgtgata aatggtgcgt tggatgcaat ttatagtagg 1320
ccagataaat tatcagaaga acagctcatt gttagaccat ttccacaaac tgtcagtaag 1380
gggaaagata ttcaagaact ttgggaggag tatagacaga gaatgattcc tcttggtggt 1440
attgcattgt ttatttttgg aaataaacaa gaggaaaata atgatatagt aaatgctagg 1500
ggagtattta gagagtttga gattgctgtt caacatggcc ttgtaccaat tcctttaccc 1560
aataccgggt atatgtctaa atatatattt gaacaaatat cacaagatcc taagaaatat 1620
tatggaagta atgaatggat atataatgat ttgtctgagc ttactgaaaa agatttttcg 1680
gtaaaaaaaa caatagaagt tgtaataaaa atcataaaca aactgaataa ataatatatt 1740
aggtgaagtt atggctcatg aaacattcat atcatataaa tatagtgaag cacaggattt 1800
aagagacaaa attgtcaagg cattaggtga tgatgctgtt tactataaag gggaaacatc 1860
ggagtctcct gatataagta atacaacagt agataatata aagaacaatc ttaaaaatat 1920
gattttcggg acatctgttt taatcgttat tatttccccg aatatgatta aaagtaaatg 1980
gattgactgg gaaattgaat atgcattgaa agaatacaag agaggaggaa ccacttcccg 2040
aactaatggt attgttggtg tgataatgaa ggttaataat agctatgact ggttagtatc 2100

ttcttcaatc aagcctgatg gatgttcatc aagaactatt gacacaacta aactatatga 2160

cattattaat aataataggt ttaatttaac ttcagaagat aaatattgtt gccctgagtg 2220

caaatcatat gatcaactga ctggttctta tatttcattg atagaagaag accgattttt 2280

agcagatgta tctttataca ttgaaaatgc ctatgagaaa tctaaaaaat taagttccta 2340

cgatttatat aaacaaaaat agatgataac ggaaaagggc cacaactatt tgtggcctat 2400

atatacacat attaatcaag cagg 2424

<210> 34
<211> 1407
<212> DNA
<213> Escherichia coli strain M17

<400> 34
taactaaaag gatgctggat caaaatgccc ctgctacgct gtggaaagtg acagaagggg 60

catgaattgt atttcattta atgtggtgaa tgatatttta ttatatcgat ttgttagata 120

ccttcatcaa tttttcttga gtttttctta ttctttcaag ctcttcaaca ataggatttt 180

tctcaccttc actttgcatg aaagggctta agtctatatt gtttttttca acaacttctt 240

tattcatata actatacgtt gcagttattg taaattgttg tggtaaaggg tttttggatt 300

ctccaaaaat aacccaacct tgaccaaggg cgaagaataa ctcttgattg ggcgctaagc 360

tatcaatagt tgatgtaaaa acacttgttt cttttaggtt tttagttggt tcatcgaact 420

gataaaaatc cttatctaat tctaatttta cattcaaggc tggtgtctta ccgctattat 480

aaattttcag gtataggaat tgcgagtgag gtcttaccat tggctggatt attatataag 540

gacgagacat tgcctcggtt tgctcattca ttatctgagc tgttcgttcg cttattctcg 600

acatcttgta tgtcaaaaat gcataaattc ctgtaatgat aacaagaatt gctgtaagta 660

tttccattgt catgattaac tcctaagtat aatgattcat accagtaagg tataatattt 720

taactattat ttcattcttt tctgttgaat agttgattat atccaagttc catttcttca 780

ataaaatctt cactaaatgt tggaaatcca tgtataatag gttctttatc tcgaattttg 840

aataagttat aaagataact actttcttta tatacgacat ggatggcagg atatggccta 900

ttttttataa tcttattgtt tagttcctta tcattgataa tgttatattg atcccctgtt 960

atatcaacta caatattatc tatctcaagc cagtaatggg ttatttgacc atttttcccg 1020

gtagctgcgc taactccttt tagctcacat tcaggccata gggtcagaaa atggtaagaa 1080

agaagcattg aggtaagctt acaactcata acaggaaaac agtctataac aagactggtg 1140

ttctttttat cacaactttc aattaatcta cgaagcttaa ctgcttcttc ttttatttcg 1200

ataatggttg gcaagatcag ctccctgact acatgatacg ccgagtattt aatatatacg 1260

atacataggt cattaaaaag tatgttctaa atttagtgtt tcttatggct cggtggactc 1320

agttttggcg cgcttccttg cctcatgtgt tctggtggca ttgaaggtgt gagatctatg 1380

cttctattaa cttccggctg ttcctga 1407

<210> 35
<211> 1449
<212> DNA
<213> Escherichia coli strain M17

<400> 35
ctttacattt cactatgtga ttatttttta attgtataca cattcttgga ttttgaagtt 60

gagattatcc ttcataaaga aggatgaaga gttccctttt aaaaatgact ctatgctatt 120

tatgtgcata tcataatcat caatgagcac cttatcttgg gatgaaccaa taaaaataac 180

attgacctta tctgcactaa ggttaatgca atgatgattt atattaaagt ctaacaagtg 240

agcaagagca taggttttga ctggaaggtt ttcttcactg aaataactat tttgccaaga 300

taaaatgaat ataaaaatta aaaaagaaaa caaaacggtt gcgaatgaac tcacaggagg 360

gagatcgaaa gcttcatctt taaataaaaa acaaattatt tttattatgt gagttaaaaa 420

gaatgttgct agaataaaca ataaaatagg gttgatatat tttaaaaata gaaaaccagt 480

taaaaaagaa cgtgtataag gaaaggatga tgaatcaatg ccaaatacac tatttaaaag 540

tgctgatgat tttgcggtgc aaaaaataat caatcctgat atggctatag aggtaataca 600

ttttgtaatt attgagttcc atagcacctt aaaaatcaat gttctttcaa taaatacacc 660

taaccataat gtagaaaaaa ttattgataa tagtatgcat gtaaattgcc attgagagaa 720

ccaatcatct gagtctgtct ttgctatacc taaagccaaa catatgagag ctaaaatgcc 780

tatcacaatt aaagcatatg atttctctga ttcatcctga cgttttttaa gctcatcata 840

gttttgtaac ttttttacaa aggctgttgg caggttgctc aattcggaag tgtcattctt 900

catatgttct ccaagataac aacgatatgc tggaagtata ccgctgtatt aaattagtgt 960

gcaagcggaa ttatggtgga gaaacattca taatatcaat tttattgtat gtgtctattt 1020

gacgtatctc tgtattgatg ataactaaat ataacattca ataaccaaag gagataaaaa 1080

acgaatgcac aactggccta tgtttttact attgatactc gtattcatca gaacagtaat 1140

gaaaagatta ctgaaatttt cattgcgaaa tataaagaag gaataacgta tcattttggc 1200

cctgatatgg aacgatttga gtcctgcttt gaagcagcgc tttttgctat tggtgaatgg 1260

caaataaaag ttgatgcgat gacacgatac catgaggaag atgaagtgat ggagttcttt 1320

agctctatac catcagatga agcgggaaat ctggcgacgt ctattttgct gttcagtgat 1380

gttctcgcca tgaaaggcgt ggatgaagta tttgcctatt ttttgggtaa ggatgatgtt 1440

gtatgagtg 1449

<210> 36
<211> 730
<212> DNA
<213> Escherichia coli strain M17

<400> 36
gtagtataca gaattgattt tgtattaatt atttgaatca aaaatgcacg acatagtaag 60

aattaaatgt gttgtgcatt ttgatttcaa cagcctactt taaccgaaaa gaggagttga 120

acgatgatct gacgatttct ttacagtttc tgtgcgatat cccagataaa accaaatggg 180

tctttcacac gggcttttaa atcaccccag aacatttcag caggttcatt tagaacagtc 240

gagccagctt caatcatttc atttaccaga gtgatcatat catctacata catataaaaa 300

ataaagggag gcatagctcc cgaagtattt gggcttttca ggtcggaatc ccatccttcc 360

ttatttatgg tgaagtttgt gccacggtaa cggatacgag caaataatac atcaccacca 420

tcaccctcaa gttcagaaat taccaccatc ttcattactt cagtgtaaaa attaatagct 480

gcttttacat cagggacatt tagtgctgtt gtcaaccatg tcagccccat accttcccaa 540

ggatgtgggc gattttctac gttattgaac cagtccagtt tactcatttc atttttctcc 600

ttaaaaacag catattcatg tgaaacgttt ctgtcgtata ttactctaaa ttgataaatc 660

ttatacaatc attaagaatt tatggtgtca gtttataaag ggcggggact atttgtagct 720

aattagaact 730

<210> 37
<211> 20
<212> DNA
<213> Artificial sequence

<220>
<223> Single strand DNA oligonucleotide

<400> 37
tgcgaatcga tgatctcaag 20

<210> 38
<211> 20
<212> DNA
<213> Artificial sequence

<220>
<223> Single strand DNA oligonucleotide

<400> 38
ttggtacaag gccatgttga 20

<210> 39
<211> 20
<212> DNA
<213> Artificial sequence

<220>
<223> Single strand DNA oligonucleotide

<400> 39
gctgtttcat gaactccggt 20

<210> 40
<211> 20
<212> DNA
<213> Artificial sequence

<220>
<223> Single strand DNA oligonucleotide

<400> 40

tggggacgaa atatcaccat                                                    20

<210>  41
<211>  20
<212>  DNA
<213>  Artificial sequence

<220>
<223>  Single strand DNA oligonucleotide

<400>  41
tccgggttga taaccatcat                                                    20

<210>  42
<211>  20
<212>  DNA
<213>  Artificial sequence

<220>
<223>  Single strand DNA oligonucleotide

<400>  42
accactctgg tccttcatgc                                                    20

<210>  43
<211>  20
<212>  DNA
<213>  Artificial sequence

<220>
<223>  Single strand DNA oligonucleotide

<400>  43
ccgtggacga atagagcatt                                                    20

<210>  44
<211>  20
<212>  DNA
<213>  Artificial sequence

<220>
<223>  Single strand DNA oligonucleotide

<400>  44
tttttggtgc attgattcca                                                    20

<210>  45
<211>  20
<212>  DNA
<213>  Artificial sequence

<220>
<223>  Single strand DNA oligonucleotide

<400>  45
atctgagctg ttcgttcgct                                                    20

<210>  46
<211>  20
<212>  DNA
<213>  Artificial sequence

<220>
<223>  Single strand DNA oligonucleotide

<400>  46
taccgggaaa aatggtcaaa                                                    20


<210>  47
<211>  144
<212>  DNA
<213>  Escherichia coli strain M17

<400>  47
gctcataaat ttaaattgtt tgagtatctc ataaaatata aggaggtggt taaggttgtt        60

gatcgcatat aatagctatt gaaagtcaag aactcggttt tatgttctac cactaattaa       120

tatattgtga gtctgacaat atat                                              144


<210>  48
<211>  322
<212>  DNA
<213>  Escherichia coli strain M17

<400>  48
atagctattt taagaaaaga aatgatgtaa atatgcatct ggtttttctt tataatgtaa        60

ggatgtccga tgaaacatca ccatcgtgaa tgttatctgg ttaataattt tcgtatgtaa       120

tattatctat gccatctggc catgttttca attgttttag ttctcgacaa ctgtttttat       180

atccagtatg attttactcg tatccggcag aatattttta gatgctgttt catgaactcc       240

ggttgaaagg tttattctct acttttggga ataatagtta aaatggagat aagtgtaatg       300

acgtttagta atgaaattga ag                                                322


<210>  49
<211>  263
<212>  DNA
<213>  Escherichia coli strain M17

<400>  49
agtaaatgct aggggagtat ttagagagtt tgagattgct gttcaacatg gccttgtacc        60

aattccttta cccaataccg ggtatatgtc taaatatata tttgaacaaa tatcacaaga       120

tcctaagaaa tattatggaa gtaatgaatg gatatataat gatttgtctg agcttactga       180

aaaagatttt tcggtaaaaa aaacaataga agttgtaata aaaatcataa acaaactgaa       240

taaataatat attaggtgaa gtt                                               263


<210>  50
<211>  188
<212>  DNA
<213>  Escherichia coli strain M17

<400>  50
taactaaaag gatgctggat caaaatgccc ctgctacgct gtggaaagtg acagaagggg        60

catgaattgt atttcattta atgtggtgaa tgatatttta ttatatcgat ttgttagata       120

ccttcatcaa tttttcttga gttttcttta ttctttcaag ctcttcaaca ataggatttt       180

tctcacct                                                              188

<210>  51
<211>  193
<212>  DNA
<213>  Escherichia coli strain M17

<400>  51
gatcagctcc ctgactacat gatacgccga gtatttaata tatacgatac ataggtcatt      60

aaaaagtatg ttctaaattt agtgtttctt atggctcggt ggactcagtt ttggcgcgct     120

tccttgcctc atgtgttctg gtggcattga aggtgtgaga tctatgcttc tattaacttc     180

cggctgttcc tga                                                        193

<210>  52
<211>  20
<212>  DNA
<213>  Artificial sequence

<220>
<223>  Single strand DNA oligonucleotide

<400>  52
aaggtaaact ctgcgctcca                                                  20

<210>  53
<211>  20
<212>  DNA
<213>  Artificial sequence

<220>
<223>  Single strand DNA oligonucleotide

<400>  53
ccctgaaaag actcctgctg                                                  20

<210>  54
<211>  20
<212>  DNA
<213>  Artificial sequence

<220>
<223>  Single strand DNA oligonucleotide

<400>  54
tggtgctaga ctctgggctt                                                  20

<210>  55
<211>  20
<212>  DNA
<213>  Artificial sequence

<220>
<223>  Single strand DNA oligonucleotide

<400>  55
tgacggaaat atccacagca                                                  20

<210>  56
<211>  20

<212>  DNA
<213>  Artificial sequence

<220>
<223>  Single strand DNA oligonucleotide

<400>  56
cgctgtggaa agtgacagaa                                                    20


<210>  57
<211>  20
<212>  DNA
<213>  Artificial sequence

<220>
<223>  Single strand DNA oligonucleotide

<400>  57
aatgaatgag caaaccgagg                                                    20


<210>  58
<211>  20
<212>  DNA
<213>  Artificial sequence

<220>
<223>  Single strand DNA oligonucleotide

<400>  58
gcgataactg ggcaaatgat                                                    20


<210>  59
<211>  20
<212>  DNA
<213>  Artificial sequence

<220>
<223>  Single strand DNA oligonucleotide

<400>  59
attgcatgag tccaaccaca                                                    20


<210>  60
<211>  20
<212>  DNA
<213>  Artificial sequence

<220>
<223>  Single strand DNA oligonucleotide

<400>  60
aaatcggata ccagtgcagc                                                    20


<210>  61
<211>  20
<212>  DNA
<213>  Artificial sequence

<220>
<223>  Single strand DNA oligonucleotide

<400>  61
gcaccagcag tacatcgaga                                                    20

<210>  62
<211>  19
<212>  DNA
<213>  Artificial sequence

<220>
<223>  Single strand DNA oligonucleotide

<400>  62
ccagctcatc gtgttttcc                                                    19


<210>  63
<211>  20
<212>  DNA
<213>  Artificial sequence

<220>
<223>  Single strand DNA oligonucleotide

<400>  63
ggtgtcgatg cgataaaacc                                                   20


<210>  64
<211>  20
<212>  DNA
<213>  Artificial sequence

<220>
<223>  Single strand DNA oligonucleotide

<400>  64
aaccctatcg gtcaggctct                                                   20


<210>  65
<211>  20
<212>  DNA
<213>  Artificial sequence

<220>
<223>  Single strand DNA oligonucleotide

<400>  65
gttgctcaag tgtcgccata                                                   20


<210>  66
<211>  20
<212>  DNA
<213>  Artificial sequence

<220>
<223>  Single strand DNA oligonucleotide

<400>  66
cctgatggcg gaaaagaata                                                   20


<210>  67
<211>  20
<212>  DNA
<213>  Artificial sequence

<220>

<223>  Single strand DNA oligonucleotide

<400>  67
ggtgtcgatg cgataaaacc                                                    20


<210>  68
<211>  20
<212>  DNA
<213>  Artificial sequence

<220>
<223>  Single strand DNA oligonucleotide

<400>  68
tccgggttga taaccatcat                                                    20


<210>  69
<211>  20
<212>  DNA
<213>  Artificial sequence

<220>
<223>  Single strand DNA oligonucleotide

<400>  69
tgacaccaac gccaataaga                                                    20


<210>  70
<211>  20
<212>  DNA
<213>  Artificial sequence

<220>
<223>  Single strand DNA oligonucleotide

<400>  70
tcggtcaggc tctctcaaat                                                    20


<210>  71
<211>  20
<212>  DNA
<213>  Artificial sequence

<220>
<223>  Single strand DNA oligonucleotide

<400>  71
cggaagctgc gaattttatt                                                    20


<210>  72
<211>  20
<212>  DNA
<213>  Artificial sequence

<220>
<223>  Single strand DNA oligonucleotide

<400>  72
cctgatggcg gaaaagaata                                                    20


<210>  73
<211>  20

<212>  DNA
<213>  Artificial sequence

<220>
<223>  Single strand DNA oligonucleotide

<400>  73
gcttttaggg cgctgagtta                                                    20


<210>  74
<211>  20
<212>  DNA
<213>  Artificial sequence

<220>
<223>  Single strand DNA oligonucleotide

<400>  74
atcggtcagg ctctctcaaa                                                    20


<210>  75
<211>  20
<212>  DNA
<213>  Artificial sequence

<220>
<223>  Single strand DNA oligonucleotide

<400>  75
gttgctcaag tgtcgccata                                                    20


<210>  76
<211>  20
<212>  DNA
<213>  Artificial sequence

<220>
<223>  Single strand DNA oligonucleotide

<400>  76
tccgggttga taaccatcat                                                    20


<210>  77
<211>  20
<212>  DNA
<213>  Artificial sequence

<220>
<223>  Single strand DNA oligonucleotide

<400>  77
accactctgg tccttcatgc                                                    20


<210>  78
<211>  20
<212>  DNA
<213>  Artificial sequence

<220>
<223>  Single strand DNA oligonucleotide

<400>  78
tcggtcaggc tctctcaaat                                                    20

<210>  79
<211>  20
<212>  DNA
<213>  Artificial sequence

<220>
<223>  Single strand DNA oligonucleotide

<400>  79
gatccccatc atggaaacat                                                    20


<210>  80
<211>  20
<212>  DNA
<213>  Artificial sequence

<220>
<223>  Single strand DNA oligonucleotide

<400>  80
tatggcgaca cttgagcaac                                                    20


<210>  81
<211>  20
<212>  DNA
<213>  Artificial sequence

<220>
<223>  Single strand DNA oligonucleotide

<400>  81
ttccgggaag ataaatggtg                                                    20


<210>  82
<211>  20
<212>  DNA
<213>  Artificial sequence

<220>
<223>  Single strand DNA oligonucleotide

<400>  82
tcttattggc gttggtgtca                                                    20


<210>  83
<211>  20
<212>  DNA
<213>  Artificial sequence

<220>
<223>  Single strand DNA oligonucleotide

<400>  83
gcagtatccg gtttttcagc                                                    20


<210>  84
<211>  20
<212>  DNA
<213>  Artificial sequence

<220>

<223>  Single strand DNA oligonucleotide

<400>  84
aaccctatcg gtcaggctct                                                    20


<210>  85
<211>  20
<212>  DNA
<213>  Artificial sequence

<220>
<223>  Single strand DNA oligonucleotide

<400>  85
gcgtagtgaa tgcggatgta                                                    20


<210>  86
<211>  20
<212>  DNA
<213>  Artificial sequence

<220>
<223>  Single strand DNA oligonucleotide

<400>  86
ggcgctcatc gtattgtgta                                                    20


<210>  87
<211>  20
<212>  DNA
<213>  Artificial sequence

<220>
<223>  Single strand DNA oligonucleotide

<400>  87
gcggaataac gagtccacat                                                    20


<210>  88
<211>  20
<212>  DNA
<213>  Artificial sequence

<220>
<223>  Single strand DNA oligonucleotide

<400>  88
agagcctcga agatgtttgc                                                    20


<210>  89
<211>  20
<212>  DNA
<213>  Artificial sequence

<220>
<223>  Single strand DNA oligonucleotide

<400>  89
acattttgct gtggaccttg                                                    20


<210>  90
<211>  20

<212>  DNA
<213>  Artificial sequence

<220>
<223>  Single strand DNA oligonucleotide

<400>  90
gcctcgaaga tgtttgctct                                                    20


<210>  91
<211>  20
<212>  DNA
<213>  Artificial sequence

<220>
<223>  Single strand DNA oligonucleotide

<400>  91
   cgggcataat gacctttttc                                                     20


<210>  92
<211>  20
<212>  DNA
<213>  Artificial sequence

<220>
<223>  Single strand DNA oligonucleotide

<400>  92
agagcctcga agatgtttgc                                                    20


<210>  93
<211>  20
<212>  DNA
<213>  Artificial sequence

<220>
<223>  Single strand DNA oligonucleotide

<400>  93
ctgtaggcca gtgagcgttt                                                    20


<210>  94
<211>  20
<212>  DNA
<213>  Artificial sequence

<220>
<223>  Single strand DNA oligonucleotide

<400>  94
cgcagaccta caggaagcat                                                    20


<210>  95
<211>  20
<212>  DNA
<213>  Artificial sequence

<220>
<223>  Single strand DNA oligonucleotide

<400>  95
gaggggaggc aaaagaaaac                                                    20

<210>  96
<211>  20
<212>  DNA
<213>  Artificial sequence

<220>
<223>  Single strand DNA oligonucleotide

<400>  96
ccggaaaaat tagcgttgaa                                                    20


<210>  97
<211>  20
<212>  DNA
<213>  Artificial sequence

<220>
<223>  Single strand DNA oligonucleotide

<400>  97
caattctccg gcatcaagtt                                                    20


<210>  98
<211>  20
<212>  DNA
<213>  Artificial sequence

<220>
<223>  Single strand DNA oligonucleotide

<400>  98
tgtggttgga ctcatgcaat                                                    20


<210>  99
<211>  20
<212>  DNA
<213>  Artificial sequence

<220>
<223>  Single strand DNA oligonucleotide

<400>  99
ttggcactaa tcgcctaacc                                                    20


<210>  100
<211>  20
<212>  DNA
<213>  Artificial sequence

<220>
<223>  Single strand DNA oligonucleotide

<400>  100
caaaaggagg gcgataatga                                                    20


<210>  101
<211>  20
<212>  DNA
<213>  Artificial sequence

<220>

<223>  Single strand DNA oligonucleotide

<400>  101
agcccaatgc tgacttgaac                                                    20


<210>  102
<211>  20
<212>  DNA
<213>  Artificial sequence

<220>
<223>  Single strand DNA oligonucleotide

<400>  102
cgggtgttgt cctaactgct                                                    20


<210>  103
<211>  20
<212>  DNA
<213>  Artificial sequence

<220>
<223>  Single strand DNA oligonucleotide

<400>  103
gagtggtcat tggcctcatt                                                    20


<210>  104
<211>  20
<212>  DNA
<213>  Artificial sequence

<220>
<223>  Single strand DNA oligonucleotide

<400>  104
ataccgccca atagggaaag                                                    20


<210>  105
<211>  20
<212>  DNA
<213>  Artificial sequence

<220>
<223>  Single strand DNA oligonucleotide

<400>  105
gagtggtcat tggcctcatt                                                    20


<210>  106
<211>  20
<212>  DNA
<213>  Artificial sequence

<220>
<223>  Single strand DNA oligonucleotide

<400>  106
ataccgccca atagggaaag                                                    20


<210>  107
<211>  20

<212>  DNA
<213>  Artificial sequence

<220>
<223>  Single strand DNA oligonucleotide

<400>  107
   tcattatcgc cctccttttg                                                    20


<210>  108
<211>  20
<212>  DNA
<213>  Artificial sequence

<220>
<223>  Single strand DNA oligonucleotide

<400>  108
ggggaaataa cgggaaaaga                                                   20


<210>  109
<211>  20
<212>  DNA
<213>  Artificial sequence

<220>
<223>  Single strand DNA oligonucleotide

<400>  109
gggcttgatc atttgtgctt                                                   20


<210>  110
<211>  20
<212>  DNA
<213>  Artificial sequence

<220>
<223>  Single strand DNA oligonucleotide

<400>  110
gcgataactg ggcaaatgat                                                   20


<210>  111
<211>  20
<212>  DNA
<213>  Artificial sequence

<220>
<223>  Single strand DNA oligonucleotide

<400>  111
attgcatgag tccaaccaca                                                   20


<210>  112
<211>  20
<212>  DNA
<213>  Artificial sequence

<220>
<223>  Single strand DNA oligonucleotide

<400>  112
tctttccgat ggctcagtct                                                   20

<210>  113
<211>  20
<212>  DNA
<213>  Artificial sequence

<220>
<223>  Single strand DNA oligonucleotide

<400>  113
cagacccgtt ttgcagagat                                                    20


<210>  114
<211>  20
<212>  DNA
<213>  Artificial sequence

<220>
<223>  Single strand DNA oligonucleotide

<400>  114
gatggctcag tctggaaagg                                                    20


<210>  115
<211>  20
<212>  DNA
<213>  Artificial sequence

<220>
<223>  Single strand DNA oligonucleotide

<400>  115
ttgaggctcc cgtaacattc                                                    20


<210>  116
<211>  20
<212>  DNA
<213>  Artificial sequence

<220>
<223>  Single strand DNA oligonucleotide

<400>  116
ggaatggtgc tgtttccatt                                                    20


<210>  117
<211>  20
<212>  DNA
<213>  Artificial sequence

<220>
<223>  Single strand DNA oligonucleotide

<400>  117
cagacccgtt ttgcagagat                                                    20


<210>  118
<211>  20
<212>  DNA
<213>  Artificial sequence

<220>

<223>  Single strand DNA oligonucleotide

<400>  118
gcacgtgtca cactgaaaaa                                                    20


<210>  119
<211>  20
<212>  DNA
<213>  Artificial sequence

<220>
<223>  Single strand DNA oligonucleotide

<400>  119
aaacagcacc attccacaca                                                    20


<210>  120
<211>  19
<212>  DNA
<213>  Artificial sequence

<220>
<223>  Single strand DNA oligonucleotide

<400>  120
aaaactccag ctgggatgg                                                     19


<210>  121
<211>  20
<212>  DNA
<213>  Artificial sequence

<220>
<223>  Single strand DNA oligonucleotide

<400>  121
cgttaatgga aacagcacca                                                    20


<210>  122
<211>  21
<212>  DNA
<213>  Artificial sequence

<220>
<223>  Single strand DNA oligonucleotide

<400>  122
cgtaggtaaa ggtctggatg g                                                  21


<210>  123
<211>  20
<212>  DNA
<213>  Artificial sequence

<220>
<223>  Single strand DNA oligonucleotide

<400>  123
gaaccgagcc cattggtact                                                    20


<210>  124
<211>  20

<212>  DNA
<213>  Artificial sequence

<220>
<223>  Single strand DNA oligonucleotide

<400>  124
gctgggatgg tgatgtcaat                                                    20


<210>  125
<211>  20
<212>  DNA
<213>  Artificial sequence

<220>
<223>  Single strand DNA oligonucleotide

<400>  125
gaaccgagcc cattggtact                                                    20


<210>  126
<211>  20
<212>  DNA
<213>  Artificial sequence

<220>
<223>  Single strand DNA oligonucleotide

<400>  126
tgctgttttt ctgacggatg                                                    20


<210>  127
<211>  20
<212>  DNA
<213>  Artificial sequence

<220>
<223>  Single strand DNA oligonucleotide

<400>  127
gaaccgagcc cattggtact                                                    20


<210>  128
<211>  19
<212>  DNA
<213>  Artificial sequence

<220>
<223>  Single strand DNA oligonucleotide

<400>  128
aaaactccag ctgggatgg                                                     19


<210>  129
<211>  20
<212>  DNA
<213>  Artificial sequence

<220>
<223>  Single strand DNA oligonucleotide

<400>  129
aaacagcacc attccacaca                                                    20

<210>  130
<211>  20
<212>  DNA
<213>  Artificial sequence

<220>
<223>  Single strand DNA oligonucleotide

<400>  130
agagtccgtc tcccatagcc                                                    20


<210>  131
<211>  20
<212>  DNA
<213>  Artificial sequence

<220>
<223>  Single strand DNA oligonucleotide

<400>  131
acttgcccag gttcttgaaa                                                    20


<210>  132
<211>  20
<212>  DNA
<213>  Artificial sequence

<220>
<223>  Single strand DNA oligonucleotide

<400>  132
gtccgtctcc catagcctta                                                    20


<210>  133
<211>  20
<212>  DNA
<213>  Artificial sequence

<220>
<223>  Single strand DNA oligonucleotide

<400>  133
acttgcccag gttcttgaaa                                                    20


<210>  134
<211>  20
<212>  DNA
<213>  Artificial sequence

<220>
<223>  Single strand DNA oligonucleotide

<400>  134
aagtgtgcca ttgcctttct                                                    20


<210>  135
<211>  20
<212>  DNA
<213>  Artificial sequence

<220>

<223>  Single strand DNA oligonucleotide

<400>  135
tattaaggcg ccacaactgg                                                    20


<210>  136
<211>  20
<212>  DNA
<213>  Artificial sequence

<220>
<223>  Single strand DNA oligonucleotide

<400>  136
caactggtac tgagcgttcg                                                    20


<210>  137
<211>  20
<212>  DNA
<213>  Artificial sequence

<220>
<223>  Single strand DNA oligonucleotide

<400>  137
gaaaaagagc gggtgaacaa                                                    20


<210>  138
<211>  20
<212>  DNA
<213>  Artificial sequence

<220>
<223>  Single strand DNA oligonucleotide

<400>  138
gttaatatcg cgcgtccatc                                                    20


<210>  139
<211>  20
<212>  DNA
<213>  Artificial sequence

<220>
<223>  Single strand DNA oligonucleotide

<400>  139
ctatccttga cccgacgaac                                                    20


<210>  140
<211>  20
<212>  DNA
<213>  Artificial sequence

<220>
<223>  Single strand DNA oligonucleotide

<400>  140
ccattgcctt tctgcttgtt                                                    20


<210>  141
<211>  20

<212>  DNA
<213>  Artificial sequence

<220>
<223>  Single strand DNA oligonucleotide

<400>  141
tattaaggcg ccacaactgg                                                    20


<210>  142
<211>  19
<212>  DNA
<213>  Artificial sequence

<220>
<223>  Single strand DNA oligonucleotide

<400>  142
ttgcttcatc atcgccatt                                                     19


<210>  143
<211>  20
<212>  DNA
<213>  Artificial sequence

<220>
<223>  Single strand DNA oligonucleotide

<400>  143
cgcaattagt gaccagatcg                                                    20


<210>  144
<211>  20
<212>  DNA
<213>  Artificial sequence

<220>
<223>  Single strand DNA oligonucleotide

<400>  144
caactggtac tgagcgttcg                                                    20


<210>  145
<211>  20
<212>  DNA
<213>  Artificial sequence

<220>
<223>  Single strand DNA oligonucleotide

<400>  145
acttgcccag gttcttgaaa                                                    20


<210>  146
<211>  19
<212>  DNA
<213>  Artificial sequence

<220>
<223>  Single strand DNA oligonucleotide

<400>  146
atgggcatca cgcaagata                                                     19

<210>  147
<211>  20
<212>  DNA
<213>  Artificial sequence

<220>
<223>  Single strand DNA oligonucleotide

<400>  147
aatcgtgtcc agccattttg                                                    20


<210>  148
<211>  20
<212>  DNA
<213>  Artificial sequence

<220>
<223>  Single strand DNA oligonucleotide

<400>  148
gttgtggagc agcttgaaca                                                    20


<210>  149
<211>  20
<212>  DNA
<213>  Artificial sequence

<220>
<223>  Single strand DNA oligonucleotide

<400>  149
gatatcctgc ggacgctcta                                                    20


<210>  150
<211>  20
<212>  DNA
<213>  Artificial sequence

<220>
<223>  Single strand DNA oligonucleotide

<400>  150
gttgtggagc agcttgaaca                                                    20


<210>  151
<211>  20
<212>  DNA
<213>  Artificial sequence

<220>
<223>  Single strand DNA oligonucleotide

<400>  151
gctctatccc tgctgaatgc                                                    20


<210>  152
<211>  20
<212>  DNA
<213>  Artificial sequence

<220>

<223>  Single strand DNA oligonucleotide

<400>  152
ccaactaccc accctgtgtc                                                    20


<210>  153
<211>  20
<212>  DNA
<213>  Artificial sequence

<220>
<223>  Single strand DNA oligonucleotide

<400>  153
ccgaatcgtt gactcgtatg                                                    20


<210>  154
<211>  20
<212>  DNA
<213>  Artificial sequence

<220>
<223>  Single strand DNA oligonucleotide

<400>  154
ccaactaccc accctgtgtc                                                    20


<210>  155
<211>  20
<212>  DNA
<213>  Artificial sequence

<220>
<223>  Single strand DNA oligonucleotide

<400>  155
caaccgctcg aacaccttag                                                    20


<210>  156
<211>  20
<212>  DNA
<213>  Artificial sequence

<220>
<223>  Single strand DNA oligonucleotide

<400>  156
ccacggtata tccctcggta                                                    20


<210>  157
<211>  20
<212>  DNA
<213>  Artificial sequence

<220>
<223>  Single strand DNA oligonucleotide

<400>  157
cttcgggatt catggttagc                                                    20


<210>  158
<211>  20

<212>  DNA
<213>  Artificial sequence

<220>
<223>  Single strand DNA oligonucleotide

<400>  158
ggcagtcttt ctggcatagg                                                    20


<210>  159
<211>  20
<212>  DNA
<213>  Artificial sequence

<220>
<223>  Single strand DNA oligonucleotide

<400>  159
attgggtgag cctgattgaa                                                    20


<210>  160
<211>  20
<212>  DNA
<213>  Artificial sequence

<220>
<223>  Single strand DNA oligonucleotide

<400>  160
ggtgctagac tctgggcttg                                                    20


<210>  161
<211>  20
<212>  DNA
<213>  Artificial sequence

<220>
<223>  Single strand DNA oligonucleotide

<400>  161
gatttcccac gctgtcactt                                                    20


<210>  162
<211>  20
<212>  DNA
<213>  Artificial sequence

<220>
<223>  Single strand DNA oligonucleotide

<400>  162
tgatcagtga ttgcgtgaca                                                    20


<210>  163
<211>  20
<212>  DNA
<213>  Artificial sequence

<220>
<223>  Single strand DNA oligonucleotide

<400>  163
aagcccagag tctagcacca                                                    20

<210>  164
<211>  20
<212>  DNA
<213>  Artificial sequence

<220>
<223>  Single strand DNA oligonucleotide

<400>  164
ggtgctagac tctgggcttg                                                    20


<210>  165
<211>  20
<212>  DNA
<213>  Artificial sequence

<220>
<223>  Single strand DNA oligonucleotide

<400>  165
agcataccca aaatggcaac                                                    20


<210>  166
<211>  20
<212>  DNA
<213>  Artificial sequence

<220>
<223>  Single strand DNA oligonucleotide

<400>  166
aggtcgatct acgcgaaaaa                                                    20


<210>  167
<211>  20
<212>  DNA
<213>  Artificial sequence

<220>
<223>  Single strand DNA oligonucleotide

<400>  167
gcaaagccta tgccagaaag                                                    20


<210>  168
<211>  20
<212>  DNA
<213>  Artificial sequence

<220>
<223>  Single strand DNA oligonucleotide

<400>  168
atcagaaccc gacgacaaag                                                    20


<210>  169
<211>  20
<212>  DNA
<213>  Artificial sequence

<220>

<223>  Single strand DNA oligonucleotide

<400>  169
tccaggcttc gaggagagta                                                    20


<210>  170
<211>  20
<212>  DNA
<213>  Artificial sequence

<220>
<223>  Single strand DNA oligonucleotide

<400>  170
aacaagtcca gtgcatcacg                                                    20


<210>  171
<211>  20
<212>  DNA
<213>  Artificial sequence

<220>
<223>  Single strand DNA oligonucleotide

<400>  171
gtcaggacga tttcgttggt                                                    20


<210>  172
<211>  20
<212>  DNA
<213>  Artificial sequence

<220>
<223>  Single strand DNA oligonucleotide

<400>  172
caggtatggc aaggacgatt                                                    20


<210>  173
<211>  20
<212>  DNA
<213>  Artificial sequence

<220>
<223>  Single strand DNA oligonucleotide

<400>  173
atgaaccctt gcgaatcaag                                                    20


<210>  174
<211>  20
<212>  DNA
<213>  Artificial sequence

<220>
<223>  Single strand DNA oligonucleotide

<400>  174
cttgattcgc aagggttcat                                                    20


<210>  175
<211>  20

<212>  DNA
<213>  Artificial sequence

<220>
<223>  Single strand DNA oligonucleotide

<400>  175
ggagttacgc gagttgcttt                                                    20


<210>  176
<211>  20
<212>  DNA
<213>  Artificial sequence

<220>
<223>  Single strand DNA oligonucleotide

<400>  176
ttccatgact cgtcagcaag                                                    20


<210>  177
<211>  20
<212>  DNA
<213>  Artificial sequence

<220>
<223>  Single strand DNA oligonucleotide

<400>  177
atgtcccata tagcccgttg                                                    20


<210>  178
<211>  20
<212>  DNA
<213>  Artificial sequence

<220>
<223>  Single strand DNA oligonucleotide

<400>  178
cttgattcgc aagggttcat                                                    20


<210>  179
<211>  20
<212>  DNA
<213>  Artificial sequence

<220>
<223>  Single strand DNA oligonucleotide

<400>  179
ctttctcgga cgaacgattt                                                    20


<210>  180
<211>  20
<212>  DNA
<213>  Artificial sequence

<220>
<223>  Single strand DNA oligonucleotide

<400>  180
ttgccaacga tacaaatcca                                                    20

<210> 181
<211> 20
<212> DNA
<213> Artificial sequence

<220>
<223> Single strand DNA oligonucleotide

<400> 181
tcgaaattga cccgaaactc 20


<210> 182
<211> 20
<212> DNA
<213> Artificial sequence

<220>
<223> Single strand DNA oligonucleotide

<400> 182
tttttggaat tggcagcatc 20


<210> 183
<211> 20
<212> DNA
<213> Artificial sequence

<220>
<223> Single strand DNA oligonucleotide

<400> 183
gcgttgtgtt cttctgtgga 20


<210> 184
<211> 20
<212> DNA
<213> Artificial sequence

<220>
<223> Single strand DNA oligonucleotide

<400> 184
gctttggctt aagggctttt 20


<210> 185
<211> 20
<212> DNA
<213> Artificial sequence

<220>
<223> Single strand DNA oligonucleotide

<400> 185
aattgagcgt gaggttttcg 20


<210> 186
<211> 20
<212> DNA
<213> Artificial sequence

<220>

<223>  Single strand DNA oligonucleotide

<400>  186
ccacatgaaa gagacggtca                                                    20


<210>  187
<211>  20
<212>  DNA
<213>  Artificial sequence

<220>
<223>  Single strand DNA oligonucleotide

<400>  187
gggcgctatt gatactcagg                                                    20


<210>  188
<211>  20
<212>  DNA
<213>  Artificial sequence

<220>
<223>  Single strand DNA oligonucleotide

<400>  188
aagcctggcc tgtacgttta                                                    20


<210>  189
<211>  20
<212>  DNA
<213>  Artificial sequence

<220>
<223>  Single strand DNA oligonucleotide

<400>  189
acctggtttc aaagggttgg                                                    20


<210>  190
<211>  20
<212>  DNA
<213>  Artificial sequence

<220>
<223>  Single strand DNA oligonucleotide

<400>  190
gcagggatcc agctaattga                                                    20


<210>  191
<211>  21
<212>  DNA
<213>  Artificial sequence

<220>
<223>  Single strand DNA oligonucleotide

<400>  191
aacagggttt gggacatttt t                                                  21


<210>  192
<211>  19

<212>  DNA
<213>  Artificial sequence

<220>
<223>  Single strand DNA oligonucleotide

<400>  192
cgattcgcaa gaatctgga                                                    19


<210>  193
<211>  20
<212>  DNA
<213>  Artificial sequence

<220>
<223>  Single strand DNA oligonucleotide

<400>  193
ttttgcctct gtcaccatca                                                   20


<210>  194
<211>  20
<212>  DNA
<213>  Artificial sequence

<220>
<223>  Single strand DNA oligonucleotide

<400>  194
aaaatgcagg gatccagcta                                                   20


<210>  195
<211>  23
<212>  DNA
<213>  Artificial sequence

<220>
<223>  Single strand DNA oligonucleotide

<400>  195
gcggataaga aaagacaata gcc                                               23


<210>  196
<211>  25
<212>  DNA
<213>  Artificial sequence

<220>
<223>  Single strand DNA oligonucleotide

<400>  196
cgagctgata ataaattatg gaacc                                             25


<210>  197
<211>  20
<212>  DNA
<213>  Artificial sequence

<220>
<223>  Single strand DNA oligonucleotide

<400>  197
gcactgcaga gccagagata                                                   20

<210>  198
<211>  25
<212>  DNA
<213>  Artificial sequence

<220>
<223>  Single strand DNA oligonucleotide

<400>  198
cgagctgata ataaattatg gaacc                                          25


<210>  199
<211>  20
<212>  DNA
<213>  Artificial sequence

<220>
<223>  Single strand DNA oligonucleotide

<400>  199
atctctcgcg ggtagttgag                                                20


<210>  200
<211>  20
<212>  DNA
<213>  Artificial sequence

<220>
<223>  Single strand DNA oligonucleotide

<400>  200
ttgatggtga cagaggcaaa                                                20


<210>  201
<211>  20
<212>  DNA
<213>  Artificial sequence

<220>
<223>  Single strand DNA oligonucleotide

<400>  201
atctctcgcg ggtagttgag                                                20


<210>  202
<211>  20
<212>  DNA
<213>  Artificial sequence

<220>
<223>  Single strand DNA oligonucleotide

<400>  202
ctcgcttttc ccaagtgaat                                                20


<210>  203
<211>  20
<212>  DNA
<213>  Artificial sequence

<220>

<223>  Single strand DNA oligonucleotide

<400>  203
gagatgagtt tcgggagcag                                                    20


<210>  204
<211>  20
<212>  DNA
<213>  Artificial sequence

<220>
<223>  Single strand DNA oligonucleotide

<400>  204
ggaaacaaac cgactggaaa                                                    20


<210>  205
<211>  20
<212>  DNA
<213>  Artificial sequence

<220>
<223>  Single strand DNA oligonucleotide

<400>  205
caccccacca gaaccataaa                                                    20


<210>  206
<211>  20
<212>  DNA
<213>  Artificial sequence

<220>
<223>  Single strand DNA oligonucleotide

<400>  206
gaatactgat gcggcagtcc                                                    20


<210>  207
<211>  20
<212>  DNA
<213>  Artificial sequence

<220>
<223>  Single strand DNA oligonucleotide

<400>  207
tgggcaatga ttgtttgtgt                                                    20


<210>  208
<211>  20
<212>  DNA
<213>  Artificial sequence

<220>
<223>  Single strand DNA oligonucleotide

<400>  208
ggcttatggg aaagcactca                                                    20


<210>  209
<211>  20

<212>  DNA
<213>  Artificial sequence

<220>
<223>  Single strand DNA oligonucleotide

<400>  209
tgggcaatga ttgtttgtgt                                                    20


<210>  210
<211>  20
<212>  DNA
<213>  Artificial sequence

<220>
<223>  Single strand DNA oligonucleotide

<400>  210
cgattgtggc tgaaattgaa                                                    20


<210>  211
<211>  20
<212>  DNA
<213>  Artificial sequence

<220>
<223>  Single strand DNA oligonucleotide

<400>  211
agctagatcg ccactggaaa                                                    20


<210>  212
<211>  20
<212>  DNA
<213>  Artificial sequence

<220>
<223>  Single strand DNA oligonucleotide

<400>  212
gcgacaacat cagaaaacga                                                    20


<210>  213
<211>  20
<212>  DNA
<213>  Artificial sequence

<220>
<223>  Single strand DNA oligonucleotide

<400>  213
ttgctcatgg cgaaataaca                                                    20


<210>  214
<211>  20
<212>  DNA
<213>  Artificial sequence

<220>
<223>  Single strand DNA oligonucleotide

<400>  214
gcccactttt tccagtcaaa                                                    20

<210>  215
<211>  20
<212>  DNA
<213>  Artificial sequence

<220>
<223>  Single strand DNA oligonucleotide

<400>  215
gctagatcgc cactggaaac                                                    20


<210>  216
<211>  20
<212>  DNA
<213>  Artificial sequence

<220>
<223>  Single strand DNA oligonucleotide

<400>  216
aggtgcagaa atgagcgagt                                                    20


<210>  217
<211>  20
<212>  DNA
<213>  Artificial sequence

<220>
<223>  Single strand DNA oligonucleotide

<400>  217
tttgccaata ttccccagag                                                    20


<210>  218
<211>  20
<212>  DNA
<213>  Artificial sequence

<220>
<223>  Single strand DNA oligonucleotide

<400>  218
gcgacaacat cagaaaacga                                                    20


<210>  219
<211>  20
<212>  DNA
<213>  Artificial sequence

<220>
<223>  Single strand DNA oligonucleotide

<400>  219
agctagatcg ccactggaaa                                                    20


<210>  220
<211>  20
<212>  DNA
<213>  Artificial sequence

<220>

<223>  Single strand DNA oligonucleotide

<400>  220
aggtgcagaa atgagcgagt                                                    20


<210>  221
<211>  21
<212>  DNA
<213>  Artificial sequence

<220>
<223>  Single strand DNA oligonucleotide

<400>  221
ggttttacga catcctcatc g                                                  21


<210>  222
<211>  20
<212>  DNA
<213>  Artificial sequence

<220>
<223>  Single strand DNA oligonucleotide

<400>  222
actttgggag aagtggctca                                                    20


<210>  223
<211>  20
<212>  DNA
<213>  Artificial sequence

<220>
<223>  Single strand DNA oligonucleotide

<400>  223
ttggtcaggg tgtcgaattt                                                    20


<210>  224
<211>  20
<212>  DNA
<213>  Artificial sequence

<220>
<223>  Single strand DNA oligonucleotide

<400>  224
tgcaaaacag agcaggaaaa                                                    20


<210>  225
<211>  20
<212>  DNA
<213>  Artificial sequence

<220>
<223>  Single strand DNA oligonucleotide

<400>  225
ggcggatatt cccaatcaat                                                    20


<210>  226
<211>  20

<212>  DNA
<213>  Artificial sequence

<220>
<223>  Single strand DNA oligonucleotide

<400>  226
gtggaacaaa tggcgatgta                                                    20


<210>  227
<211>  20
<212>  DNA
<213>  Artificial sequence

<220>
<223>  Single strand DNA oligonucleotide

<400>  227
cggcattttt gctcctttag                                                    20


<210>  228
<211>  20
<212>  DNA
<213>  Artificial sequence

<220>
<223>  Single strand DNA oligonucleotide

<400>  228
tgcaaaacag agcaggaaaa                                                    20


<210>  229
<211>  20
<212>  DNA
<213>  Artificial sequence

<220>
<223>  Single strand DNA oligonucleotide

<400>  229
caccgataca atctgcttgc                                                    20


<210>  230
<211>  20
<212>  DNA
<213>  Artificial sequence

<220>
<223>  Single strand DNA oligonucleotide

<400>  230
atctgctgca aaacagagca                                                    20


<210>  231
<211>  19
<212>  DNA
<213>  Artificial sequence

<220>
<223>  Single strand DNA oligonucleotide

<400>  231
gagtggcggt acagggatt                                                     19

<210> 232
<211> 19
<212> DNA
<213> Artificial sequence

<220>
<223> Single strand DNA oligonucleotide

<400> 232
gcagggcttg gagatcatt 19


<210> 233
<211> 20
<212> DNA
<213> Artificial sequence

<220>
<223> Single strand DNA oligonucleotide

<400> 233
gagggcggga tttctacttc 20


<210> 234
<211> 20
<212> DNA
<213> Artificial sequence

<220>
<223> Single strand DNA oligonucleotide

<400> 234
agccaggcaa aaggatacaa 20


<210> 235
<211> 20
<212> DNA
<213> Artificial sequence

<220>
<223> Single strand DNA oligonucleotide

<400> 235
catgccaatc catcactgaa 20


<210> 236
<211> 20
<212> DNA
<213> Artificial sequence

<220>
<223> Single strand DNA oligonucleotide

<400> 236
cattcgtgca agcaagagaa 20


<210> 237
<211> 20
<212> DNA
<213> Artificial sequence

<220>

<223>  Single strand DNA oligonucleotide

<400>  237
tttcaagacc tgcaccttca                                                    20


<210>  238
<211>  20
<212>  DNA
<213>  Artificial sequence

<220>
<223>  Single strand DNA oligonucleotide

<400>  238
aattatggga gcaaggcaga                                                    20


<210>  239
<211>  20
<212>  DNA
<213>  Artificial sequence

<220>
<223>  Single strand DNA oligonucleotide

<400>  239
cctctaggat ccggctcaat                                                    20


<210>  240
<211>  20
<212>  DNA
<213>  Artificial sequence

<220>
<223>  Single strand DNA oligonucleotide

<400>  240
tgaaggtgca ggtcttgaaa                                                    20


<210>  241
<211>  20
<212>  DNA
<213>  Artificial sequence

<220>
<223>  Single strand DNA oligonucleotide

<400>  241
ggattcctca gcgctaactg                                                    20


<210>  242
<211>  20
<212>  DNA
<213>  Artificial sequence

<220>
<223>  Single strand DNA oligonucleotide

<400>  242
gcgcaacgga taaggataaa                                                    20


<210>  243
<211>  21

<212>  DNA
<213>  Artificial sequence

<220>
<223>  Single strand DNA oligonucleotide

<400>  243
ccgccatatc gttgttatac g                                                21


<210>  244
<211>  20
<212>  DNA
<213>  Artificial sequence

<220>
<223>  Single strand DNA oligonucleotide

<400>  244
ggcattaacc cgtcttctga                                                  20


<210>  245
<211>  20
<212>  DNA
<213>  Artificial sequence

<220>
<223>  Single strand DNA oligonucleotide

<400>  245
ctgttttcgg aaatgcctgt                                                  20


<210>  246
<211>  20
<212>  DNA
<213>  Artificial sequence

<220>
<223>  Single strand DNA oligonucleotide

<400>  246
gggcagacta tcaggcagag                                                  20


<210>  247
<211>  20
<212>  DNA
<213>  Artificial sequence

<220>
<223>  Single strand DNA oligonucleotide

<400>  247
tgctgtgcgt aattgtggat                                                  20


<210>  248
<211>  20
<212>  DNA
<213>  Artificial sequence

<220>
<223>  Single strand DNA oligonucleotide

<400>  248
gggcagacta tcaggcagag                                                  20

<210>  249
<211>  20
<212>  DNA
<213>  Artificial sequence

<220>
<223>  Single strand DNA oligonucleotide

<400>  249
cgagtttgat acgcccttct                                                    20


<210>  250
<211>  20
<212>  DNA
<213>  Artificial sequence

<220>
<223>  Single strand DNA oligonucleotide

<400>  250
ggaggttcag caacaacgat                                                    20


<210>  251
<211>  20
<212>  DNA
<213>  Artificial sequence

<220>
<223>  Single strand DNA oligonucleotide

<400>  251
gctcgtcaac acttccttcc                                                    20


<210>  252
<211>  20
<212>  DNA
<213>  Artificial sequence

<220>
<223>  Single strand DNA oligonucleotide

<400>  252
caattacgca cagcaactgg                                                    20


<210>  253
<211>  20
<212>  DNA
<213>  Artificial sequence

<220>
<223>  Single strand DNA oligonucleotide

<400>  253
cgagtttgat acgcccttct                                                    20


<210>  254
<211>  19
<212>  DNA
<213>  Artificial sequence

<220>

<223>  Single strand DNA oligonucleotide

<400>  254
   acgctccccc tgtaaaaca                                                    19


<210>  255
<211>  20
<212>  DNA
<213>  Artificial sequence

<220>
<223>  Single strand DNA oligonucleotide

<400>  255
ggatgtgatc catctggtga                                                  20


<210>  256
<211>  20
<212>  DNA
<213>  Artificial sequence

<220>
<223>  Single strand DNA oligonucleotide

<400>  256
gaggcataaa cccatgctgt                                                  20


<210>  257
<211>  20
<212>  DNA
<213>  Artificial sequence

<220>
<223>  Single strand DNA oligonucleotide

<400>  257
atggatttgc ctgtctgacc                                                  20


<210>  258
<211>  20
<212>  DNA
<213>  Artificial sequence

<220>
<223>  Single strand DNA oligonucleotide

<400>  258
tggcataacc gatgaacaga                                                  20


<210>  259
<211>  20
<212>  DNA
<213>  Artificial sequence

<220>
<223>  Single strand DNA oligonucleotide

<400>  259
agacaacggg ctgtagcatt                                                  20


<210>  260
<211>  20

<212>  DNA
<213>  Artificial sequence

<220>
<223>  Single strand DNA oligonucleotide

<400>  260
cgtacctgga ggcataaacc                                                    20


<210>  261
<211>  20
<212>  DNA
<213>  Artificial sequence

<220>
<223>  Single strand DNA oligonucleotide

<400>  261
ggatgtgatc catctggtga                                                    20


<210>  262
<211>  24
<212>  DNA
<213>  Artificial sequence

<220>
<223>  Single strand DNA oligonucleotide

<400>  262
aacagttcct gagtgtaatc acca                                               24


<210>  263
<211>  20
<212>  DNA
<213>  Artificial sequence

<220>
<223>  Single strand DNA oligonucleotide

<400>  263
actaatgcga gtgcttgctg                                                    20


<210>  264
<211>  20
<212>  DNA
<213>  Artificial sequence

<220>
<223>  Single strand DNA oligonucleotide

<400>  264
atgttccgat tcgcaatgtt                                                    20


<210>  265
<211>  20
<212>  DNA
<213>  Artificial sequence

<220>
<223>  Single strand DNA oligonucleotide

<400>  265
ccagcaatcg tttgtgtttg                                                    20

<210>  266
<211>  20
<212>  DNA
<213>  Artificial sequence

<220>
<223>  Single strand DNA oligonucleotide

<400>  266
attcgcccga acatacaaac                                                    20


<210>  267
<211>  20
<212>  DNA
<213>  Artificial sequence

<220>
<223>  Single strand DNA oligonucleotide

<400>  267
gccaatatga ctcacccaca                                                    20


<210>  268
<211>  20
<212>  DNA
<213>  Artificial sequence

<220>
<223>  Single strand DNA oligonucleotide

<400>  268
aatatacccg ctggtccaaa                                                    20


<210>  269
<211>  20
<212>  DNA
<213>  Artificial sequence

<220>
<223>  Single strand DNA oligonucleotide

<400>  269
ccgggtatgg aaatcacttg                                                    20


<210>  270
<211>  20
<212>  DNA
<213>  Artificial sequence

<220>
<223>  Single strand DNA oligonucleotide

<400>  270
atgttccgat tcgcaatgtt                                                    20


<210>  271
<211>  20
<212>  DNA
<213>  Artificial sequence

<220>

<223>  Single strand DNA oligonucleotide

<400>  271
actaatgcga gtgcttgctg                                                    20


<210>  272
<211>  20
<212>  DNA
<213>  Artificial sequence

<220>
<223>  Single strand DNA oligonucleotide

<400>  272
attcgcccga acatacaaac                                                    20


<210>  273
<211>  20
<212>  DNA
<213>  Artificial sequence

<220>
<223>  Single strand DNA oligonucleotide

<400>  273
actaatgcga gtgcttgctg                                                    20


<210>  274
<211>  20
<212>  DNA
<213>  Artificial sequence

<220>
<223>  Single strand DNA oligonucleotide

<400>  274
gtactttcac ctcccgacca                                                    20


<210>  275
<211>  20
<212>  DNA
<213>  Artificial sequence

<220>
<223>  Single strand DNA oligonucleotide

<400>  275
caatgacatg gacgctggta                                                    20


<210>  276
<211>  20
<212>  DNA
<213>  Artificial sequence

<220>
<223>  Single strand DNA oligonucleotide

<400>  276
gcaccctatc cattcaccat                                                    20


<210>  277
<211>  20

<212>  DNA
<213>  Artificial sequence

<220>
<223>  Single strand DNA oligonucleotide

<400>  277
gcatcgccag cgttattatt                                                    20


<210>  278
<211>  20
<212>  DNA
<213>  Artificial sequence

<220>
<223>  Single strand DNA oligonucleotide

<400>  278
tgccagtcag tgagctatgg                                                    20


<210>  279
<211>  20
<212>  DNA
<213>  Artificial sequence

<220>
<223>  Single strand DNA oligonucleotide

<400>  279
aaagccatta aggcgtaggg                                                    20


<210>  280
<211>  20
<212>  DNA
<213>  Artificial sequence

<220>
<223>  Single strand DNA oligonucleotide

<400>  280
aataataacg ctggcgatgc                                                    20


<210>  281
<211>  20
<212>  DNA
<213>  Artificial sequence

<220>
<223>  Single strand DNA oligonucleotide

<400>  281
tgccatagct cactgactgg                                                    20


<210>  282
<211>  20
<212>  DNA
<213>  Artificial sequence

<220>
<223>  Single strand DNA oligonucleotide

<400>  282
ggtagcacca gtcaggctgt                                                    20

<210>  283
<211>  20
<212>  DNA
<213>  Artificial sequence

<220>
<223>  Single strand DNA oligonucleotide

<400>  283
cggaattgaa aacctctgct                                                    20


<210>  284
<211>  20
<212>  DNA
<213>  Artificial sequence

<220>
<223>  Single strand DNA oligonucleotide

<400>  284
tgaaagttcg ttcagcttgc                                                    20


<210>  285
<211>  20
<212>  DNA
<213>  Artificial sequence

<220>
<223>  Single strand DNA oligonucleotide

<400>  285
gcatcgccag cgttattatt                                                    20


<210>  286
<211>  20
<212>  DNA
<213>  Artificial sequence

<220>
<223>  Single strand DNA oligonucleotide

<400>  286
ggtagcacca gtcaggctgt                                                    20


<210>  287
<211>  21
<212>  DNA
<213>  Artificial sequence

<220>
<223>  Single strand DNA oligonucleotide

<400>  287
gcccagtatg atgtccagaa a                                                  21


<210>  288
<211>  21
<212>  DNA
<213>  Artificial sequence

<220>

<223>  Single strand DNA oligonucleotide

<400>  288
catcctggaa cagactgggt a                                                21


<210>  289
<211>  20
<212>  DNA
<213>  Artificial sequence

<220>
<223>  Single strand DNA oligonucleotide

<400>  289
ataaaaccaa tcggcccaac                                                  20


<210>  290
<211>  20
<212>  DNA
<213>  Artificial sequence

<220>
<223>  Single strand DNA oligonucleotide

<400>  290
catggcaact tacggcatta                                                  20


<210>  291
<211>  20
<212>  DNA
<213>  Artificial sequence

<220>
<223>  Single strand DNA oligonucleotide

<400>  291
cgtaacaggg aggaaagacg                                                  20


<210>  292
<211>  20
<212>  DNA
<213>  Artificial sequence

<220>
<223>  Single strand DNA oligonucleotide

<400>  292
acgcatggga gaagaaagag                                                  20


<210>  293
<211>  20
<212>  DNA
<213>  Artificial sequence

<220>
<223>  Single strand DNA oligonucleotide

<400>  293
cgataatgct gcaagcaaac                                                  20


<210>  294
<211>  20

<212>  DNA
<213>  Artificial sequence

<220>
<223>  Single strand DNA oligonucleotide

<400>  294
acagcaactg cgtctttcct                                                    20


<210>  295
<211>  20
<212>  DNA
<213>  Artificial sequence

<220>
<223>  Single strand DNA oligonucleotide

<400>  295
ataaaaccaa tcggcccaac                                                    20


<210>  296
<211>  20
<212>  DNA
<213>  Artificial sequence

<220>
<223>  Single strand DNA oligonucleotide

<400>  296
ccaataggca tcgtcacctc                                                    20


<210>  297
<211>  21
<212>  DNA
<213>  Artificial sequence

<220>
<223>  Single strand DNA oligonucleotide

<400>  297
gccagatgtt ctatccccag t                                                  21


<210>  298
<211>  20
<212>  DNA
<213>  Artificial sequence

<220>
<223>  Single strand DNA oligonucleotide

<400>  298
aatggttgcg gtaaatcgac                                                    20


<210>  299
<211>  20
<212>  DNA
<213>  Artificial sequence

<220>
<223>  Single strand DNA oligonucleotide

<400>  299
tggcatttgc attaggttga                                                    20

<210>  300
<211>  20
<212>  DNA
<213>  Artificial sequence

<220>
<223>  Single strand DNA oligonucleotide

<400>  300
attctgggac caaatggttg                                                    20


<210>  301
<211>  20
<212>  DNA
<213>  Artificial sequence

<220>
<223>  Single strand DNA oligonucleotide

<400>  301
tggcatttgc attaggttga                                                    20


<210>  302
<211>  20
<212>  DNA
<213>  Artificial sequence

<220>
<223>  Single strand DNA oligonucleotide

<400>  302
tacctgaact gcaacgagga                                                    20


<210>  303
<211>  20
<212>  DNA
<213>  Artificial sequence

<220>
<223>  Single strand DNA oligonucleotide

<400>  303
aatgtcgatt taccgcaacc                                                    20


<210>  304
<211>  20
<212>  DNA
<213>  Artificial sequence

<220>
<223>  Single strand DNA oligonucleotide

<400>  304
cgctatcgca ggagtttgtt                                                    20


<210>  305
<211>  20
<212>  DNA
<213>  Artificial sequence

<220>

<223>  Single strand DNA oligonucleotide

<400>  305
cgcgtaggga aaccagaata                                                    20


<210>  306
<211>  20
<212>  DNA
<213>  Artificial sequence

<220>
<223>  Single strand DNA oligonucleotide

<400>  306
cgctatcgca ggagtttgtt                                                    20


<210>  307
<211>  18
<212>  DNA
<213>  Artificial sequence

<220>
<223>  Single strand DNA oligonucleotide

<400>  307
atctttgacg cgcgtagg                                                      18


<210>  308
<211>  20
<212>  DNA
<213>  Artificial sequence

<220>
<223>  Single strand DNA oligonucleotide

<400>  308
cgctatcgca ggagtttgtt                                                    20


<210>  309
<211>  18
<212>  DNA
<213>  Artificial sequence

<220>
<223>  Single strand DNA oligonucleotide

<400>  309
atgtttcccg cccatctt                                                      18


<210>  310
<211>  23
<212>  DNA
<213>  Artificial sequence

<220>
<223>  Single strand DNA oligonucleotide

<400>  310
ccatcagttc catgttatgg att                                                23


<210>  311
<211>  20

<212>  DNA
<213>  Artificial sequence

<220>
<223>  Single strand DNA oligonucleotide

<400>  311
ctctgatgta tccggtgtgc                                                    20


<210>  312
<211>  20
<212>  DNA
<213>  Artificial sequence

<220>
<223>  Single strand DNA oligonucleotide

<400>  312
tacaatgaat gccctgttgg                                                    20


<210>  313
<211>  20
<212>  DNA
<213>  Artificial sequence

<220>
<223>  Single strand DNA oligonucleotide

<400>  313
atggccagtg agcgtaaaaa                                                    20


<210>  314
<211>  23
<212>  DNA
<213>  Artificial sequence

<220>
<223>  Single strand DNA oligonucleotide

<400>  314
ccatcagttc catgttatgg att                                                23


<210>  315
<211>  19
<212>  DNA
<213>  Artificial sequence

<220>
<223>  Single strand DNA oligonucleotide

<400>  315
ggtgcattga ttccacgtc                                                     19


<210>  316
<211>  20
<212>  DNA
<213>  Artificial sequence

<220>
<223>  Single strand DNA oligonucleotide

<400>  316
ccgtggacga atagagcatt                                                    20

<210>  317
<211>  20
<212>  DNA
<213>  Artificial sequence

<220>
<223>  Single strand DNA oligonucleotide

<400>  317
tgagaagttc cgggagagaa                                                    20


<210>  318
<211>  20
<212>  DNA
<213>  Artificial sequence

<220>
<223>  Single strand DNA oligonucleotide

<400>  318
agttgctgct gacgaccttc                                                    20


<210>  319
<211>  20
<212>  DNA
<213>  Artificial sequence

<220>
<223>  Single strand DNA oligonucleotide

<400>  319
tttttggtgc attgattcca                                                    20


<210>  320
<211>  20
<212>  DNA
<213>  Artificial sequence

<220>
<223>  Single strand DNA oligonucleotide

<400>  320
ctgtggttga ggtttgagca                                                    20


<210>  321
<211>  20
<212>  DNA
<213>  Artificial sequence

<220>
<223>  Single strand DNA oligonucleotide

<400>  321
atggccagtg agcgtaaaaa                                                    20


<210>  322
<211>  20
<212>  DNA
<213>  Artificial sequence

<220>

<223>  Single strand DNA oligonucleotide

<400>  322
cctctagttg ctgctgacga                                                    20


<210>  323
<211>  20
<212>  DNA
<213>  Artificial sequence

<220>
<223>  Single strand DNA oligonucleotide

<400>  323
tttttggtgc attgattcca                                                    20


<210>  324
<211>  20
<212>  DNA
<213>  Artificial sequence

<220>
<223>  Single strand DNA oligonucleotide

<400>  324
cggtttcgat acgctctttt                                                    20


<210>  325
<211>  20
<212>  DNA
<213>  Artificial sequence

<220>
<223>  Single strand DNA oligonucleotide

<400>  325
atgggttttg gatctgaacg                                                    20


<210>  326
<211>  20
<212>  DNA
<213>  Artificial sequence

<220>
<223>  Single strand DNA oligonucleotide

<400>  326
ggtctcggaa gatcgagaaa                                                    20


<210>  327
<211>  20
<212>  DNA
<213>  Artificial sequence

<220>
<223>  Single strand DNA oligonucleotide

<400>  327
ccgggcaaaa gtatcaaaaa                                                    20


<210>  328
<211>  20

<212>  DNA
<213>  Artificial sequence

<220>
<223>  Single strand DNA oligonucleotide

<400>  328
tggaatcaat gcaccaaaaa                                                    20


<210>  329
<211>  20
<212>  DNA
<213>  Artificial sequence

<220>
<223>  Single strand DNA oligonucleotide

<400>  329
ccgggcaaaa gtatcaaaaa                                                    20


<210>  330
<211>  20
<212>  DNA
<213>  Artificial sequence

<220>
<223>  Single strand DNA oligonucleotide

<400>  330
ttctctcccg gaacttctca                                                    20


<210>  331
<211>  20
<212>  DNA
<213>  Artificial sequence

<220>
<223>  Single strand DNA oligonucleotide

<400>  331
aaatacgggg aattgtgtgg                                                    20


<210>  332
<211>  20
<212>  DNA
<213>  Artificial sequence

<220>
<223>  Single strand DNA oligonucleotide

<400>  332
gtggaatcaa tgcaccaaaa                                                    20


<210>  333
<211>  20
<212>  DNA
<213>  Artificial sequence

<220>
<223>  Single strand DNA oligonucleotide

<400>  333
ccgggcaaaa gtatcaaaaa                                                    20

<210>  334
<211>  20
<212>  DNA
<213>  Artificial sequence

<220>
<223>  Single strand DNA oligonucleotide

<400>  334
ccgtaacggt caaaaatcgt                                                    20


<210>  335
<211>  20
<212>  DNA
<213>  Artificial sequence

<220>
<223>  Single strand DNA oligonucleotide

<400>  335
ttttgttcac cacgacctca                                                    20


<210>  336
<211>  20
<212>  DNA
<213>  Artificial sequence

<220>
<223>  Single strand DNA oligonucleotide

<400>  336
aaaaatcgtg gcgttgacac                                                    20


<210>  337
<211>  20
<212>  DNA
<213>  Artificial sequence

<220>
<223>  Single strand DNA oligonucleotide

<400>  337
ttttgttcac cacgacctca                                                    20


<210>  338
<211>  20
<212>  DNA
<213>  Artificial sequence

<220>
<223>  Single strand DNA oligonucleotide

<400>  338
gtatcgactg gtggcatctg                                                    20


<210>  339
<211>  20
<212>  DNA
<213>  Artificial sequence

<220>

<223>  Single strand DNA oligonucleotide

<400>  339
tgactgcctt tctcccactt                                                    20


<210>  340
<211>  20
<212>  DNA
<213>  Artificial sequence

<220>
<223>  Single strand DNA oligonucleotide

<400>  340
ggcatctgga gatacgcttt                                                    20


<210>  341
<211>  20
<212>  DNA
<213>  Artificial sequence

<220>
<223>  Single strand DNA oligonucleotide

<400>  341
tgactgcctt tctcccactt                                                    20


<210>  342
<211>  21
<212>  DNA
<213>  Artificial sequence

<220>
<223>  Single strand DNA oligonucleotide

<400>  342
tgctttttga tgatggaaac a                                                  21


<210>  343
<211>  20
<212>  DNA
<213>  Artificial sequence

<220>
<223>  Single strand DNA oligonucleotide

<400>  343
caaacgggga atgtagcaat                                                    20


<210>  344
<211>  21
<212>  DNA
<213>  Artificial sequence

<220>
<223>  Single strand DNA oligonucleotide

<400>  344
tgcattgcgg ttttaatctt t                                                  21


<210>  345
<211>  21

<212>  DNA
<213>  Artificial sequence

<220>
<223>  Single strand DNA oligonucleotide

<400>  345
agccaagcca atttatttca a                                                21


<210>  346
<211>  20
<212>  DNA
<213>  Artificial sequence

<220>
<223>  Single strand DNA oligonucleotide

<400>  346
gtatcgactg gtggcatctg                                                  20


<210>  347
<211>  20
<212>  DNA
<213>  Artificial sequence

<220>
<223>  Single strand DNA oligonucleotide

<400>  347
cagcatgact gcctttctcc                                                  20


<210>  348
<211>  22
<212>  DNA
<213>  Artificial sequence

<220>
<223>  Single strand DNA oligonucleotide

<400>  348
aatgcattgc ggttttaatc tt                                               22


<210>  349
<211>  20
<212>  DNA
<213>  Artificial sequence

<220>
<223>  Single strand DNA oligonucleotide

<400>  349
ttgaatagcc aagccaattt                                                  20


<210>  350
<211>  20
<212>  DNA
<213>  Artificial sequence

<220>
<223>  Single strand DNA oligonucleotide

<400>  350
ctgcagcatt gacgatttgt                                                  20

<210> 351
<211> 20
<212> DNA
<213> Artificial sequence

<220>
<223> Single strand DNA oligonucleotide

<400> 351
caaacgggga atgtagcaat 20


<210> 352
<211> 20
<212> DNA
<213> Artificial sequence

<220>
<223> Single strand DNA oligonucleotide

<400> 352
tgcagcattg acgatttgtt 20


<210> 353
<211> 19
<212> DNA
<213> Artificial sequence

<220>
<223> Single strand DNA oligonucleotide

<400> 353
tatcacccaa acggggaat 19


<210> 354
<211> 25
<212> DNA
<213> Artificial sequence

<220>
<223> Single strand DNA oligonucleotide

<400> 354
cggacataaa tatctcaaaa tgaca 25


<210> 355
<211> 20
<212> DNA
<213> Artificial sequence

<220>
<223> Single strand DNA oligonucleotide

<400> 355
caaacgggga atgtagcaat 20


<210> 356
<211> 20
<212> DNA
<213> Artificial sequence

<220>

<223>  Single strand DNA oligonucleotide

<400>  356
gagcgtctgc tcaaacaggt                                                    20


<210>  357
<211>  19
<212>  DNA
<213>  Artificial sequence

<220>
<223>  Single strand DNA oligonucleotide

<400>  357
gtagtgggtg aggggctgt                                                     19


<210>  358
<211>  21
<212>  DNA
<213>  Artificial sequence

<220>
<223>  Single strand DNA oligonucleotide

<400>  358
tcaaacaggt tatccgtcag g                                                  21


<210>  359
<211>  19
<212>  DNA
<213>  Artificial sequence

<220>
<223>  Single strand DNA oligonucleotide

<400>  359
gtagtgggtg aggggctgt                                                     19


<210>  360
<211>  20
<212>  DNA
<213>  Artificial sequence

<220>
<223>  Single strand DNA oligonucleotide

<400>  360
tccgtcagga agaggaaaaa                                                    20


<210>  361
<211>  20
<212>  DNA
<213>  Artificial sequence

<220>
<223>  Single strand DNA oligonucleotide

<400>  361
cagagcgtca agcaaccttt                                                    20


<210>  362
<211>  20

<212>  DNA
<213>  Artificial sequence

<220>
<223>  Single strand DNA oligonucleotide

<400>  362
tccgtcagga agaggaaaaa                                                    20


<210>  363
<211>  20
<212>  DNA
<213>  Artificial sequence

<220>
<223>  Single strand DNA oligonucleotide

<400>  363
ccttacctat ttccgctggt                                                    20


<210>  364
<211>  20
<212>  DNA
<213>  Artificial sequence

<220>
<223>  Single strand DNA oligonucleotide

<400>  364
ttgaaaacca gagccttgct                                                    20


<210>  365
<211>  22
<212>  DNA
<213>  Artificial sequence

<220>
<223>  Single strand DNA oligonucleotide

<400>  365
cgttgaccat aagaaaacct ca                                                 22


<210>  366
<211>  21
<212>  DNA
<213>  Artificial sequence

<220>
<223>  Single strand DNA oligonucleotide

<400>  366
aatgaaaaag gaaacgccat a                                                  21


<210>  367
<211>  22
<212>  DNA
<213>  Artificial sequence

<220>
<223>  Single strand DNA oligonucleotide

<400>  367
cgttgaccat aagaaaacct ca                                                 22

<210>  368
<211>  20
<212>  DNA
<213>  Artificial sequence

<220>
<223>  Single strand DNA oligonucleotide

<400>  368
ttttctgctg caagcacttc                                                    20


<210>  369
<211>  20
<212>  DNA
<213>  Artificial sequence

<220>
<223>  Single strand DNA oligonucleotide

<400>  369
ggcccgttta tcagaaaggt                                                    20


<210>  370
<211>  21
<212>  DNA
<213>  Artificial sequence

<220>
<223>  Single strand DNA oligonucleotide

<400>  370
aaaagggaag gccttatgat g                                                  21


<210>  371
<211>  21
<212>  DNA
<213>  Artificial sequence

<220>
<223>  Single strand DNA oligonucleotide

<400>  371
tccaaaatgg gaaagaagag g                                                  21


<210>  372
<211>  22
<212>  DNA
<213>  Artificial sequence

<220>
<223>  Single strand DNA oligonucleotide

<400>  372
tttcatagga agtggaggtg gt                                                 22


<210>  373
<211>  20
<212>  DNA
<213>  Artificial sequence

<220>

<223>  Single strand DNA oligonucleotide

<400>  373
aaaatggccc gtttatcaga                                                    20


<210>  374
<211>  20
<212>  DNA
<213>  Artificial sequence

<220>
<223>  Single strand DNA oligonucleotide

<400>  374
ttttctgctg caagcacttc                                                    20


<210>  375
<211>  20
<212>  DNA
<213>  Artificial sequence

<220>
<223>  Single strand DNA oligonucleotide

<400>  375
ccaaaatggg aaagaagagg                                                    20


<210>  376
<211>  21
<212>  DNA
<213>  Artificial sequence

<220>
<223>  Single strand DNA oligonucleotide

<400>  376
ccttgcttag acctgtgtcc a                                                  21


<210>  377
<211>  20
<212>  DNA
<213>  Artificial sequence

<220>
<223>  Single strand DNA oligonucleotide

<400>  377
ggcccgttta tcagaaaggt                                                    20


<210>  378
<211>  20
<212>  DNA
<213>  Artificial sequence

<220>
<223>  Single strand DNA oligonucleotide

<400>  378
acgtgggttg gatcattgtt                                                    20


<210>  379
<211>  20

<212>  DNA
<213>  Artificial sequence

<220>
<223>  Single strand DNA oligonucleotide

<400>  379
aaaatggccc gtttatcaga                                                    20


<210>  380
<211>  21
<212>  DNA
<213>  Artificial sequence

<220>
<223>  Single strand DNA oligonucleotide

<400>  380
gggtctcgat ttgatgattg a                                                  21


<210>  381
<211>  22
<212>  DNA
<213>  Artificial sequence

<220>
<223>  Single strand DNA oligonucleotide

<400>  381
ggctttaccg tggtagtact gg                                                 22


<210>  382
<211>  20
<212>  DNA
<213>  Artificial sequence

<220>
<223>  Single strand DNA oligonucleotide

<400>  382
tcttggtagg gacgtggttt                                                    20


<210>  383
<211>  22
<212>  DNA
<213>  Artificial sequence

<220>
<223>  Single strand DNA oligonucleotide

<400>  383
ggctttaccg tggtagtact gg                                                 22


<210>  384
<211>  20
<212>  DNA
<213>  Artificial sequence

<220>
<223>  Single strand DNA oligonucleotide

<400>  384
   cttcattttg gcctctttgc                                                     20

<210>  385
<211>  20
<212>  DNA
<213>  Artificial sequence

<220>
<223>  Single strand DNA oligonucleotide

<400>  385
ttgggtagcc acatccctta                                                    20


<210>  386
<211>  20
<212>  DNA
<213>  Artificial sequence

<220>
<223>  Single strand DNA oligonucleotide

<400>  386
gatgtggcta cccaagcaat                                                    20


<210>  387
<211>  20
<212>  DNA
<213>  Artificial sequence

<220>
<223>  Single strand DNA oligonucleotide

<400>  387
cacccaagga ctgaaggaag                                                    20


<210>  388
<211>  20
<212>  DNA
<213>  Artificial sequence

<220>
<223>  Single strand DNA oligonucleotide

<400>  388
gggcatgggc atacttatca                                                    20


<210>  389
<211>  20
<212>  DNA
<213>  Artificial sequence

<220>
<223>  Single strand DNA oligonucleotide

<400>  389
atttcccctc aattccttcg                                                    20


<210>  390
<211>  20
<212>  DNA
<213>  Artificial sequence

<220>

<223>  Single strand DNA oligonucleotide

<400>  390
tgtgcacctt agtcgctttg                                                    20


<210>  391
<211>  20
<212>  DNA
<213>  Artificial sequence

<220>
<223>  Single strand DNA oligonucleotide

<400>  391
atttcccctc aattccttcg                                                    20


<210>  392
<211>  20
<212>  DNA
<213>  Artificial sequence

<220>
<223>  Single strand DNA oligonucleotide

<400>  392
cttcattttg gcctctttgc                                                    20


<210>  393
<211>  21
<212>  DNA
<213>  Artificial sequence

<220>
<223>  Single strand DNA oligonucleotide

<400>  393
   tctgagcgat ttttcttgag c                                                   21


<210>  394
<211>  21
<212>  DNA
<213>  Artificial sequence

<220>
<223>  Single strand DNA oligonucleotide

<400>  394
atggcggtag ctcatacctt t                                                  21


<210>  395
<211>  20
<212>  DNA
<213>  Artificial sequence

<220>
<223>  Single strand DNA oligonucleotide

<400>  395
atttcccctc aattccttcg                                                    20


<210>  396
<211>  22

<212>  DNA
<213>  Artificial sequence

<220>
<223>  Single strand DNA oligonucleotide

<400>  396
cgccccatag tatattggac at                                                22


<210>  397
<211>  20
<212>  DNA
<213>  Artificial sequence

<220>
<223>  Single strand DNA oligonucleotide

<400>  397
acgatatcag cgaaggtgct                                                   20


<210>  398
<211>  19
<212>  DNA
<213>  Artificial sequence

<220>
<223>  Single strand DNA oligonucleotide

<400>  398
tccaggtcgc cccatagta                                                    19


<210>  399
<211>  20
<212>  DNA
<213>  Artificial sequence

<220>
<223>  Single strand DNA oligonucleotide

<400>  399
gctgcactgg tatccgattt                                                   20


<210>  400
<211>  20
<212>  DNA
<213>  Artificial sequence

<220>
<223>  Single strand DNA oligonucleotide

<400>  400
ggggctcctg attcataacc                                                   20


<210>  401
<211>  20
<212>  DNA
<213>  Artificial sequence

<220>
<223>  Single strand DNA oligonucleotide

<400>  401
acgatatcag cgaaggtgct                                                   20

<210>  402
<211>  20
<212>  DNA
<213>  Artificial sequence

<220>
<223>  Single strand DNA oligonucleotide

<400>  402
caagccaaat gctgacaaaa                                                    20


<210>  403
<211>  20
<212>  DNA
<213>  Artificial sequence

<220>
<223>  Single strand DNA oligonucleotide

<400>  403
attaagaacc gacgccagtg                                                    20


<210>  404
<211>  20
<212>  DNA
<213>  Artificial sequence

<220>
<223>  Single strand DNA oligonucleotide

<400>  404
cggttctggc tcaggtagtt                                                    20


<210>  405
<211>  20
<212>  DNA
<213>  Artificial sequence

<220>
<223>  Single strand DNA oligonucleotide

<400>  405
tgaatcccac agcgtcatta                                                    20


<210>  406
<211>  20
<212>  DNA
<213>  Artificial sequence

<220>
<223>  Single strand DNA oligonucleotide

<400>  406
ttatttagag ccgcgctgac                                                    20


<210>  407
<211>  20
<212>  DNA
<213>  Artificial sequence

<220>

<223>  Single strand DNA oligonucleotide

<400>  407
gcgaagatcc tctggtaacg                                                    20


<210>  408
<211>  20
<212>  DNA
<213>  Artificial sequence

<220>
<223>  Single strand DNA oligonucleotide

<400>  408
agcatccccc ttgttattga                                                    20


<210>  409
<211>  20
<212>  DNA
<213>  Artificial sequence

<220>
<223>  Single strand DNA oligonucleotide

<400>  409
gcaccagcag tacatcgaga                                                    20


<210>  410
<211>  20
<212>  DNA
<213>  Artificial sequence

<220>
<223>  Single strand DNA oligonucleotide

<400>  410
atacccgctt tctcaagtgc                                                    20


<210>  411
<211>  20
<212>  DNA
<213>  Artificial sequence

<220>
<223>  Single strand DNA oligonucleotide

<400>  411
gctacgtgct ggggtatctc                                                    20


<210>  412
<211>  20
<212>  DNA
<213>  Artificial sequence

<220>
<223>  Single strand DNA oligonucleotide

<400>  412
cgggccatac atccagtaat                                                    20


<210>  413
<211>  20

<212>  DNA
<213>  Artificial sequence

<220>
<223>  Single strand DNA oligonucleotide

<400>  413
gtgttcggct tgcagctatc                                                    20


<210>  414
<211>  20
<212>  DNA
<213>  Artificial sequence

<220>
<223>  Single strand DNA oligonucleotide

<400>  414
ttctttagct tcgggccata                                                    20


<210>  415
<211>  19
<212>  DNA
<213>  Artificial sequence

<220>
<223>  Single strand DNA oligonucleotide

<400>  415
ctatcggggg cgtagagaa                                                     19


<210>  416
<211>  20
<212>  DNA
<213>  Artificial sequence

<220>
<223>  Single strand DNA oligonucleotide

<400>  416
taatgacgct gtgggattca                                                    20


<210>  417
<211>  20
<212>  DNA
<213>  Artificial sequence

<220>
<223>  Single strand DNA oligonucleotide

<400>  417
gatgtatggc ccgaagctaa                                                    20


<210>  418
<211>  20
<212>  DNA
<213>  Artificial sequence

<220>
<223>  Single strand DNA oligonucleotide

<400>  418
gtcaagccaa atgctgacaa                                                    20

<210>  419
<211>  20
<212>  DNA
<213>  Artificial sequence

<220>
<223>  Single strand DNA oligonucleotide

<400>  419
gcaccagcag tacatcgaga                                                    20


<210>  420
<211>  20
<212>  DNA
<213>  Artificial sequence

<220>
<223>  Single strand DNA oligonucleotide

<400>  420
cgggcaaaga ctacacacag                                                    20


<210>  421
<211>  24
<212>  DNA
<213>  Artificial sequence

<220>
<223>  Single strand DNA oligonucleotide

<400>  421
cagatcatag gtgtaatgat cgaa                                               24


<210>  422
<211>  20
<212>  DNA
<213>  Artificial sequence

<220>
<223>  Single strand DNA oligonucleotide

<400>  422
gtatgcagga aagcaccaca                                                    20


<210>  423
<211>  20
<212>  DNA
<213>  Artificial sequence

<220>
<223>  Single strand DNA oligonucleotide

<400>  423
aaaccgctca aaggtgaatg                                                    20


<210>  424
<211>  20
<212>  DNA
<213>  Artificial sequence

<220>

<223>  Single strand DNA oligonucleotide

<400>  424
atacgttttc acgccgtttc                                                    20


<210>  425
<211>  20
<212>  DNA
<213>  Artificial sequence

<220>
<223>  Single strand DNA oligonucleotide

<400>  425
cggcaatacg ggtcagtaag                                                    20


<210>  426
<211>  20
<212>  DNA
<213>  Artificial sequence

<220>
<223>  Single strand DNA oligonucleotide

<400>  426
atcgttttgg ctttggtgtc                                                    20


<210>  427
<211>  20
<212>  DNA
<213>  Artificial sequence

<220>
<223>  Single strand DNA oligonucleotide

<400>  427
cgtaaatatc gggaggcgta                                                    20


<210>  428
<211>  20
<212>  DNA
<213>  Artificial sequence

<220>
<223>  Single strand DNA oligonucleotide

<400>  428
tggtattgtg cgtacgtggt                                                    20


<210>  429
<211>  20
<212>  DNA
<213>  Artificial sequence

<220>
<223>  Single strand DNA oligonucleotide

<400>  429
cgtaaatatc gggaggcgta                                                    20


<210>  430
<211>  20

<212>  DNA
<213>  Artificial sequence

<220>
<223>  Single strand DNA oligonucleotide

<400>  430
ccctggtgca ggacatagat                                                    20


<210>  431
<211>  20
<212>  DNA
<213>  Artificial sequence

<220>
<223>  Single strand DNA oligonucleotide

<400>  431
agctccccat ggtttgctat                                                    20


<210>  432
<211>  20
<212>  DNA
<213>  Artificial sequence

<220>
<223>  Single strand DNA oligonucleotide

<400>  432
gacaacaaac tcccctggtg                                                    20


<210>  433
<211>  20
<212>  DNA
<213>  Artificial sequence

<220>
<223>  Single strand DNA oligonucleotide

<400>  433
ttgctatcgg acatgggtta                                                    20


<210>  434
<211>  20
<212>  DNA
<213>  Artificial sequence

<220>
<223>  Single strand DNA oligonucleotide

<400>  434
ctggttcgtc cactttcgat                                                    20


<210>  435
<211>  20
<212>  DNA
<213>  Artificial sequence

<220>
<223>  Single strand DNA oligonucleotide

<400>  435
gcacgtcgtt tggaattagg                                                    20

<210>  436
<211>  20
<212>  DNA
<213>  Artificial sequence

<220>
<223>  Single strand DNA oligonucleotide

<400>  436
agctctcctg cctgaacgta                                                    20


<210>  437
<211>  20
<212>  DNA
<213>  Artificial sequence

<220>
<223>  Single strand DNA oligonucleotide

<400>  437
agcccagact ggctactgaa                                                    20


<210>  438
<211>  20
<212>  DNA
<213>  Artificial sequence

<220>
<223>  Single strand DNA oligonucleotide

<400>  438
cgacccgagt aaaagaacga                                                    20


<210>  439
<211>  20
<212>  DNA
<213>  Artificial sequence

<220>
<223>  Single strand DNA oligonucleotide

<400>  439
gccgagcaat aacaccactt                                                    20


<210>  440
<211>  20
<212>  DNA
<213>  Artificial sequence

<220>
<223>  Single strand DNA oligonucleotide

<400>  440
agccagatcc agaagattgc                                                    20


<210>  441
<211>  20
<212>  DNA
<213>  Artificial sequence

<220>

<223>  Single strand DNA oligonucleotide

<400>  441
gccgagcaat aacaccactt                                                    20


<210>  442
<211>  20
<212>  DNA
<213>  Artificial sequence

<220>
<223>  Single strand DNA oligonucleotide

<400>  442
agctctcctg cctgaacgta                                                    20


<210>  443
<211>  20
<212>  DNA
<213>  Artificial sequence

<220>
<223>  Single strand DNA oligonucleotide

<400>  443
ggtcgctaag gtcattgctt                                                    20


<210>  444
<211>  20
<212>  DNA
<213>  Artificial sequence

<220>
<223>  Single strand DNA oligonucleotide

<400>  444
tcgatacgta atgcgaagca                                                    20


<210>  445
<211>  20
<212>  DNA
<213>  Artificial sequence

<220>
<223>  Single strand DNA oligonucleotide

<400>  445
ttttactcgg gtcgctaagg                                                    20


<210>  446
<211>  20
<212>  DNA
<213>  Artificial sequence

<220>
<223>  Single strand DNA oligonucleotide

<400>  446
ggcgtggtac atggatatga                                                    20


<210>  447
<211>  20

<212>  DNA
<213>  Artificial sequence

<220>
<223>  Single strand DNA oligonucleotide

<400>  447
gggcgacgaa tgtattcaaa                                                    20


<210>  448
<211>  20
<212>  DNA
<213>  Artificial sequence

<220>
<223>  Single strand DNA oligonucleotide

<400>  448
ccaggcgtgg tacatggata                                                    20


<210>  449
<211>  20
<212>  DNA
<213>  Artificial sequence

<220>
<223>  Single strand DNA oligonucleotide

<400>  449
gggcgacgaa tgtattcaaa                                                    20


<210>  450
<211>  20
<212>  DNA
<213>  Artificial sequence

<220>
<223>  Single strand DNA oligonucleotide

<400>  450
gcggtggcta cactatggtt                                                    20


<210>  451
<211>  20
<212>  DNA
<213>  Artificial sequence

<220>
<223>  Single strand DNA oligonucleotide

<400>  451
attggtaccc agttcggtga                                                    20


<210>  452
<211>  20
<212>  DNA
<213>  Artificial sequence

<220>
<223>  Single strand DNA oligonucleotide

<400>  452
atggcttcac ggttaaatgc                                                    20

<210>  453
<211>  20
<212>  DNA
<213>  Artificial sequence

<220>
<223>  Single strand DNA oligonucleotide

<400>  453
tggtacccag ttcggtgatt                                                    20


<210>  454
<211>  20
<212>  DNA
<213>  Artificial sequence

<220>
<223>  Single strand DNA oligonucleotide

<400>  454
ggcggatctg tatacgcaat                                                    20


<210>  455
<211>  20
<212>  DNA
<213>  Artificial sequence

<220>
<223>  Single strand DNA oligonucleotide

<400>  455
acgcagaaaa ggcagctaac                                                    20


<210>  456
<211>  20
<212>  DNA
<213>  Artificial sequence

<220>
<223>  Single strand DNA oligonucleotide

<400>  456
tgtatacgca atcggctttg                                                    20


<210>  457
<211>  20
<212>  DNA
<213>  Artificial sequence

<220>
<223>  Single strand DNA oligonucleotide

<400>  457
tggtacccag ttcggtgatt                                                    20


<210>  458
<211>  20
<212>  DNA
<213>  Artificial sequence

<220>

<223>  Single strand DNA oligonucleotide

<400>  458
tcatcggatc tttcccttgt                                                    20


<210>  459
<211>  20
<212>  DNA
<213>  Artificial sequence

<220>
<223>  Single strand DNA oligonucleotide

<400>  459
agcgggtttg aaaggatgta                                                    20


<210>  460
<211>  20
<212>  DNA
<213>  Artificial sequence

<220>
<223>  Single strand DNA oligonucleotide

<400>  460
agttgccaac cttgatcctg                                                    20


<210>  461
<211>  20
<212>  DNA
<213>  Artificial sequence

<220>
<223>  Single strand DNA oligonucleotide

<400>  461
aagcgggttt gaaaggatgt                                                    20


<210>  462
<211>  20
<212>  DNA
<213>  Artificial sequence

<220>
<223>  Single strand DNA oligonucleotide

<400>  462
tcatcggatc tttcccttgt                                                    20


<210>  463
<211>  20
<212>  DNA
<213>  Artificial sequence

<220>
<223>  Single strand DNA oligonucleotide

<400>  463
gaatcaggaa cggcttttg                                                     20


<210>  464
<211>  20

<212>  DNA
<213>  Artificial sequence

<220>
<223>  Single strand DNA oligonucleotide

<400>  464
cagttgccaa ccttgatcct                                                    20


<210>  465
<211>  20
<212>  DNA
<213>  Artificial sequence

<220>
<223>  Single strand DNA oligonucleotide

<400>  465
cctgctgaaa catggcaata                                                    20


<210>  466
<211>  20
<212>  DNA
<213>  Artificial sequence

<220>
<223>  Single strand DNA oligonucleotide

<400>  466
accgaaaaag agcaagagca                                                    20


<210>  467
<211>  20
<212>  DNA
<213>  Artificial sequence

<220>
<223>  Single strand DNA oligonucleotide

<400>  467
caatgcaaaa tcccatccat                                                    20


<210>  468
<211>  20
<212>  DNA
<213>  Artificial sequence

<220>
<223>  Single strand DNA oligonucleotide

<400>  468
attcggatgg tatcgacgaa                                                    20


<210>  469
<211>  20
<212>  DNA
<213>  Artificial sequence

<220>
<223>  Single strand DNA oligonucleotide

<400>  469
aggatcaagg ttggcaactg                                                    20

<210>  470
<211>  20
<212>  DNA
<213>  Artificial sequence

<220>
<223>  Single strand DNA oligonucleotide

<400>  470
tgccggtagt gttatggaca                                                    20


<210>  471
<211>  20
<212>  DNA
<213>  Artificial sequence

<220>
<223>  Single strand DNA oligonucleotide

<400>  471
ccctgaaaag actcctgctg                                                    20


<210>  472
<211>  20
<212>  DNA
<213>  Artificial sequence

<220>
<223>  Single strand DNA oligonucleotide

<400>  472
aaggtaaact ctgcgctcca                                                    20


<210>  473
<211>  20
<212>  DNA
<213>  Artificial sequence

<220>
<223>  Single strand DNA oligonucleotide

<400>  473
aggatcaagg ttggcaactg                                                    20


<210>  474
<211>  20
<212>  DNA
<213>  Artificial sequence

<220>
<223>  Single strand DNA oligonucleotide

<400>  474
tgccggtagt gttatggaca                                                    20


<210>  475
<211>  20
<212>  DNA
<213>  Artificial sequence

<220>

<223>  Single strand DNA oligonucleotide

<400>  475
tccctgaaaa gactcctgct                                                    20


<210>  476
<211>  20
<212>  DNA
<213>  Artificial sequence

<220>
<223>  Single strand DNA oligonucleotide

<400>  476
gctgccggta gtgttatgga                                                    20


<210>  477
<211>  20
<212>  DNA
<213>  Artificial sequence

<220>
<223>  Single strand DNA oligonucleotide

<400>  477
ccctgaaaag actcctgctg                                                    20


<210>  478
<211>  20
<212>  DNA
<213>  Artificial sequence

<220>
<223>  Single strand DNA oligonucleotide

<400>  478
aaacaaagcg tcgcaaaaag                                                    20


<210>  479
<211>  20
<212>  DNA
<213>  Artificial sequence

<220>
<223>  Single strand DNA oligonucleotide

<400>  479
caatgcaaaa tcccatccat                                                    20


<210>  480
<211>  20
<212>  DNA
<213>  Artificial sequence

<220>
<223>  Single strand DNA oligonucleotide

<400>  480
tacggtgaaa gagtggcatt                                                    20


<210>  481
<211>  20

<212>  DNA
<213>  Artificial sequence

<220>
<223>  Single strand DNA oligonucleotide

<400>  481
ctgcaacaat ggctttttgt                                                    20


<210>  482
<211>  22
<212>  DNA
<213>  Artificial sequence

<220>
<223>  Single strand DNA oligonucleotide

<400>  482
tgtctgcata attggcttta cc                                                 22


<210>  483
<211>  19
<212>  DNA
<213>  Artificial sequence

<220>
<223>  Single strand DNA oligonucleotide

<400>  483
ctcgttccct gcaacaatg                                                     19


<210>  484
<211>  20
<212>  DNA
<213>  Artificial sequence

<220>
<223>  Single strand DNA oligonucleotide

<400>  484
acggtgaaag agtggcattg                                                    20


<210>  485
<211>  21
<212>  DNA
<213>  Artificial sequence

<220>
<223>  Single strand DNA oligonucleotide

<400>  485
tgggctttct gtaggttttg a                                                  21


<210>  486
<211>  20
<212>  DNA
<213>  Artificial sequence

<220>
<223>  Single strand DNA oligonucleotide

<400>  486
ggctacttct ccccaccatt                                                    20

<210> 487
<211> 20
<212> DNA
<213> Artificial sequence

<220>
<223> Single strand DNA oligonucleotide

<400> 487
gggacgttac gcaaatttct 20


<210> 488
<211> 20
<212> DNA
<213> Artificial sequence

<220>
<223> Single strand DNA oligonucleotide

<400> 488
ttctcgtcag gcatttttcc 20


<210> 489
<211> 21
<212> DNA
<213> Artificial sequence

<220>
<223> Single strand DNA oligonucleotide

<400> 489
tgggctttct gtaggttttg a 21


<210> 490
<211> 20
<212> DNA
<213> Artificial sequence

<220>
<223> Single strand DNA oligonucleotide

<400> 490
tgatcaaacc agccatcaac 20


<210> 491
<211> 20
<212> DNA
<213> Artificial sequence

<220>
<223> Single strand DNA oligonucleotide

<400> 491
   ctgcaacaat ggctttttgt 20


<210> 492
<211> 20
<212> DNA
<213> Artificial sequence

<220>

<223>  Single strand DNA oligonucleotide

<400>  492
tcgtcaggca tttttccttt                                                    20


<210>  493
<211>  21
<212>  DNA
<213>  Artificial sequence

<220>
<223>  Single strand DNA oligonucleotide

<400>  493
tgggctttct gtaggttttg a                                                  21


<210>  494
<211>  20
<212>  DNA
<213>  Artificial sequence

<220>
<223>  Single strand DNA oligonucleotide

<400>  494
ggctacttct ccccaccatt                                                    20


<210>  495
<211>  20
<212>  DNA
<213>  Artificial sequence

<220>
<223>  Single strand DNA oligonucleotide

<400>  495
gggcaacagc cataggtaaa                                                    20


<210>  496
<211>  20
<212>  DNA
<213>  Artificial sequence

<220>
<223>  Single strand DNA oligonucleotide

<400>  496
tctcgtcagg catttttcct                                                    20


<210>  497
<211>  20
<212>  DNA
<213>  Artificial sequence

<220>
<223>  Single strand DNA oligonucleotide

<400>  497
tcagttgatg gctggtttga                                                    20


<210>  498
<211>  26

<212>  DNA
<213>  Artificial sequence

<220>
<223>  Single strand DNA oligonucleotide

<400>  498
tgaaaaataa cttaaataag ggatgg                                          26


<210>  499
<211>  26
<212>  DNA
<213>  Artificial sequence

<220>
<223>  Single strand DNA oligonucleotide

<400>  499
gcgaattatt tagtacaaaa agcgta                                          26


<210>  500
<211>  25
<212>  DNA
<213>  Artificial sequence

<220>
<223>  Single strand DNA oligonucleotide

<400>  500
tttcattatg ctatttaaga tgtga                                           25


<210>  501
<211>  27
<212>  DNA
<213>  Artificial sequence

<220>
<223>  Single strand DNA oligonucleotide

<400>  501
aatataaata tatcccatcc cttattt                                         27


<210>  502
<211>  22
<212>  DNA
<213>  Artificial sequence

<220>
<223>  Single strand DNA oligonucleotide

<400>  502
cagagtaaaa atgtaacgct ga                                              22


<210>  503
<211>  26
<212>  DNA
<213>  Artificial sequence

<220>
<223>  Single strand DNA oligonucleotide

<400>  503
cccatccctt atttaagtta tttttc                                          26

<210>  504
<211>  20
<212>  DNA
<213>  Artificial sequence

<220>
<223>  Single strand DNA oligonucleotide

<400>  504
gcaacatcaa aatggctgag                                                    20


<210>  505
<211>  21
<212>  DNA
<213>  Artificial sequence

<220>
<223>  Single strand DNA oligonucleotide

<400>  505
tgattttcgt cagactgcaa g                                                  21


<210>  506
<211>  20
<212>  DNA
<213>  Artificial sequence

<220>
<223>  Single strand DNA oligonucleotide

<400>  506
gcaacatcaa aatggctgag                                                    20


<210>  507
<211>  20
<212>  DNA
<213>  Artificial sequence

<220>
<223>  Single strand DNA oligonucleotide

<400>  507
aacctttcaa ccggagttca                                                    20


<210>  508
<211>  20
<212>  DNA
<213>  Artificial sequence

<220>
<223>  Single strand DNA oligonucleotide

<400>  508
tggccttgta ccaattcctt                                                    20


<210>  509
<211>  21
<212>  DNA
<213>  Artificial sequence

<220>

<223>  Single strand DNA oligonucleotide

<400>  509
tgattttcgt cagactgcaa g                                                21


<210>  510
<211>  20
<212>  DNA
<213>  Artificial sequence

<220>
<223>  Single strand DNA oligonucleotide

<400>  510
tccgatgaaa catcaccatc                                                  20


<210>  511
<211>  20
<212>  DNA
<213>  Artificial sequence

<220>
<223>  Single strand DNA oligonucleotide

<400>  511
caattgaaaa catggccaga                                                  20


<210>  512
<211>  21
<212>  DNA
<213>  Artificial sequence

<220>
<223>  Single strand DNA oligonucleotide

<400>  512
gatgtccgat gaaacatcac c                                                21


<210>  513
<211>  20
<212>  DNA
<213>  Artificial sequence

<220>
<223>  Single strand DNA oligonucleotide

<400>  513
caattgaaaa catggccaga                                                  20


<210>  514
<211>  20
<212>  DNA
<213>  Artificial sequence

<220>
<223>  Single strand DNA oligonucleotide

<400>  514
tggccttgta ccaattcctt                                                  20


<210>  515
<211>  25

<212>  DNA
<213>  Artificial sequence

<220>
<223>  Single strand DNA oligonucleotide

<400>  515
tttttcagta agctcagaca aatca                                          25


<210>  516
<211>  20
<212>  DNA
<213>  Artificial sequence

<220>
<223>  Single strand DNA oligonucleotide

<400>  516
tggccttgta ccaattcctt                                                20


<210>  517
<211>  21
<212>  DNA
<213>  Artificial sequence

<220>
<223>  Single strand DNA oligonucleotide

<400>  517
aaacagatgt cccgaaaatc a                                              21


<210>  518
<211>  20
<212>  DNA
<213>  Artificial sequence

<220>
<223>  Single strand DNA oligonucleotide

<400>  518
cctgctgata gtgccatgaa                                                20


<210>  519
<211>  20
<212>  DNA
<213>  Artificial sequence

<220>
<223>  Single strand DNA oligonucleotide

<400>  519
taatcaggaa cagccggaag                                                20


<210>  520
<211>  20
<212>  DNA
<213>  Artificial sequence

<220>
<223>  Single strand DNA oligonucleotide

<400>  520
cctgctgata gtgccatgaa                                                20

<210>  521
<211>  20
<212>  DNA
<213>  Artificial sequence

<220>
<223>  Single strand DNA oligonucleotide

<400>  521
cggtaagaca ccagccttga                                                    20


<210>  522
<211>  20
<212>  DNA
<213>  Artificial sequence

<220>
<223>  Single strand DNA oligonucleotide

<400>  522
tccttgcctc atgtgttctg                                                    20


<210>  523
<211>  20
<212>  DNA
<213>  Artificial sequence

<220>
<223>  Single strand DNA oligonucleotide

<400>  523
gagatcaagc gtttcccaag                                                    20


<210>  524
<211>  20
<212>  DNA
<213>  Artificial sequence

<220>
<223>  Single strand DNA oligonucleotide

<400>  524
ggaattgcga gtgaggtctt                                                    20


<210>  525
<211>  20
<212>  DNA
<213>  Artificial sequence

<220>
<223>  Single strand DNA oligonucleotide

<400>  525
agcgaacgaa cagctcagat                                                    20


<210>  526
<211>  20
<212>  DNA
<213>  Artificial sequence

<220>

<223>  Single strand DNA oligonucleotide

<400>  526
aggtcttacc attggctgga                                                    20


<210>  527
<211>  20
<212>  DNA
<213>  Artificial sequence

<220>
<223>  Single strand DNA oligonucleotide

<400>  527
tgtcgagaat aagcgaacga                                                    20


<210>  528
<211>  21
<212>  DNA
<213>  Artificial sequence

<220>
<223>  Single strand DNA oligonucleotide

<400>  528
cactttgcat gaaagggctt a                                                  21


<210>  529
<211>  20
<212>  DNA
<213>  Artificial sequence

<220>
<223>  Single strand DNA oligonucleotide

<400>  529
agcgaacgaa cagctcagat                                                    20


<210>  530
<211>  20
<212>  DNA
<213>  Artificial sequence

<220>
<223>  Single strand DNA oligonucleotide

<400>  530
aggatgctgg atcaaaatgc                                                    20


<210>  531
<211>  20
<212>  DNA
<213>  Artificial sequence

<220>
<223>  Single strand DNA oligonucleotide

<400>  531
ttgatagctt agcgcccaat                                                    20


<210>  532
<211>  21

<212>  DNA
<213>  Artificial sequence

<220>
<223>  Single strand DNA oligonucleotide

<400>  532
tctcaccttc actttgcatg a                                                21


<210>  533
<211>  20
<212>  DNA
<213>  Artificial sequence

<220>
<223>  Single strand DNA oligonucleotide

<400>  533
agcgaacgaa cagctcagat                                                  20


<210>  534
<211>  22
<212>  DNA
<213>  Artificial sequence

<220>
<223>  Single strand DNA oligonucleotide

<400>  534
tcacatgtgt tgcaccttta ca                                               22


<210>  535
<211>  24
<212>  DNA
<213>  Artificial sequence

<220>
<223>  Single strand DNA oligonucleotide

<400>  535
gcaacctttt gctttaatgt tttt                                             24


<210>  536
<211>  20
<212>  DNA
<213>  Artificial sequence

<220>
<223>  Single strand DNA oligonucleotide

<400>  536
tgcacaactg gcctatgttt                                                  20


<210>  537
<211>  24
<212>  DNA
<213>  Artificial sequence

<220>
<223>  Single strand DNA oligonucleotide

<400>  537
gcaacctttt gctttaatgt tttt                                             24

<210>  538
<211>  20
<212>  DNA
<213>  Artificial sequence

<220>
<223>  Single strand DNA oligonucleotide

<400>  538
ggagagtgcg gggtatttta                                                    20


<210>  539
<211>  20
<212>  DNA
<213>  Artificial sequence

<220>
<223>  Single strand DNA oligonucleotide

<400>  539
agttcattcg caaccgtttt                                                    20


<210>  540
<211>  20
<212>  DNA
<213>  Artificial sequence

<220>
<223>  Single strand DNA oligonucleotide

<400>  540
tgctattggt gaatggcaaa                                                    20


<210>  541
<211>  20
<212>  DNA
<213>  Artificial sequence

<220>
<223>  Single strand DNA oligonucleotide

<400>  541
cccgcttcat ctgatggtat                                                    20


<210>  542
<211>  20
<212>  DNA
<213>  Artificial sequence

<220>
<223>  Single strand DNA oligonucleotide

<400>  542
gttggcaggt tgctcaattc                                                    20


<210>  543
<211>  20
<212>  DNA
<213>  Artificial sequence

<220>

<223>  Single strand DNA oligonucleotide

<400>  543
ctccaccata attccgcttg                                                    20


<210>  544
<211>  22
<212>  DNA
<213>  Artificial sequence

<220>
<223>  Single strand DNA oligonucleotide

<400>  544
tgagcaagag cataggtttt ga                                                 22


<210>  545
<211>  20
<212>  DNA
<213>  Artificial sequence

<220>
<223>  Single strand DNA oligonucleotide

<400>  545
cgcaaccgtt ttgttttctt                                                    20


<210>  546
<211>  20
<212>  DNA
<213>  Artificial sequence

<220>
<223>  Single strand DNA oligonucleotide

<400>  546
gttggcaggt tgctcaattc                                                    20


<210>  547
<211>  20
<212>  DNA
<213>  Artificial sequence

<220>
<223>  Single strand DNA oligonucleotide

<400>  547
tgccattcac caatagcaaa                                                    20


<210>  548
<211>  20
<212>  DNA
<213>  Artificial sequence

<220>
<223>  Single strand DNA oligonucleotide

<400>  548
gcggaattat ggtggagaaa                                                    20


<210>  549
<211>  20

<212>  DNA
<213>  Artificial sequence

<220>
<223>  Single strand DNA oligonucleotide

<400>  549
atagacgtcg ccagatttcc                                                    20


<210>  550
<211>  20
<212>  DNA
<213>  Artificial sequence

<220>
<223>  Single strand DNA oligonucleotide

<400>  550
tacaaaggct gttggcaggt                                                    20


<210>  551
<211>  20
<212>  DNA
<213>  Artificial sequence

<220>
<223>  Single strand DNA oligonucleotide

<400>  551
tgccattcac caatagcaaa                                                    20


<210>  552
<211>  21
<212>  DNA
<213>  Artificial sequence

<220>
<223>  Single strand DNA oligonucleotide

<400>  552
ggatgatgaa tcaatgccaa a                                                  21


<210>  553
<211>  20
<212>  DNA
<213>  Artificial sequence

<220>
<223>  Single strand DNA oligonucleotide

<400>  553
cacttccgaa ttgagcaacc                                                    20


<210>  554
<211>  27
<212>  DNA
<213>  Artificial sequence

<220>
<223>  Single strand DNA oligonucleotide

<400>  554
cgagcactgt tatagtaatt tcagaag                                            27

<210> 555
<211> 20
<212> DNA
<213> Artificial sequence

<220>
<223> Single strand DNA oligonucleotide

<400> 555
gagtggtcgg ggtattgtgt 20


<210> 556
<211> 20
<212> DNA
<213> Artificial sequence

<220>
<223> Single strand DNA oligonucleotide

<400> 556
tgaagtttgt gccacggtaa 20


<210> 557
<211> 20
<212> DNA
<213> Artificial sequence

<220>
<223> Single strand DNA oligonucleotide

<400> 557
gatgagtggt cggggtattg 20


<210> 558
<211> 27
<212> DNA
<213> Artificial sequence

<220>
<223> Single strand DNA oligonucleotide

<400> 558
cgagcactgt tatagtaatt tcagaag 27


<210> 559
<211> 20
<212> DNA
<213> Artificial sequence

<220>
<223> Single strand DNA oligonucleotide

<400> 559
cgacctgaaa agcccaaata 20


<210> 560
<211> 20
<212> DNA
<213> Artificial sequence

<220>

<223>  Single strand DNA oligonucleotide

<400>  560
acagtcgagc cagcttcaat                                                    20


<210>  561
<211>  20
<212>  DNA
<213>  Artificial sequence

<220>
<223>  Single strand DNA oligonucleotide

<400>  561
cgacctgaaa agcccaaata                                                    20


<210>  562
<211>  20
<212>  DNA
<213>  Artificial sequence

<220>
<223>  Single strand DNA oligonucleotide

<400>  562
acagtcgagc cagcttcaat                                                    20


<210>  563
<211>  20
<212>  DNA
<213>  Artificial sequence

<220>
<223>  Single strand DNA oligonucleotide

<400>  563
cccaaatact tcgggagcta                                                    20


<210>  564
<211>  20
<212>  DNA
<213>  Artificial sequence

<220>
<223>  Single strand DNA oligonucleotide

<400>  564
ccaccatcac cctcaagttc                                                    20


<210>  565
<211>  20
<212>  DNA
<213>  Artificial sequence

<220>
<223>  Single strand DNA oligonucleotide

<400>  565
tgacatggtt gacaacagca                                                    20


<210>  566
<211>  20

<212>  DNA
<213>  Artificial sequence

<220>
<223>  Single strand DNA oligonucleotide

<400>  566
cccgaagtat ttgggctttt                                                    20


<210>  567
<211>  20
<212>  DNA
<213>  Artificial sequence

<220>
<223>  Single strand DNA oligonucleotide

<400>  567
gagggtgatg gtggtgatgt                                                    20


<210>  568
<211>  21
<212>  DNA
<213>  Artificial sequence

<220>
<223>  Single strand DNA oligonucleotide

<400>  568
ttgaatcaaa aatgcacgac a                                                  21


<210>  569
<211>  20
<212>  DNA
<213>  Artificial sequence

<220>
<223>  Single strand DNA oligonucleotide

<400>  569
tgaacttgag ggtgatggtg                                                    20


<210>  570
<211>  20
<212>  DNA
<213>  Artificial sequence

<220>
<223>  Single strand DNA oligonucleotide

<400>  570
cccgaagtat ttgggctttt                                                    20


<210>  571
<211>  20
<212>  DNA
<213>  Artificial sequence

<220>
<223>  Single strand DNA oligonucleotide

<400>  571
caaatagtcc ccgcccttta                                                    20

<210> 572
<211> 20
<212> DNA
<213> Artificial sequence

<220>
<223> Single strand DNA oligonucleotide

<400> 572
ccgaaaagag gagttgaacg 20


<210> 573
<211> 20
<212> DNA
<213> Artificial sequence

<220>
<223> Single strand DNA oligonucleotide

<400> 573
tgacatggtt gacaacagca 20


<210> 574
<211> 25
<212> DNA
<213> Artificial sequence

<220>
<223> Exemplary probe that may be used to identify M17

<400> 574
ttgtccatct tcctgatatt ggtat 25


<210> 575
<211> 20
<212> DNA
<213> Artificial sequence

<220>
<223> Exemplary probe that may be used to identify M17

<400> 575
tcttcccgga aaatgagatg 20


<210> 576
<211> 25
<212> DNA
<213> Artificial sequence

<220>
<223> Exemplary probe that may be used to identify M17

<400> 576
tgtatcaagc tttcaacgtt actga 25


<210> 577
<211> 20
<212> DNA
<213> Artificial sequence

<220>

<223>  Exemplary probe that may be used to identify M17

<400>  577
gtgcggtgaa aaaggtcatt                                                    20


<210>  578
<211>  25
<212>  DNA
<213>  Artificial sequence

<220>
<223>  Exemplary probe that may be used to identify M17

<400>  578
ggttactttt tgttcaagtc agcat                                              25


<210>  579
<211>  20
<212>  DNA
<213>  Artificial sequence

<220>
<223>  Exemplary probe that may be used to identify M17

<400>  579
gagggcgata atgaaatcga                                                    20


<210>  580
<211>  25
<212>  DNA
<213>  Artificial sequence

<220>
<223>  Exemplary probe that may be used to identify M17

<400>  580
gtaggtaaag gtctggatgg tagtg                                              25


<210>  581
<211>  20
<212>  DNA
<213>  Artificial sequence

<220>
<223>  Exemplary probe that may be used to identify M17

<400>  581
tgtgtggaat ggtgctgttt                                                    20


<210>  582
<211>  25
<212>  DNA
<213>  Artificial sequence

<220>
<223>  Exemplary probe that may be used to identify M17

<400>  582
cagatacacc ggatatttag gaatg                                              25


<210>  583
<211>  20

<212>  DNA
<213>  Artificial sequence

<220>
<223>  Exemplary probe that may be used to identify M17

<400>  583
cgtcgggtca aggataggta                                                    20


<210>  584
<211>  25
<212>  DNA
<213>  Artificial sequence

<220>
<223>  Exemplary probe that may be used to identify M17

<400>  584
ccgctagata aaaactgtat tgcat                                              25


<210>  585
<211>  20
<212>  DNA
<213>  Artificial sequence

<220>
<223>  Exemplary probe that may be used to identify M17

<400>  585
gttgtggagc agcttgaaca                                                    20


<210>  586
<211>  25
<212>  DNA
<213>  Artificial sequence

<220>
<223>  Exemplary probe that may be used to identify M17

<400>  586
aaagtgtttt caattcaaca ggaag                                              25


<210>  587
<211>  20
<212>  DNA
<213>  Artificial sequence

<220>
<223>  Exemplary probe that may be used to identify M17

<400>  587
ggtgctagac tctgggcttg                                                    20


<210>  588
<211>  25
<212>  DNA
<213>  Artificial sequence

<220>
<223>  Exemplary probe that may be used to identify M17

<400>  588
gtagtcgtca agccttcatt cttta                                              25

<210> 589
<211> 20
<212> DNA
<213> Artificial sequence

<220>
<223> Exemplary probe that may be used to identify M17

<400> 589
actaagcaga agccgccata 20


<210> 590
<211> 25
<212> DNA
<213> Artificial sequence

<220>
<223> Exemplary probe that may be used to identify M17

<400> 590
gttccgttcc tctggtaaat tagtt 25


<210> 591
<211> 20
<212> DNA
<213> Artificial sequence

<220>
<223> Exemplary probe that may be used to identify M17

<400> 591
gagctttggc ttaagggctt 20


<210> 592
<211> 25
<212> DNA
<213> Artificial sequence

<220>
<223> Exemplary probe that may be used to identify M17

<400> 592
gcttaagtac ggtgacattg ttctt 25


<210> 593
<211> 20
<212> DNA
<213> Artificial sequence

<220>
<223> Exemplary probe that may be used to identify M17

<400> 593
ctacccgtgg cacagtaggt 20


<210> 594
<211> 25
<212> DNA
<213> Artificial sequence

<220>

<223>  Exemplary probe that may be used to identify M17

<400>  594
ggagaacaaa gatttttacc caatt                                          25


<210>  595
<211>  20
<212>  DNA
<213>  Artificial sequence

<220>
<223>  Exemplary probe that may be used to identify M17

<400>  595
ggaaacaaac cgactggaaa                                                20


<210>  596
<211>  25
<212>  DNA
<213>  Artificial sequence

<220>
<223>  Exemplary probe that may be used to identify M17

<400>  596
aatatattac tggggctaaa gtccg                                          25


<210>  597
<211>  20
<212>  DNA
<213>  Artificial sequence

<220>
<223>  Exemplary probe that may be used to identify M17

<400>  597
tttccagtgg cgatctagct                                                20


<210>  598
<211>  25
<212>  DNA
<213>  Artificial sequence

<220>
<223>  Exemplary probe that may be used to identify M17

<400>  598
atctaatcat gtaccgacat caggt                                          25


<210>  599
<211>  20
<212>  DNA
<213>  Artificial sequence

<220>
<223>  Exemplary probe that may be used to identify M17

<400>  599
ggcaagcaga ttgtatcggt                                                20


<210>  600
<211>  25

<212>  DNA
<213>  Artificial sequence

<220>
<223>  Exemplary probe that may be used to identify M17

<400>  600
cttggtattg ggaaaaagat atcct                                          25


<210>  601
<211>  20
<212>  DNA
<213>  Artificial sequence

<220>
<223>  Exemplary probe that may be used to identify M17

<400>  601
gaaattatgg gagcaaggca                                                20


<210>  602
<211>  25
<212>  DNA
<213>  Artificial sequence

<220>
<223>  Exemplary probe that may be used to identify M17

<400>  602
gacggataaa cagatccaca attac                                          25


<210>  603
<211>  20
<212>  DNA
<213>  Artificial sequence

<220>
<223>  Exemplary probe that may be used to identify M17

<400>  603
gggcagacta tcaggcagag                                                20


<210>  604
<211>  25
<212>  DNA
<213>  Artificial sequence

<220>
<223>  Exemplary probe that may be used to identify M17

<400>  604
gtcagacagg caaatccata gatag                                          25


<210>  605
<211>  20
<212>  DNA
<213>  Artificial sequence

<220>
<223>  Exemplary probe that may be used to identify M17

<400>  605
gaggcataaa cccatgctgt                                                20

<210> 606
<211> 25
<212> DNA
<213> Artificial sequence

<220>
<223> Exemplary probe that may be used to identify M17

<400> 606
ggacattaat atctgtgggt gagtc 25


<210> 607
<211> 20
<212> DNA
<213> Artificial sequence

<220>
<223> Exemplary probe that may be used to identify M17

<400> 607
ttgaatttat tcgcccgaac 20


<210> 608
<211> 25
<212> DNA
<213> Artificial sequence

<220>
<223> Exemplary probe that may be used to identify M17

<400> 608
gagaatgtga cgtttatgtg ttcag 25


<210> 609
<211> 20
<212> DNA
<213> Artificial sequence

<220>
<223> Exemplary probe that may be used to identify M17

<400> 609
ccagtcagtg agctatggca 20


<210> 610
<211> 25
<212> DNA
<213> Artificial sequence

<220>
<223> Exemplary probe that may be used to identify M17

<400> 610
agagcgttaa gttttggtat caatg 25


<210> 611
<211> 20
<212> DNA
<213> Artificial sequence

<220>

<223>  Exemplary probe that may be used to identify M17

<400>  611
acagcaactg cgtctttcct                                                    20


<210>  612
<211>  25
<212>  DNA
<213>  Artificial sequence

<220>
<223>  Exemplary probe that may be used to identify M17

<400>  612
tctttcactg cataaattaa atgca                                              25


<210>  613
<211>  20
<212>  DNA
<213>  Artificial sequence

<220>
<223>  Exemplary probe that may be used to identify M17

<400>  613
tcaacctaat gcaaatgcca                                                    20


<210>  614
<211>  25
<212>  DNA
<213>  Artificial sequence

<220>
<223>  Exemplary probe that may be used to identify M17

<400>  614
ttctcttgag cgaagtgttt tagtt                                              25


<210>  615
<211>  20
<212>  DNA
<213>  Artificial sequence

<220>
<223>  Exemplary probe that may be used to identify M17

<400>  615
acgccagaga atctggctaa                                                    20


<210>  616
<211>  25
<212>  DNA
<213>  Artificial sequence

<220>
<223>  Exemplary probe that may be used to identify M17

<400>  616
ttgttatcac tgaatacttg gggtt                                              25


<210>  617
<211>  20

<212>  DNA
<213>  Artificial sequence

<220>
<223>  Exemplary probe that may be used to identify M17

<400>  617
cccgtttggg tgataatgtc                                                    20


<210>  618
<211>  25
<212>  DNA
<213>  Artificial sequence

<220>
<223>  Exemplary probe that may be used to identify M17

<400>  618
tatcactgtt aggttgggaa tgaat                                              25


<210>  619
<211>  20
<212>  DNA
<213>  Artificial sequence

<220>
<223>  Exemplary probe that may be used to identify M17

<400>  619
gaaaaggttg cttgacgctc                                                    20


<210>  620
<211>  25
<212>  DNA
<213>  Artificial sequence

<220>
<223>  Exemplary probe that may be used to identify M17

<400>  620
gagataatga gtcctcttct ttccc                                              25


<210>  621
<211>  20
<212>  DNA
<213>  Artificial sequence

<220>
<223>  Exemplary probe that may be used to identify M17

<400>  621
gggttggatc attgttccac                                                    20


<210>  622
<211>  25
<212>  DNA
<213>  Artificial sequence

<220>
<223>  Exemplary probe that may be used to identify M17

<400>  622
cattaggact tttgtgcacc ttagt                                              25

<210> 623
<211> 20
<212> DNA
<213> Artificial sequence

<220>
<223> Exemplary probe that may be used to identify M17

<400> 623
gtcgctttgc tgcatattga 20


<210> 624
<211> 25
<212> DNA
<213> Artificial sequence

<220>
<223> Exemplary probe that may be used to identify M17

<400> 624
cagtaatcgt tttactgtcc gaact 25


<210> 625
<211> 20
<212> DNA
<213> Artificial sequence

<220>
<223> Exemplary probe that may be used to identify M17

<400> 625
tctcgatgta ctgctggtgc 20


<210> 626
<211> 25
<212> DNA
<213> Artificial sequence

<220>
<223> Exemplary probe that may be used to identify M17

<400> 626
cagcttcgac ttgtatcagt agaca 25


<210> 627
<211> 20
<212> DNA
<213> Artificial sequence

<220>
<223> Exemplary probe that may be used to identify M17

<400> 627
atacgttttc acgccgtttc 20


<210> 628
<211> 25
<212> DNA
<213> Artificial sequence

<220>

<223>  Exemplary probe that may be used to identify M17

<400>  628
aacgacgtaa agaactcaaa atgac                                          25


<210>  629
<211>  20
<212>  DNA
<213>  Artificial sequence

<220>
<223>  Exemplary probe that may be used to identify M17

<400>  629
cgaccctaat tggctgttgt                                                20


<210>  630
<211>  25
<212>  DNA
<213>  Artificial sequence

<220>
<223>  Exemplary probe that may be used to identify M17

<400>  630
agttatcgac tatcaacggt gaaag                                          25


<210>  631
<211>  20
<212>  DNA
<213>  Artificial sequence

<220>
<223>  Exemplary probe that may be used to identify M17

<400>  631
gcggtggcta cactatggtt                                                20


<210>  632
<211>  25
<212>  DNA
<213>  Artificial sequence

<220>
<223>  Exemplary probe that may be used to identify M17

<400>  632
cacctgaact tcttgagaga gtttc                                          25


<210>  633
<211>  20
<212>  DNA
<213>  Artificial sequence

<220>
<223>  Exemplary probe that may be used to identify M17

<400>  633
atcgcggtaa cacttggttc                                                20


<210>  634
<211>  25

<212>  DNA
<213>  Artificial sequence

<220>
<223>  Exemplary probe that may be used to identify M17

<400>  634
agcaagtctc tcaaaaccta cagaa                                           25


<210>  635
<211>  20
<212>  DNA
<213>  Artificial sequence

<220>
<223>  Exemplary probe that may be used to identify M17

<400>  635
tttacctatg gctgttgccc                                                 20


<210>  636
<211>  25
<212>  DNA
<213>  Artificial sequence

<220>
<223>  Exemplary probe that may be used to identify M17

<400>  636
tttttgttaa atgatgcgca ttata                                           25


<210>  637
<211>  20
<212>  DNA
<213>  Artificial sequence

<220>
<223>  Exemplary probe that may be used to identify M17

<400>  637
tttttgttaa atgatgcgca                                                 20


<210>  638
<211>  25
<212>  DNA
<213>  Artificial sequence

<220>
<223>  Exemplary probe that may be used to identify M17

<400>  638
aaaaatagat gataacggaa aaggg                                           25


<210>  639
<211>  20
<212>  DNA
<213>  Artificial sequence

<220>
<223>  Exemplary probe that may be used to identify M17

<400>  639
tggtgatatt tcgtccccat                                                 20

<210>  640
<211>  25
<212>  DNA
<213>  Artificial sequence

<220>
<223>  Exemplary probe that may be used to identify M17

<400>  640
gaaaatggta agaaagaagc attga                                          25


<210>  641
<211>  20
<212>  DNA
<213>  Artificial sequence

<220>
<223>  Exemplary probe that may be used to identify M17

<400>  641
cgctgtggaa agtgacagaa                                                20


<210>  642
<211>  25
<212>  DNA
<213>  Artificial sequence

<220>
<223>  Exemplary probe that may be used to identify M17

<400>  642
taccgctgta ttaaattagt gtgca                                          25


<210>  643
<211>  20
<212>  DNA
<213>  Artificial sequence

<220>
<223>  Exemplary probe that may be used to identify M17

<400>  643
tgcgaatgaa ctcacaggag                                                20


<210>  644
<211>  25
<212>  DNA
<213>  Artificial sequence

<220>
<223>  Exemplary probe that may be used to identify M17

<400>  644
ggatacgagc aaataataca tcacc                                          25


<210>  645
<211>  20
<212>  DNA
<213>  Artificial sequence

<220>

<223>  Exemplary probe that may be used to identify M17

<400>  645
acagtcgagc cagcttcaat                                                    20


<210>  646
<211>  393
<212>  DNA
<213>  Escherichia coli strain M17

<400>  646
ctttacattt cactatgtga ttatttttta attgtataca cattcttgga ttttgaagtt        60

gagattatcc ttcataaaga aggatgaaga gttccctttt aaaaatgact ctatgctatt       120

tatgtgcata tcataatcat caatgagcac cttatcttgg gatgaaccaa taaaaataac       180

attgacctta tctgcactaa ggttaatgca atgatgattt atattaaagt ctaacaagtg       240

agcaagagca taggttttga ctggaaggtt ttcttcactg aaataactat tttgccaaga       300

taaaatgaat ataaaaatta aaaaagaaaa caaaacggtt gcgaatgaac tcacaggagg       360

gagatcgaaa gcttcatctt taaataaaaa aca                                    393


<210>  647
<211>  958
<212>  DNA
<213>  Escherichia coli strain M17

<400>  647
gtgtataagg aaaggatgat gaatcaatgc caaatacact atttaaaagt gctgatgatt        60

ttgcggtgca aaaaataatc aatcctgata tggctataga ggtaatacat tttgtaatta       120

ttgagttcca tagcacctta aaaatcaatg ttctttcaat aaatacacct aaccataatg       180

tagaaaaaat tattgataat agtatgcatg taaattgcca ttgagagaac caatcatctg       240

agtctgtctt tgctatacct aaagccaaac atatgagagc taaaatgcct atcacaatta       300

aagcatatga tttctctgat tcatcctgac gttttttaag ctcatcatag ttttgtaact        360

tttttacaaa ggctgttggc aggttgctca attcggaagt gtcattcttc atatgttctc       420

caagataaca acgatatgct ggaagtatac cgctgtatta aattagtgtg caagcggaat       480

tatggtggag aaacattcat aatatcaatt ttattgtatg tgtctatttg acgtatctct       540

gtattgatga taactaaata taacattcaa taaccaaagg agataaaaaa cgaatgcaca       600

actggcctat gtttttacta ttgatactcg tattcatcag aacagtaatg aaaagattac       660

tgaaattttc attgcgaaat ataaagaagg aataacgtat cattttggcc ctgatatgga       720

acgatttgag tcctgctttg aagcagcgct ttttgctatt ggtgaatggc aaataaaagt        780

tgatgcgatg acacgatacc atgaggaaga tgaagtgatg gagttcttta gctctatacc       840

atcagatgaa gcgggaaatc tggcgacgtc tattttgctg ttcagtgatg ttctcgccat       900

gaaaggcgtg gatgaagtat ttgcctattt tttgggtaag gatgatgttg tatgagtg         958

## Description

### FIELD AND BACKGROUND OF THE INVENTION

The present invention, in some embodiments thereof, relates to a method for identifying *E*. *coli* M-17 in a biological sample and oligonucleotides capable of same.

The intestinal microflora is important for maturation of the immune system, the development of normal intestinal morphology and for maintenance of a chronic and immunologically balanced inflammatory response. The microflora reinforces the barrier function of the intestinal mucosa by preventing attachment of pathogenic microorganisms and the entry of allergens. Some members of the microflora may contribute to the body's requirements for certain vitamins, including biotin, pantothenic acid and vitamin B₁₂. Alteration of the microbial flora of the intestine, such as may occur with antibiotic use, disease and aging, can negatively affect its beneficial role.

Probiotics are a class of microorganisms defined as live microbial organisms that beneficially affect animal and human hosts. Such beneficial effects may be due to improvement of the microbial balance of the intestinal microflora and/or improvement of the properties of the indigenous microflora. The beneficial effects of probiotics may be mediated by a direct antagonistic effect against specific groups of organisms, resulting in a decrease in numbers, by an effect on their metabolism and/or by stimulation of immunity. The mechanisms underlying the proposed actions remain vastly unknown, partly as a consequence of the complexity of the gastro-intestinal ecosystem with which these biotherapeutic agents interact. Probiotics may suppress viable counts of an undesired organism by producing antibacterial compounds, by competing for nutrients or for adhesion sites. They may alter microbial metabolism by increasing or decreasing enzyme activity. Alternatively or additionally they may stimulate the immune system by increasing antibody levels or macrophage activity.

Known probiotic strains include, for example, *Bifidobacteria, Lactobacillus, Lactococcus, Saccharomyces, Streptococcus thermophilus, Enterococcus* and *E. coli.* It is well known that under conditions where the balance of the GI microflora is adversely affected, probiotics become of potential value in restoring the GI microflora enabling the individual host to return to normal.

Recently, it was uncovered that a single species of a non-pathogenic probiotic microorganism derived from *E. coli* is, alone, capable of restoring normal GI flora of human and of a variety of mammals and avians. The beneficial physiological and therapeutic activity of this species in the GI tract is described in detail in U.S. Patent No. 6,500,423, and in WO 02/43649, which are incorporated by reference as if fully set forth herein. These references teach that the *Escherichia coli* strain BU-230-98 ATCC Deposit No. 202226 (DSM 12799), which is an isolate of the commercially available probiotic *E. coli* M-17 strain, is highly effective in preventing or treating gastro-enteric infections or disorders, maintaining or reinstating normal gastro-intestinal microflora, preventing or treating diarrhea, preventing or treating gastro-enteric infection caused by an enteric pathogen, such as a Gram negative bacterium or Gram positive bacterium, preventing or treating gastro-enteric *Salmonella* infection, preventing or treating infectious diarrhea, caused by, for example *C*. *difficile, Salmonella,* particularly *S*. *Shigella, Campylobacter, E. coli, Proteus, Pseudomonas* or *Clostridium* or diarrhea resulting from antibiotic therapy, radiotherapy or chemotherapy, and/or for normalizing the physiological activity of the gastrointestinal tract. Furthermore, U.S. Patent Application No. 20040067223 teaches that strain BU-230-98 ATCC Deposit No. 202226 (DSM 12799), while altering the microbial balance in the GI tract, is highly efficacious agent for treating IBD, such as Crohn's disease and the symptoms associated therewith and for treating other idiopathic inflammation of the small and proximal intestine.

WO2007/136553 teaches identification of M17_{SNAR} by amplification of sequences therein.

### SUMMARY OF THE INVENTION

According to an aspect of some embodiments of the present invention there is provided a method of identifying an M17 strain of *E*. *coli* in a human biological sample, the method comprising analyzing products of an amplification reaction using DNA extracted from the human biological sample and a primer pair which amplifies an M17 specific nucleic acid sequence of an M17 nucleic acid sequence, wherein said primer pair is selected from the group consisting of SEQ ID NOs: 37 and 38; SEQ ID NO: 39 and 40; and SEQ ID NOs: 45 and 46, wherein a product of the amplification reaction is indicative of an M17 strain of *E.coli.*

According to an aspect of some embodiments of the present invention there is provided a method of identifying an M 17 strain of *E*. *coli* in a human sample, the method comprising analyzing DNA extracted from the human sample for a presence or absence of at least one M17 specific nucleic acid sequence of an M17 nucleic acid sequence being selected from the group consisting of SEQ ID NOs: 3, 30, 31, 33, 34, 35 and 36 under experimental conditions, the at least one M17 specific nucleic acid sequence being distinguishable from non M17 nucleic acid sequences in the DNA under the experimental conditions, wherein a presence of the at least one M17 specific nucleic acid sequence is indicative of M 17 in the human sample.

According to an aspect of some embodiments of the present invention there is provided a method of identifying an M17 strain of *E*. *coli* in a human fecal sample, the method comprising analyzing DNA extracted from the human fecal sample for a presence or absence of at least one M17 specific nucleic acid sequence of an M17 nucleic acid sequence being selected from the group consisting of SEQ ID NOs: 1-36 under experimental conditions, the at least one M17 specific nucleic acid sequence being distinguishable from non M17 nucleic acid sequences in the DNA under the experimental conditions, wherein a presence of the at least one M17 specific nucleic acid sequence is indicative of M 17 in the human fecal sample.

According to an aspect of some embodiments of the present invention there is provided a kit for identifying an M17 strain of *E*. *coli* in a human fecal sample comprising at least one oligonucleotide which hybridizes under experimental conditions to an M17 polynucleotide sequence selected from the group consisting of SEQ ID NOs: 1-36, the at least one oligonucleotide being at least 13 bases, the at least one oligonucleotide not being capable of hybridizing to a non M17 polynucleotide sequence under identical experimental conditions.

According to an aspect of some embodiments of the present invention there is provided a primer pair which amplifies an M17 specific nucleic acid sequence of an M17 nucleic acid sequence being selected from the group consisting of SEQ ID NOs: 1-36 under experimental conditions and does not amplify a non-M17 specific nucleic acid sequence under the experimental conditions, each primer of the pair being at least 13 bases.

According to some embodiments of the invention, the M17 nucleic acid sequence is selected from the group consisting of SEQ ID NOs: 3, 30 and 36.

According to some embodiments of the invention, the M17 nucleic acid sequence is selected from the group consisting of SEQ ID NOs: 34 and 35.

According to some embodiments of the invention, the method further comprises quantifying an amount of M17 in the sample.

According to some embodiments of the invention, the analyzing is effected using at least one oligonucleotide being at least 13 bases which hybridizes to the M 17 specific nucleic acid sequence to provide a detectable signal under the experimental conditions and which does not hybridize to the non M17 nucleic acid sequences to provide a detectable signal under the experimental conditions.

According to some embodiments of the invention, the M17 strain of E.coli is *E. coli* M17p (M 17 parent) - ATCC Deposit No. 202226 or *E.coli* M17SNAR - ATCC Deposit No. 7295.

According to some embodiments of the invention, the biological sample comprises a fecal sample.

According to some embodiments of the invention, the at least one oligonucleotide is fully complementary to the M17 specific polynucleotide sequence.

According to some embodiments of the invention, the analyzing is effected using two oligonucleotides, each of the two oligonucleotides being at least 13 bases.

According to some embodiments of the invention, the at least one oligonucleotide comprises two oligonucleotides, wherein a second of the two oligonucleotides hybridizes to an additional M17 polynucleotide sequence under the experimental conditions.

According to some embodiments of the invention, the determining is effected by PCR analysis.

According to some embodiments of the invention, the second of the two oligonucleotides does not hybridize to a non-M17 polynucleotide sequence under the experimental conditions.

According to some embodiments of the invention, the second of the two oligonucleotides binds to a non-M17 polynucleotide sequence under the experimental conditions.

According to some embodiments of the invention, the M17 nucleic acid sequence is selected from the group consisting of SEQ ID NOs: 3, 30, 31, 33, 34, 35 and 36.

According to some embodiments of the invention, at least one of the primers of the pair hybridizes to a polynucleotide sequence which is unique to M17.

According to some embodiments of the invention, the at least one of the primers has a nucleotide sequence as set forth in SEQ ID NO: 37-40, 45, 46 and 62-573.

According to some embodiments of the invention, a first primer of the pair is as set forth in SEQ ID NO: 37 and a second primer of the pair is as set forth in SEQ ID NO: 38.

According to some embodiments of the invention, a first primer of the pair is as set forth in SEQ ID NO: 39 and a second primer of the pair is as set forth in SEQ ID NO: 40.

According to some embodiments of the invention, a first primer of the pair is as set forth in SEQ ID NO: 45 and a second primer of the pair is as set forth in SEQ ID NO: 46.

According to some embodiments of the invention, two of the primers of the primer pair hybridize to a polynucleotide sequence which is unique to M17.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE DRAWINGS

Some embodiments of the invention are herein described, by way of example only, with reference to the accompanying images. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:
FIGs. 1A-C are gel images (0.8 % agarose) of extracted gDNA from M17p, M17SNAR and the 72 ECOR collection *E*. *coli* culture samples. The ECOR collection *E*. *coli* culture extracted gDNA samples (ECOR1-72) are numbered above as labeled gel lanes 1-72, respectively. Lane 74* contains M17SNAR extracted gDNA.
FIG. 2 is an electropherogram of the M17p generated, 8 kb paired end library displaying expected yield and size.
FIG. 3 is a gel image of the PCR products obtained with primer pair CP11+CP12 using the extracted gDNA from the project samples: ECOR collection E. coli culture samples (ECOR1-72), the M17 Parent Strain (M17), M17SNAR (SN) and H2O (H) as a negative control template.
FIG. 4 is a gel image of the PCR products obtained with primer pair CP13+CP14 using the extracted gDNA from the project samples: ECOR collection *E*. *coli* culture samples (ECOR1-72), the M17 Parent Strain (M17), M17SNAR (SN) and H₂O (H) as a negative control template. The artifact observed between lanes 29-30 in figure 3b does not correspond to an amplified DNA fragment but was most likely due to a particulate present on the surface of the transilluminator.
FIG. 5 is a gel image of the PCR products obtained with primer pair CP19+CP20 using the extracted gDNA from the project samples: ECOR collection *E*. *coli* culture samples (ECOR1-72), the M17 Parent Strain (M17), M17SNAR (SN) and H₂O (H) as a negative control template.
FIG. 6 is a growth curve of the cell cultures as determined from the average 600 nm absorbance readings over a time course 2, 4, 6, 8, 10, 12, 14, 16, 18, 20, and 24 hours measured by Nanodrop ND-1000 Spectrophotometer.

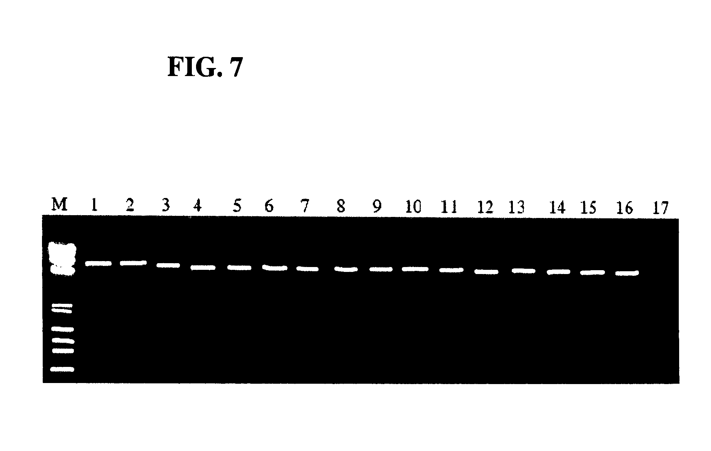

FIG. 7 is a photograph of an agarose gel illustrating DNA extracted from spiked fecal samples. M: size marker (from top down are 23, 9.0, 6.6, 4.4, 2.3, 2.0, 1.3, 1.0, 0.8, and 0.6 Kb). All lanes were 2 µl DNA isolated from samples spiked with 50 µl of the various cell culture dilutions. Lane 1: 10¹⁰ (for extraction control, not used in PCR); Lane 2: 10°; Lane 3: 10-¹; Lane 4: 10-²; Lane 5: 10-³; Lane 6: 10-⁴; Lane 7: 10-⁵; Lane 8: 10-⁶; Lane 9: 10-⁷; Lane 10: 10-⁸; Lane 11: 10-⁹; Lane 12: 10-¹⁰; Lane 13: 10-¹¹; Lane 14: 10-¹²; Lane 15 10-¹³; Lane 16: Stool sample with no spike. Lane 17: PBS buffer with 10³.
FIG. 8 is a photograph of the first duplicate of an agarose gel illustrating DNA amplified using primer set CP11/CP12.
FIG. 9 is a photograph of the first duplicate of an agarose gel illustrating DNA amplified using primer set CP13/CP14.
FIG. 10 is a photograph of the first duplicate of an agarose gel illustrating DNA amplified using primer set CP 19/CP20.
FIG. 11 is a photograph of the second duplicate of an agarose gel illustrating DNA amplified using primer set CP11/CP12.
FIG. 12 is a photograph of the second duplicate of an agarose gel illustrating DNA amplified using primer set CP13/CP14.
FIG. 13 is a photograph of the second duplicate of an agarose gel illustrating DNA amplified using primer set CP 19/CP20.

### DESCRIPTION OF SPECIFIC EMBODIMENTS OF THE INVENTION

The present invention, in some embodiments thereof, relates to a method for identifying *E*. *coli* M-17 in a biological sample and oligonucleotides capable of same.

Before explaining at least one embodiment of the invention in detail, it is to be understood that the invention is not necessarily limited in its application to the details set forth in the following description or exemplified by the Examples. The invention is capable of other embodiments or of being practiced or carried out in various ways.

The probiotic activities of E. coli BU-230-98, ATCC Deposit No. 202226 (DSM 12799) (M17) render it a favorable therapeutic tool for the treatment of a myriad of gastrointestinal disorders and related disorders (e.g., immune related), indicating that antibiotic resistant strains of this bacterial species may be of regulatory importance and used in combination with antibiotic treatment.

Following administration of the probiotic, identification and quantification thereof becomes important for a variety of reasons, including regulatory and determination of dose and treatment regimen.

The present inventors sequenced the genome of the M17 parent strain of E.coli in order to identify unique sequences that could be used for its specific identification. Specifically, the present inventors used the 454 Sequencing™ process which uses a sequencing by synthesis approach to generate sequence data for M17.

Sequence data available from public sources was compared to the M17p 454 sequence data in the Cross-Match software package (using the default screening settings) and 36 fragments were identified that are present the M17p strain (see Example 1, herein below) and not found in other organisms which infect human feces. Using the Blast program, the list of sequences was narrowed down further to comprise in total 3 unique fragments and 3 partially unique sequences.

The present inventors then selected primer sequences that could be used to specifically amplify M17 sequences. The selection was based on identification of primers that could amplify a DNA segment from one of the 36 fragments of the M17p bacteria under particular experimental conditions while not being capable of amplifying sequences from other sources under the same experimental conditions.

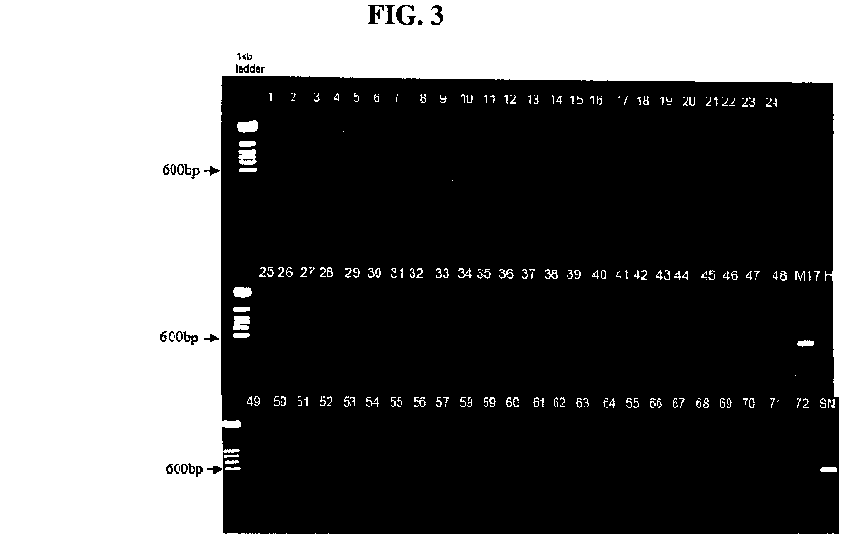

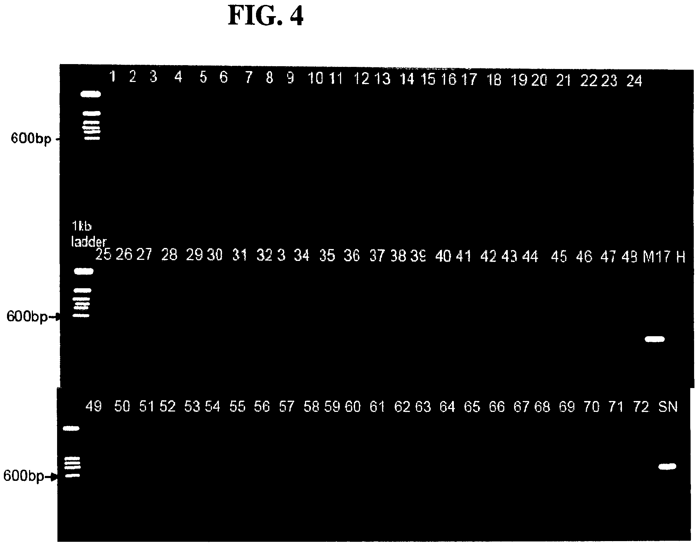

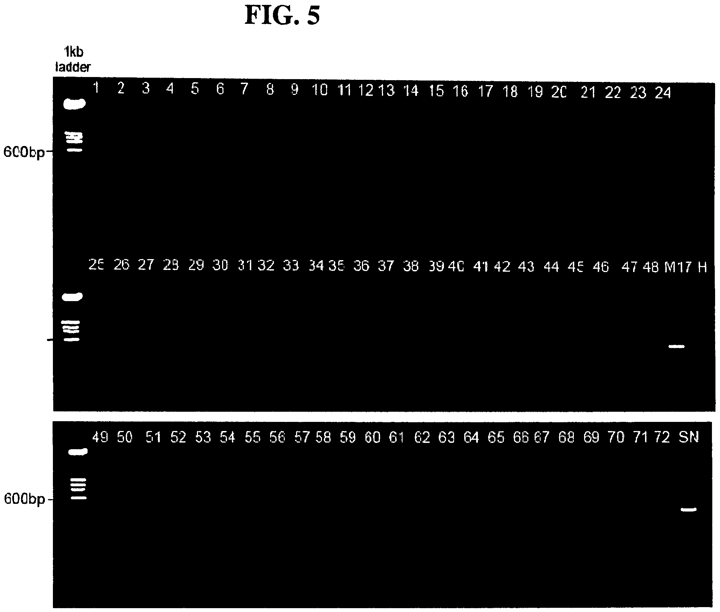

Three sets of primers were shown to be capable of specifically identifying M17 from 72 other *E.coli* strains (see Figures 3-6).

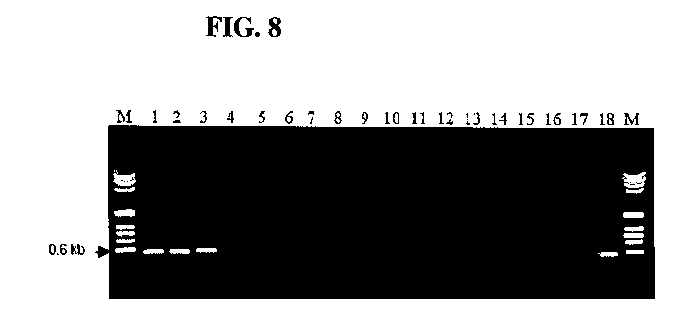

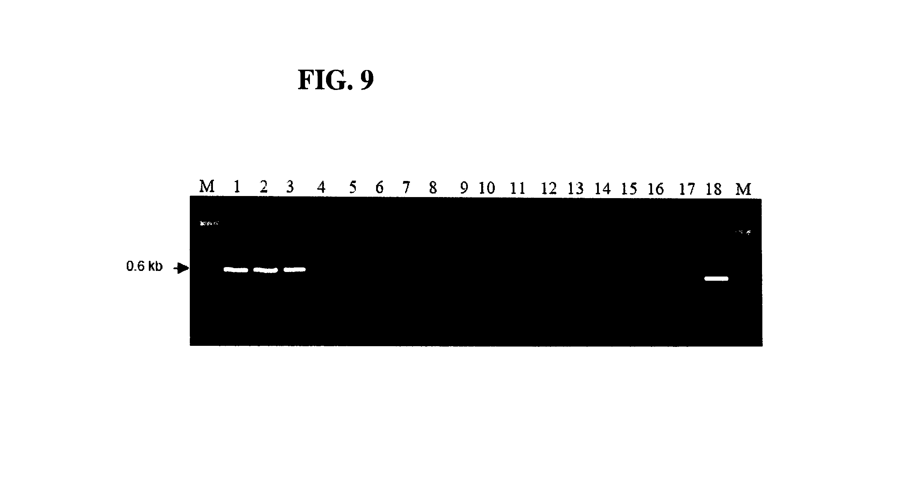

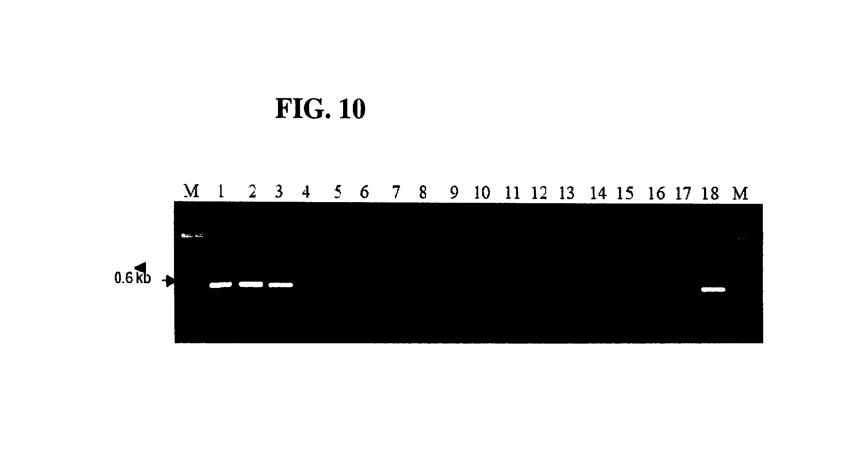

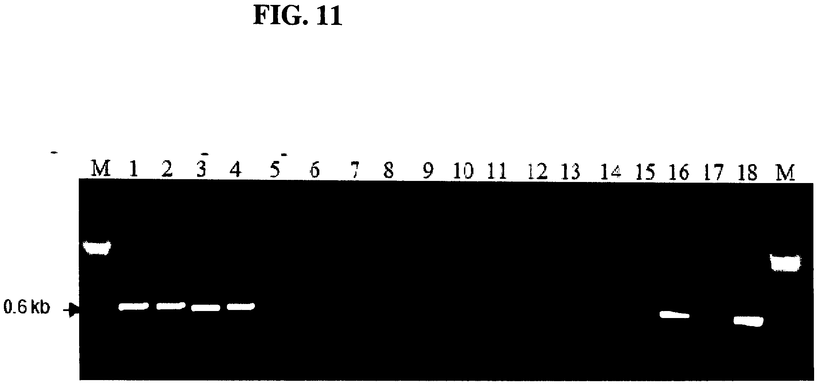

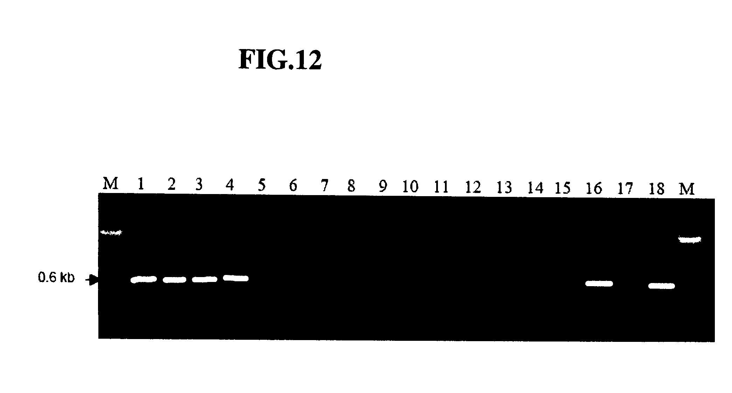

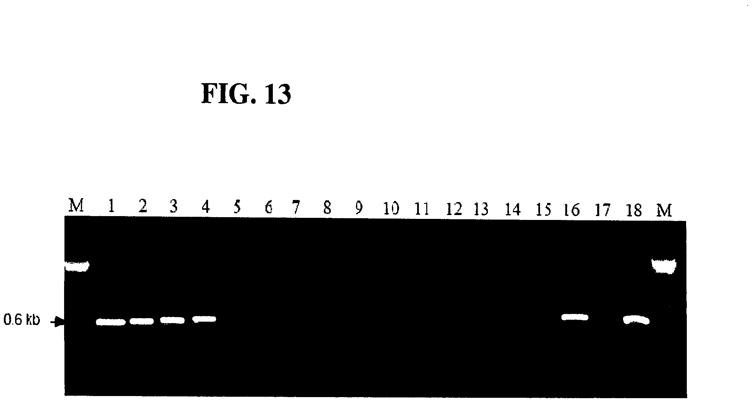

To further analyze the specificity of the three primer sets, the present inventors extracted DNA from M17p cell spiked biological stool samples. The three primer sets were able to specifically identify M17 in the stool samples (Figures 8-13).

The results demonstrated in the Examples section herein support the notion that the 36 fragments identified by the present inventors comprise nucleic acid sequences which may be used to distinguish M17 from other human feces infecting bacteria.

Thus, according to one aspect of the present invention, there is provided a method of identifying an M17 strain of *E. coli* in a human biological sample, the method comprising analyzing DNA extracted from the human biological sample for a presence or absence of at least one M17 specific nucleic acid sequence of an M17 nucleic acid sequence being selected from the group consisting of 3, 30, 31, 33, 34, 35 and 36 under experimental conditions, the at least one M17 specific nucleic acid sequence being distinguishable from non M17 nucleic acid sequences in the DNA under the experimental conditions, wherein a presence of the at least one M17 specific nucleic acid sequence is indicative of M17 in the human biological sample.

As used herein a "M17 bacterial strain of *E. coli"* refers to the strain *per se* and non-pathogenic derived strains which maintain a probiotic activity and biochemical characteristics as listed in Tables 1-3, below.

According to a particular embodiment of this aspect of the present invention, the *E. coli* M17 bacterial strain is BU-239, BU-230-98, BU-230-01, ATCC Deposit No. 202226 (DSM 12799). According to another embodiment, the *E. coli* M17 bacterial strain is a nalidixic acid-resistant mutant derivative of E. coli BU-230-98, ATCC Deposit No. 202226 (DSM 12799) such as the one deposited under the Budapest Treaty in the American Type Culture Collection (ATCC) on December 22, 2005, as strain PTA - 7295 (referred to herein as M17_{SNAR}).

**Table 1- In Vitro Characterization Studies: Various E. coli Probiotic Strains**

| **Strain/Code** | **BU 239 (original M-¹⁷)** | **BU 230-98 (BioBalance, M-17 Industrial Stock)** | **BU 230-01 (BioBalance, M-17 Industrial Stock)** |
|---|---|---|---|
| Serotype | 02 | 02 | 02 |
| Physical Characterization | Gram negative rods | Gram negative rods | Gram negative rods |
| Metabolic Characterization | Ferments glucose | Ferments glucose | Ferments glucose |
| | Reduces nitrates to nitrites | Reduces nitrates to nitrites | Reduces nitrates to nitrites |
| | Oxidase neg. Catalase pos. | Oxidase neg. Catalase pos. | Oxidase neg. Catalase pos. |

**Table 2 - Fermentation Profile for Various E. coli strain M-17 Samples using API 20E**

| **Fermentation Substrate** | **BU-239 (Original *E*. *coli* strain M-17)** | ***E. coli* strain M-17, ATCC 202226 (DSM 12799) (BioBalance Deposited Master Seed Stock)** | ***E. coli* strain M-17 (Taresevich Institute, Moscow, Official Sample)** |
|---|---|---|---|
| Ortho-nitrophenyl-beta-D-galactnpyranoside | + | + | + |
| Arginine dihydrolase | - | - | - |
| Lysine decarboxylase | + | + | + |
| Ornithine decarboxylase | + | + | + |
| Citrate | - | - | - |
| H₂S | - | - | - |
| Urease | - | - | - |
| Tryptophan deaminase | - | - | - |
| Indole | + | + | + |
| Voges-Proskauer | - | - | - |
| Gelatin | - | - | - |
| Glucose | + | + | + |
| Mannitol | + | + | + |
| Inositiol | - | - | - |
| Sorbitol | + | + | + |
| Rhamnose | + | + | + |
| Sucrose | + | + | + |
| Melibiose | + | + | + |
| Amygdalin | - | - | - |
| Arabinose | + | + | + |

**Table 3 - In Vitro Characterization Studies: Presence of Virulence Factors in E. coli Strain M-17 as Detected by PCR**

| **Category of Pathogenic *E*. *coli*** | **Type of Virulence Factor(s)** | **Virulence Factor Designation(s)** | ***E. coli* Strain M-17 Isolate** | | |
|---|---|---|---|---|---|
| | | | **BU-239 (original)** | **ATCC 202226 (DSM 12799)** | **Tarasevich (Russian)** |
| Uropathogenic | Adhesion factors | Type I (Fim A) | + | + | + |
| | | | - | - | - |
| | | AFA | - | - | - |
| | | SFA | | | |
| Uropathogenic -septicemic | Adhesion | PapC | - | - | - |
| | factors (P fimbriae) | PapG | - | - | - |
| Uropathogenic -septicemic -meningitis assoc. | Aerobactin | iuc | - | - | - |
| Enterohemorragic - uropathogenic | Hemolysins | HlyA, HlyC | - | - | - |
| | | Ehx | - | - | - |
| Enterohemorragic - | Attaching and effacing gene | pas | - | - | - |
| enteropathogenic | Intimin | eae | - | - | - |
| Enterohemorragic | Shigatoxins | Stx1, Stx2 | - | - | - |
| | | VT2vpl, VT2vh | - | - | - |
| | | SLT I, SLT II | | | |
| | Flagellar antigen | FliC | - | - | - |
| | O serogroup | O157 | - | - | - |
| | H serotype | H7 | - | - | - |
| Enteropathogenic | Attaching and effacing factor | EAE | - | - | - |
| | Bundle forming pili | bfp | - | - | - |
| Enteroaggregative | Adhesion | aggR | - | - | - |
| | factors | AAF/1 | - | - | - |
| | Toxin | EAST1 | - | - | - |
| Enterotoxigenic | Adhesion factors | CFA1, | - | - | - |
| | | CFA2 | - | - | - |
| | | (CS1coo) | - | - | - |
| | | CFA2 (CS3 cst) | | | |
| | Adhesion | F4 (K88) | - | - | - |
| | factors (shared | F5 (K99) | - | - | - |
| | by porcine and | F18 | - | - | - |
| | bovine | F41 | - | - | - |
| | Enterotoxins | LT, StaH | - | - | - |
| | | STaP,STb | - | - | - |
| Extraintestinal | Adhesion factor | CS31a | - | - | - |
| | Autotransporter | Tsh | - | - | - |

The present invention contemplates identifying M 17 in any biological sample which comprises nucleic acids (DNA and/or RNA). The biological sample typically comprises a body fluid or part of an organism. The sample may be blood, feces, semen, skin, cheek cell, urine cerebrospinal fluid and saliva. According to one embodiment, the biological sample is retrieved from a human subject. The sample may also be a food or feed sample. The sample may be fresh or frozen.

Isolation, extraction or derivation of DNA may be carried out by any suitable method. Isolating DNA from a biological sample generally includes treating a biological sample in such a manner that genomic DNA present in the sample is extracted and made available for analysis. Any isolation method that results in extracted genomic DNA may be used in the practice of the present invention. It will be understood that the particular method used to extract DNA will depend on the nature of the source.

Methods of DNA extraction are well-known in the art. A classical DNA isolation protocol is based on extraction using organic solvents such as a mixture of phenol and chloroform, followed by precipitation with ethanol (J. Sambrook et al., "Molecular Cloning: A Laboratory Manual", 1989, 2.sup.nd Ed., Cold Spring Harbour Laboratory Press: New York, N.Y.). Other methods include: salting out DNA extraction (P. Sunnucks et al., Genetics, 1996, 144: 747-756; S. M. Aljanabi and I. Martinez, Nucl. Acids Res. 1997, 25: 4692-4693), trimethylammonium bromide salts DNA extraction (S. Gustincich et al., BioTechniques, 1991, 11: 298-302) and guanidinium thiocyanate DNA extraction (J. B. W. Hammond et al., Biochemistry, 1996, 240: 298-300).

There are also numerous versatile kits that can be used to extract DNA from tissues and bodily fluids and that are commercially available from, for example, BD Biosciences Clontech (Palo Alto, Calif.), Epicentre Technologies (Madison, Wis.), Gentra Systems, Inc. (Minneapolis, Minn.), MicroProbe Corp. (Bothell, Wash.), Organon Teknika (Durham, N.C.), and Qiagen Inc. (Valencia, Calif.). User Guides that describe in great detail the protocol to be followed are usually included in all these kits. Sensitivity, processing time and cost may be different from one kit to another. One of ordinary skill in the art can easily select the kit(s) most appropriate for a particular situation.

The sample may be processed before the method is carried out, for example DNA purification may be carried out following the extraction procedure. The DNA in the sample may be cleaved either physically or chemically (e.g. using a suitable enzyme). Processing of the sample may involve one or more of: filtration, distillation, centrifugation, extraction, concentration, dilution, purification, inactivation of interfering components, addition of reagents, and the like.

As mentioned, the method is effected by identifying at least one M17 specific nucleic acid sequence of an M17 nucleic acid sequence being selected from the group consisting of SEQ ID NOs: 3, 30, 31, 33, 34, 35 and 36.

The phrase "M17 specific nucleic acid sequence" as used herein refers to a sequence which is unique to M17 bacteria and is not present in non-M17 nucleic acid sequences. Such a sequence is detectable and distinguishable using molecular biology tools, as further described herein below.

Preferably the sequence is 100 % unique (as verified using a sequence alignment software such as BLAST analysis) but it may comprise a certain level of homology/identity. Thus according to a specific embodiment, the sequence is at least no more than 70 % homologous, 75 % homologous, 80 % homologous, 85 % homologous, 90 % homologous with non-M17 nucleic acid sequences.

The M17 specific nucleic is at least about 13, 16, 18, 20, 22, 25, 30, 40, 50, 55, 60, 65, 70, 80, 90, 100, 120 or more nucleotides.

According to a particular embodiment, the method is effected by identifying at least one M17 specific nucleic acid sequence of an M17 nucleic acid sequence being selected from the group consisting of SEQ ID NOs: 3, 30, 36, 47-51 and 646-647.

Sequences 47-51 and 646-647 are comprised in the SEQ ID NOs: 31, 33, 34 and 35 and have been shown by BLAST analysis not to comprise nucleic acid sequences which have more than 75 % identity with another nucleic acid sequence, as detailed in Table 4 herein below.

**Table 4**

| ***Fragment*** | ***SEQ ID NO:*** | ***alignment with other strains or species*** | ***Unique region*** | ***Unique region*** |
|---|---|---|---|---|
| frag-03 | 3 | unique | all | |
| frag-30 | 30 | unique | all | |
| frag-31 | 31 | partial | 620-763 SEQ ID NO: 47 | |
| frag-33 | 33 | partial | 1-322 SEQ ID NO: 48 | 1488-1750 SEQ ID NO: 49 |
| frag-34 | 34 | partial | 1-188 SEQ ID NO: 50 | 1215-1407 SEQ ID NO: 51 |
| frag-35 | 35 | partial | 1-393 SEQ ID NO: 646 | 492-1449 SEQ ID NO: 647 |
| frag-36 | 36 | unique | all | |

| | | | | |
|---|---|---|---|---|
| * partially unique sequences are defined as a fragment having regions portions similar to a DNA sequence in the database with at least 75 % identities and longer 100nt long and portions which do not align to any sequence in the data base under these terms. **the unique sequences are defmed as a fragment lacking any region similar to a DNA sequence in the database with at least 75 % identities and longer 100 nt long. | | | | |

Table 5 herein below provides the alignments for SEQ ID NO: 31.

**Table 5**

| partial alignment | with | score (local) |
|---|---|---|
| 763-881 | >gb\|CP00127.1\| Salmonella enterica subsp. enterica serovar Sc... 71.6 8e-09 | Identities = 93/123 (75%) |
| 763-881 | >gb\|CP001846.1\| Escherichia coli O55:H7 str. CB9615, complete genome | Identities = 91/122 (74%) |
| 763-881 | >gb\|CP001063.1\| Shigella boydii CDC 3083-94, complete genome | Identities = 91/122 (74%) |
| 101-619 | Marinobacter sp. ELB 17 1101232001211 | Identities = 393/519 (76 %) |

Table 6 herein below provides the alignments for SEQ ID NO: 33.

**Table 6**

| partial alignment | with | score (local) |
|---|---|---|
| 1750-2223 | >emb\|FP929037.1\| Clostridium saccharolyticum-like K10 draft genome | Identities = 341/482 (70%) |
| 1752-2329 | >emb\|AM990992.1\| Staphylococcus aureus subsp. aureus ST398 complete genome, isolate | Identities = 394/583 (67%) |
| 325-900 | >emb\|AM990992.1\| Staphylococcus aureus subsp. aureus ST398 complete genome, isolate | Identities = 382/593 (64%) |
| 1751-2070 | >gb\|CP000721.1\| Clostridium beijerinckii NCIMB 8052, complete genome | Identities = 228/320 (71%) |
| 725-917 | >gb\|CP000721.1\| Clostridium beijerinckii NCIMB 8052, complete genome | Identities = 137/196 (69%) |
| 582-1488 | >gb\|CP001740.1\| Sebaldella termitidis ATCC 33386 plasmid pSTERM01, complete sequence | Identities = 601/931 (64%) |
| 1761-2094 | >gb\|CP001740.1\| Sebaldella termitidis ATCC 33386 plasmid pSTERM01, complete sequence | Identities = 236/340 (69%) |
| 402-918 | >gb\|CP000569.1\| Actinobacillus pleuropneumoniae L20 serotype 5b complete genome | Identities = 347/529 (65%) |

Table 7A herein below provides the alignments for SEQ ID NO: 34.

**Table 7A**

| partial alignment | with | score (local) |
|---|---|---|
| 771-1214 | >gb\|CP000891.1\| Shewanella baltica OS 195, complete genome | Identities = 321/445 (72%) |
| 188-688 | Vibrio alginolyticus | Identities 414/559 (75%) |

Table 7B herein below provides the alignments for SEQ ID NO: 35.

**Table 7B**

| partial alignment | with | score (local) |
|---|---|---|
| 393-491 | Populus trichocarpa Ptrichocarpa_Cont20220 | Identities = 75/101 (75%) |

Typically, the method of this aspect of the present invention is carried out using an isolated oligonucleotide which hybridizes to an M17 nucleic acid sequence by complementary base-pairing in a sequence specific manner, and discriminates the M17 nucleic acid sequence from other nucleic acid sequences in the DNA sample. Oligonucleotides typically comprises a region of complementary nucleotide sequence that hybridizes under stringent conditions to at least about 8, 10, 13, 16, 18, 20, 22, 25, 30, 40, 50, 55, 60, 65, 70, 80, 90, 100, 120 (or any other number in-between) or more consecutive nucleotides in a target nucleic acid molecule. Depending on the particular assay, the consecutive nucleotides can either include the M17 specific nucleic acid sequence, or be a specific region in close enough proximity 5' and/or 3' to the M17 specific nucleic acid sequence to carry out the desired assay.

The term "isolated", as used herein in reference to an oligonucleotide, means an oligonucleotide, which by virtue of its origin or manipulation, is separated from at least some of the components with which it is naturally associated or with which it is associated when initially obtained. By "isolated", it is alternatively or additionally meant that the oligonucleotide of interest is produced or synthesized by the hand of man.

As mentioned herein above, the present inventors have identified 36 fragments (SEQ ID NOs:1-36) which may be used to distinguish M17 from other human feces infecting bacteria. Oligonucleotides which specifically hybridize to any one of these fragments may be used to identify M17 in human fecal samples.

In order to identify an oligonucleotide specific for any of the M17 sequences SEQ ID NOs: 1-36, the gene/transcript and/or context sequence surrounding the SNP of interest is typically examined using a computer algorithm which starts at the 5' or at the 3' end of the nucleotide sequence. Typical algorithms will then identify oligonucleotides of defined length that are unique to the gene/SNP context sequence, have a GC content within a range suitable for hybridization, lack predicted secondary structure that may interfere with hybridization, and/or possess other desired characteristics or that lack other undesired characteristics.

Following identification of the oligonucleotide it may be tested for specificity towards M17 under wet or dry conditions. Thus, for example, in the case where the oligonucleotide is a primer, the primer may be tested for its ability to amplify a sequence of M 17 using PCR to generate a detectable product and for its non ability to amplify other bacterial strains. The products of the PCR reaction may be analyzed on a gel and verified according to presence and/or size.

Additionally, or alternatively, the sequence of the oligonucleotide may be analyzed by computer analysis to see if it is homologous (or is capable of hybridizing to) other known sequences. A BLAST 2.2.10 (Basic Local Alignment Search Tool) analysis may be performed on the chosen oligonucleotide (worldwidewebdotncbidotnlmdotnihdotgov/blast/). The BLAST program finds regions of local similarity between sequences. It compares nucleotide or protein sequences to sequence databases and calculates the statistical significance of matches thereby providing valuable information about the possible identity and integrity of the 'query' sequences.

According to one embodiment, the olignoucleotide is a probe. As used herein, the term "probe" refers to an oligonucleotide which hybridizes to the M17 specific nucleic acid sequence to provide a detectable signal under experimental conditions and which does not hybridize to non M17 nucleic acid sequences to provide a detectable signal under identical experimental conditions.

Below is a list of exemplary probes that may be used to identify M17 specific sequences (SEQ ID NOs: 1-36).

**Table 8**

| Seq ID | start | length | Tm (50mM salt)_ | Sequence | SEQ ID NO: |
|---|---|---|---|---|---|
| frag-01 | 973 | 25 | 59.99 | TTGTCCATCTT CCTGATATTGGTAT | 574 |
| frag-01 | 730 | 20 | 60.01 | TCTTCCCGGAA AATGAGATG | 575 |
| frag-02 | 53 | 25 | 60.22 | TGTATCAAGCTT TCAACGTTACTGA | 576 |
| frag-02 | 122 | 20 | 59.98 | GTGCGGTGAAAA AGGTCATT | 577 |
| frag-03 | 1218 | 25 | 59.99 | GGTTACTTTTTGT TCAAGTCAGCAT | 578 |
| frag-03 | 1157 | 20 | 60 | GAGGGCGATAA TGAAATCGA | 579 |
| frag-04 | 5 | 25 | 59.41 | GTAGGTAAAGG TCTGGATGGTAGTG | 580 |
| frag-04 | 109 | 20 | 60.01 | TGTGTGGAATG GTGCTGTTT | 581 |
| frag-05 | 30 | 25 | 60 | CAGATACACCG GATATTTAGGAATG | 582 |
| frag-05 | 171 | 20 | 59.95 | CGTCGGGTCAA GGATAGGTA | 583 |
| frag-06 | 97 | 25 | 59.97 | CCGCTAGATAA AAACTGTATTGCAT | 584 |
| frag-06 | 264 | 20 | 60.03 | GTTGTGGAGCA GCTTGAACA | 585 |
| frag-07 | 283 | 25 | 59.96 | AAAGTGTTTTCA ATTCAACAGGAAG | 586 |
| frag-07 | 428 | 20 | 60.01 | GGTGCTAGACTC TGGGCTTG | 587 |
| frag-08 | 218 | 25 | 60.19 | GTAGTCGTCAAG CCTTCATTCTTTA | 588 |
| frag-08 | 298 | 20 | 60 | ACTAAGCAGAAG CCGCCATA | 589 |
| frag-09 | 284 | 25 | 60.17 | GTTCCGTTCCTC TGGTAAATTAGTT | 590 |
| frag-09 | 148 | 20 | 59.99 | GAGCTTTGGCTT AAGGGCTT | 591 |
| frag-10 | 202 | 25 | 60.01 | GCTTAAGTACGG TGACATTGTTCTT | 592 |
| frag-10 | 228 | 20 | 60.05 | CTACCCGTGGCA CAGTAGGT | 593 |
| frag-11 | 195 | 25 | 60.01 | GGAGAACAAAG ATTTTTACCCAATT | 594 |
| frag-11 | 9 | 20 | 59.95 | GGAAACAAACC GACTGGAAA | 595 |
| frag-12 | 415 | 25 | 60.03 | AATATATTACTG GGGCTAAAGTCCG | 596 |
| frag-12 | 445 | 20 | 59.98 | TTTCCAGTGGC GATCTAGCT | 597 |
| frag-13 | 96 | 25 | 60.04 | ATCTAATCATGT ACCGACATCAGGT | 598 |
| frag-13 | 188 | 20 | 60.1 | GGCAAGCAGA TTGTATCGGT | 599 |
| frag-14 | 44 | 25 | 59.99 | CTTGGTATTGGG AAAAAGATATCCT | 600 |
| frag-14 | 124 | 20 | 60.04 | GAAATTATGG GAGCAAGGCA | 601 |
| frag-15 | 203 | 25 | 60.03 | GACGGATAAAC AGATCCACAATTAC | 602 |
| frag-15 | 132 | 20 | 59.97 | GGGCAGACTA TCAGGCAGAG | 603 |
| frag-16 | 104 | 25 | 60.38 | GTCAGACAGGC AAATCCATAGATAG | 604 |
| frag-16 | 24 | 20 | 59.96 | GAGGCATAAA CCCATGCTGT | 605 |
| frag-17 | 130 | 25 | 60.02 | GGACATTAATA TCTGTGGGTGAGTC | 606 |
| frag-17 | 56 | 20 | 59.91 | TTGAATTTATT CGCCCGAAC | 607 |
| frag-18 | 194 | 25 | 59.98 | GAGAATGTGACG TTTATGTGTTCAG | 608 |
| frag-18 | 444 | 20 | 60.01 | CCAGTCAGTGA GCTATGGCA | 609 |
| frag-19 | 85 | 25 | 59.97 | AGAGCGTTAAG TTTTGGTATCAATG | 610 |
| frag-19 | 111 | 20 | 60.06 | ACAGCAACTG CGTCTTTCCT | 611 |
| frag-20 | 44 | 25 | 60.04 | TCTTTCACTGC ATAAATTAAATGCA | 612 |
| frag-20 | 229 | 20 | 60.07 | TCAACCTAATG CAAATGCCA | 613 |
| frag-21 | 720 | 25 | 60 | TTCTCTTGAGCG AAGTGTTTTAGTT | 614 |
| frag-21 | 146 | 20 | 59.98 | ACGCCAGAGAA TCTGGCTAA | 615 |
| frag-22 | 130 | 25 | 60.05 | TTGTTATCACTGA ATACTTGGGGTT | 616 |
| frag-22 | 501 | 20 | 60.05 | CCCGTTTGGG TGATAATGTC | 617 |
| frag-23 | 152 | 25 | 60.02 | TATCACTGTTAG GTTGGGAATGAAT | 618 |
| frag-23 | 87 | 20 | 60 | GAAAAGGTTGC TTGACGCTC | 619 |
| frag-24 | 587 | 25 | 59.99 | GAGATAATGAG TCCTCTTCTTTCCC | 620 |
| frag-24 | 31 | 20 | 60.03 | GGGTTGGATCA TTGTTCCAC | 621 |
| frag-25 | 214 | 25 | 60 | CATTAGGACTTTT GTGCACCTTAGT | 622 |
| frag-25 | 237 | 20 | 59.98 | GTCGCTTTGCTG CATATTGA | 623 |
| frag-26 | 877 | 25 | 60 | CAGTAATCGTTT TACTGTCCGAACT | 624 |
| frag-26 | 800 | 20 | 60.02 | TCTCGATGTACT GCTGGTGC | 625 |
| frag-27 | 105 | 25 | 60.02 | CAGCTTCGACTT GTATCAGTAGACA | 626 |
| frag-27 | 58 | 20 | 60 | ATACGTTTTCA CGCCGTTTC | 627 |
| frag-28 | 543 | 25 | 59.99 | AACGACGTAAAG AACTCAAAATGAC | 628 |
| frag-28 | 682 | 20 | 59.99 | CGACCCTAATTG GCTGTTGT | 629 |
| frag-29 | 149 | 25 | 59.97 | AGTTATCGACTA TCAACGGTGAAAG | 630 |
| frag-29 | 116 | 20 | 60.02 | GCGGTGGCTA CACTATGGTT | 631 |
| frag-30 | 532 | 25 | 59.98 | CACCTGAACTTC TTGAGAGAGTTTC | 632 |
| frag-30 | 817 | 20 | 60 | ATCGCGGTAAC ACTTGGTTC | 633 |
| frag-31 | 427 | 25 | 60 | AGCAAGTCTCTCA AAACCTACAGAA | 634 |
| frag-31 | 502 | 20 | 59.96 | TTTACCTATGG CTGTTGCCC | 635 |
| frag-32 | 28 | 25 | 59.81 | TTTTTGTTAAATG ATGCGCATTATA | 636 |
| frag-32 | 28 | 20 | 57.87 | TTTTTGTTAAA TGATGCGCA | 637 |
| frag-33 | 2355 | 25 | 59.99 | AAAAATAGATG ATAACGGAAAAGGG | 638 |
| frag-33 | 750 | 20 | 60.01 | TGGTGATATTTC GTCCCCAT | 639 |
| frag-34 | 1067 | 25 | 60.02 | GAAAATGGTAAGA AAGAAGCATTGA | 640 |
| frag-34 | 37 | 20 | 60.02 | CGCTGTGGAAA GTGACAGAA | 641 |
| frag-35 | 939 | 25 | 59.99 | TACCGCTGTATTA AATTAGTGTGCA | 642 |
| frag-35 | 340 | 20 | 59.98 | TGCGAATGAAC TCACAGGAG | 643 |
| frag-36 | 392 | 25 | 60.02 | GGATACGAGCAA ATAATACATCACC | 644 |
| frag-36 | 235 | 20 | 60.02 | ACAGTCGAGCC AGCTTCAAT | 645 |

The probes of this embodiment of this aspect of the present invention may be, for example, affixed to a solid support (e.g., arrays or beads).

According to another embodiment, the oligonucleotide is a primer of a primer pair. As used herein, the term "primer" refers to an oligonucleotide which acts as a point of initiation of a template-directed synthesis using methods such as PCR (polymerase chain reaction) or LCR (ligase chain reaction) under appropriate conditions (e.g., in the presence of four different nucleotide triphosphates and a polymerization agent, such as DNA polymerase, RNA polymerase or reverse-transcriptase, DNA ligase, etc, in an appropriate buffer solution containing any necessary co-factors and at suitable temperature(s)). Such a template directed synthesis is also called "primer extension". For example, a primer pair may be designed to amplify a region of DNA using PCR. Such a pair will include a "forward primer" and a "reverse primer" that hybridize to complementary strands of a DNA molecule and that delimit a region to be synthesized/amplified. A primer of this aspect of the present invention is capable of amplifying, together with its pair (e.g. by PCR) an M17 specific nucleic acid sequence to provide a detectable signal under experimental conditions and which does not amplify non M17 nucleic acid sequence to provide a detectable signal under identical experimental conditions.

According to additional embodiments, the oligonucleotide is about 8, 9, 10, 11, 12, 13, 14, 15, 16, 17, 18, 19, 20, 21, 22, 23, 24 or 25 nucleotides in length. While the maximal length of a probe can be as long as the target sequence to be detected, depending on the type of assay in which it is employed, it is typically less than about 50, 60, 65, or 70 nucleotides in length. In the case of a primer, it is typically less than about 30 nucleotides in length. In a specific preferred embodiment of the invention, a primer or a probe is within the length of about 18 and about 28 nucleotides. It will be appreciated that when attached to a solid support, the probe may be of about 30-70, 75, 80, 90, 100, or more nucleotides in length.

The oligonucleotide of this aspect of the present invention need not reflect the exact sequence of the M17 specific nucleic acid sequence (i.e. need not be fully complementary), but must be sufficiently complementary to hybridize with the M17 specific nucleic acid sequence under the particular experimental conditions. Accordingly, the sequence of the oligonucleotide typically has at least 70 % homology, preferably at least 80 %, 90 %, 95 %, 97 %, 99 % or 100 % homology, for example over a region of at least 13 or more contiguous nucleotides with the target M17 nucleic acid sequence. The conditions are selected such that hybridization of the oligonucleotide to the M17 nucleic acid sequence is favored and hybridization to other non M17 nucleic acid sequences is minimized.

By way of example, hybridization of short nucleic acids (below 200 bp in length, e.g. 13-50 bp in length) can be effected by the following hybridization protocols depending on the desired stringency; (i) hybridization solution of 6 x SSC and 1 % SDS or 3 M TMACl, 0.01 M sodium phosphate (pH 6.8), 1 mM EDTA (pH 7.6), 0.5 % SDS, 100 µg/ml denatured salmon sperm DNA and 0.1 % nonfat dried milk, hybridization temperature of 1 - 1.5 °C below the Tm, final wash solution of 3 M TMACl, 0.01 M sodium phosphate (pH 6.8), 1 mM EDTA (pH 7.6), 0.5 % SDS at 1 - 1.5 °C below the Tm (stringent hybridization conditions) (ii) hybridization solution of 6 x SSC and 0.1 % SDS or 3 M TMACI, 0.01 M sodium phosphate (pH 6.8), 1 mM EDTA (pH 7.6), 0.5 % SDS, 100 µg/ml denatured salmon sperm DNA and 0.1 % nonfat dried milk, hybridization temperature of 2 - 2.5 °C below the Tm, final wash solution of 3 M TMACl, 0.01 M sodium phosphate (pH 6.8), 1 mM EDTA (pH 7.6), 0.5 % SDS at 1 - 1.5 °C below the Tm, final wash solution of 6 x SSC, and final wash at 22 °C (stringent to moderate hybridization conditions); and (iii) hybridization solution of 6 x SSC and 1 % SDS or 3 M TMACI, 0.01 M sodium phosphate (pH 6.8), 1 mM EDTA (pH 7.6), 0.5 % SDS, 100 µg/ml denatured salmon sperm DNA and 0.1 % nonfat dried milk, hybridization temperature at 2.5-3 °C below the Tm and final wash solution of 6 x SSC at 22 °C (moderate hybridization solution).

Various considerations must be taken into account when selecting the stringency of the hybridization conditions. For example, the more closely the oligonucleotide reflects a sequence that is present in the non-M17 nucleic acid, the higher the stringency of the assay conditions should be, although the stringency must not be too high so as to prevent hybridization of the oligonucleotides to the M17 specific nucleic acid sequence. Further, the lower the homology of the oligonucleotide to the M17 specific nucleic acid sequence, the lower the stringency of the assay conditions should be, although the stringency must not be too low to allow hybridization to non M17 specific nucleic acid sequences.

Oligonucleotides of the invention may be prepared by any of a variety of methods (see, for example, J. Sambrook et al., "Molecular Cloning: A Laboratory Manual", 1989, 2.sup.nd Ed., Cold Spring Harbour Laboratory Press: New York, N.Y.; "PCR Protocols: A Guide to Methods and Applications", 1990, M. A. Innis (Ed.), Academic Press: New York, N.Y.; P. Tijssen "Hybridization with Nucleic Acid Probes--Laboratory Techniques in Biochemistry and Molecular Biology (Parts I and II)", 1993, Elsevier Science; "PCR Strategies", 1995, M. A. Innis (Ed.), Academic Press: New York, N.Y.; and "Short Protocols in Molecular Biology", 2002, F. M. Ausubel (Ed.), 5.sup.th Ed., John Wiley & Sons: Secaucus, N.J.). For example, oligonucleotides may be prepared using any of a variety of chemical techniques well-known in the art, including, for example, chemical synthesis and polymerization based on a template as described, for example, in S. A. Narang et al., Meth. Enzymol. 1979, 68: 90-98; E. L. Brown et al., Meth. Enzymol. 1979, 68: 109-151; E. S. Belousov et al., Nucleic Acids Res. 1997, 25: 3440-3444; D. Guschin et al., Anal. Biochem. 1997, 250: 203-211; M. J. Blommers et al., Biochemistry, 1994, 33: 7886-7896; and K. Frenkel et al., Free Radic. Biol. Med. 1995, 19: 373-380; and U.S. Pat. No. 4,458,066.

For example, oligonucleotides may be prepared using an automated, solid-phase procedure based on the phosphoramidite approach. In such a method, each nucleotide is individually added to the 5'-end of the growing oligonucleotide chain, which is attached at the 3'-end to a solid support. The added nucleotides are in the form of trivalent 3'-phosphoramidites that are protected from polymerization by a dimethoxytriyl (or DMT) group at the 5'-position. After base-induced phosphoramidite coupling, mild oxidation to give a pentavalent phosphotriester intermediate and DMT removal provides a new site for oligonucleotide elongation. The oligonucleotides are then cleaved off the solid support, and the phosphodiester and exocyclic amino groups are deprotected with ammonium hydroxide. These syntheses may be performed on oligo synthesizers such as those commercially available from Perkin Elmer/Applied Biosystems, Inc. (Foster City, Calif.), DuPont (Wilmington, Del.) or Milligen (Bedford, Mass.). Alternatively, oligonucleotides can be custom made and ordered from a variety of commercial sources well-known in the art, including, for example, the Midland Certified Reagent Company (Midland, Tex.), ExpressGen, Inc. (Chicago, Ill.), Operon Technologies, Inc. (Huntsville, Ala.), and many others.

Purification of the oligonucleotides of the invention, where necessary or desirable, may be carried out by any of a variety of methods well-known in the art. Purification of oligonucleotides is typically performed either by native acrylamide gel electrophoresis, by anion-exchange HPLC as described, for example, by J. D. Pearson and F. E. Regnier (J. Chrom., 1983, 255: 137-149) or by reverse phase HPLC (G. D. McFarland and P. N. Borer, Nucleic Acids Res., 1979, 7: 1067-1080).

The sequence of oligonucleotides can be verified using any suitable sequencing method including, but not limited to, chemical degradation (A. M. Maxam and W. Gilbert, Methods of Enzymology, 1980, 65: 499-560), matrix-assisted laser desorption ionization time-of-flight (MALDI-TOF) mass spectrometry (U. Pieles et al., Nucleic Acids Res., 1993, 21: 3191-3196), mass spectrometry following a combination of alkaline phosphatase and exonuclease digestions (H. Wu and H. Aboleneen, Anal. Biochem., 2001, 290: 347-352), and the like.

As already mentioned above, modified oligonucleotides may be prepared using any of several means known in the art. Non-limiting examples of such modifications include methylation, "caps", substitution of one or more of the naturally occurring nucleotides with an analog, and internucleotide modifications such as, for example, those with uncharged linkages (e.g., methyl phosphonates, phosphotriesters, phosphoroamidates, carbamates, etc), or charged linkages (e.g., phosphorothioates, phosphorodithioates, etc). Oligonucleotides may contain one or more additional covalently linked moieties, such as, for example, proteins (e.g., nucleases, toxins, antibodies, signal peptides, poly-L-lysine, etc), intercalators (e.g., acridine, psoralen, etc), chelators (e.g., metals, radioactive metals, iron, oxidative metals, etc), and alkylators. The oligonucleotide may also be derivatized by formation of a methyl or ethyl phosphotriester or an alkyl phosphoramidate linkage. Furthermore, the oligonucleotide sequences of the present invention may also be modified with a label.

In certain embodiments, the detection probes or amplification primers or both probes and primers are labeled with a detectable agent or moiety before being used in amplification/detection assays. In certain embodiments, the detection probes are labeled with a detectable agent. Preferably, a detectable agent is selected such that it generates a signal which can be measured and whose intensity is related (e.g., proportional) to the amount of amplification products in the sample being analyzed.

The association between the oligonucleotide and detectable agent can be covalent or non-covalent. Labeled detection probes can be prepared by incorporation of or conjugation to a detectable moiety. Labels can be attached directly to the nucleic acid sequence or indirectly (e.g., through a linker). Linkers or spacer arms of various lengths are known in the art and are commercially available, and can be selected to reduce steric hindrance, or to confer other useful or desired properties to the resulting labeled molecules (see, for example, E. S. Mansfield et al., Mol. Cell. Probes, 1995, 9: 145-156).

Methods for labeling nucleic acid molecules are well-known in the art. For a review of labeling protocols, label detection techniques, and recent developments in the field, see, for example, L. J. Kricka, Ann. Clin. Biochem. 2002, 39: 114-129; R. P. van Gijlswijk et al., Expert Rev. Mol. Diagn. 2001, 1: 81-91; and S. Joos et al., J. Biotechnol. 1994, 35: 135-153. Standard nucleic acid labeling methods include: incorporation of radioactive agents, direct attachments of fluorescent dyes (L. M. Smith et al., Nucl. Acids Res., 1985, 13: 2399-2412) or of enzymes (B. A. Connoly and O. Rider, Nucl. Acids. Res., 1985, 13: 4485-4502); chemical modifications of nucleic acid molecules making them detectable immunochemically or by other affinity reactions (T. R. Broker et al., Nucl. Acids Res. 1978, 5: 363-384; E. A. Bayer et al., Methods of Biochem. Analysis, 1980, 26: 1-45; R. Langer et al., Proc. Natl. Acad. Sci. USA, 1981, 78: 6633-6637; R. W. Richardson et al., Nucl. Acids Res. 1983, 11: 6167-6184; D. J. Brigati et al., Virol. 1983, 126: 32-50; P. Tchen et al., Proc. Natl. Acad. Sci. USA, 1984, 81: 3466-3470; J. E. Landegent et al., Exp. Cell Res. 1984, 15: 61-72; and A. H. Hopman et al., Exp. Cell Res. 1987, 169: 357-368); and enzyme-mediated labeling methods, such as random priming, nick translation, PCR and tailing with terminal transferase (for a review on enzymatic labeling, see, for example, J. Temsamani and S. Agrawal, Mol. Biotechnol. 1996, 5: 223-232). More recently developed nucleic acid labeling systems include, but are not limited to: ULS (Universal Linkage System), which is based on the reaction of mono-reactive cisplatin derivatives with the N7 position of guanine moieties in DNA (R. J. Heetebrij et al., Cytogenet. Cell. Genet. 1999, 87: 47-52), psoralen-biotin, which intercalates into nucleic acids and upon UV irradiation becomes covalently bonded to the nucleotide bases (C. Levenson et al., Methods Enzymol. 1990, 184: 577-583; and C. Pfannschmidt et al., Nucleic Acids Res. 1996, 24: 1702-1709), photoreactive azido derivatives (C. Neves et al., Bioconjugate Chem. 2000, 11: 51-55), and DNA alkylating agents (M. G. Sebestyen et al., Nat. Biotechnol. 1998, 16: 568-576).

Any of a wide variety of detectable agents can be used in the practice of the present invention. Suitable detectable agents include, but are not limited to, various ligands, radionuclides (such as, for example, .sup.32P, sup.35S, .sup.3H, .sup.14C, .sup.125I, .sup.131I, and the like); fluorescent dyes (for specific exemplary fluorescent dyes, see below); chemiluminescent agents (such as, for example, acridinium esters, stabilized dioxetanes, and the like); spectrally resolvable inorganic fluorescent semiconductor nanocrystals (i.e., quantum dots), metal nanoparticles (e.g., gold, silver, copper and platinum) or nanoclusters; enzymes (such as, for example, those used in an ELISA, i.e., horseradish peroxidase, beta-galactosidase, luciferase, alkaline phosphatase); colorimetric labels (such as, for example, dyes, colloidal gold, and the like); magnetic labels (such as, for example, Dynabeads.TM.); and biotin, dioxigenin or other haptens and proteins for which antisera or monoclonal antibodies are available.

In certain embodiments, the inventive detection probes are fluorescently labeled. Numerous known fluorescent labeling moieties of a wide variety of chemical structures and physical characteristics are suitable for use in the practice of this invention. Suitable fluorescent dyes include, but are not limited to, fluorescein and fluorescein dyes (e.g., fluorescein isothiocyanine or FITC, naphthofluorescein, 4',5'-dichloro-2',7'-dimethoxy-fluorescein, 6 carboxyfluorescein or FAM), carbocyanine, merocyanine, styryl dyes, oxonol dyes, phycoerythrin, erythrosin, eosin, rhodamine dyes (e.g., carboxytetramethylrhodamine or TAMRA, carboxyrhodamine 6G, carboxy-X-rhodamine (ROX), lissamine rhodamine B, rhodamine 6G, rhodamine Green, rhodamine Red, tetramethylrhodamine or TMR), coumarin and coumarin dyes (e.g., methoxycoumarin, dialkylaminocoumarin, hydroxycoumarin and aminomethylcoumarin or AMCA), Oregon Green Dyes (e.g., Oregon Green 488, Oregon Green 500, Oregon Green 514), Texas Red, Texas Red-X, Spectrum Red.TM., Spectrum Green.TM., cyanine dyes (e.g., Cy-3.TM., Cy-5.TM., Cy-3.5.TM., Cy-5.5.TM.), Alexa Fluor dyes (e.g., Alexa Fluor 350, Alexa Fluor 488, Alexa Fluor 532, Alexa Fluor 546, Alexa Fluor 568, Alexa Fluor 594, Alexa Fluor 633, Alexa Fluor 660 and Alexa Fluor 680), BODIPY dyes (e.g., BODIPY FL, BODIPY R6G, BODIPY TMR, BODIPY TR, BODIPY 530/550, BODIPY 558/568, BODIPY 564/570, BODIPY 576/589, BODIPY 581/591, BODIPY 630/650, BODIPY 650/665), IRDyes (e.g., IRD40, IRD 700, IRD 800), and the like. For more examples of suitable fluorescent dyes and methods for linking or incorporating fluorescent dyes to nucleic acid molecules see, for example, "The Handbook of Fluorescent Probes and Research Products", 9th Ed., Molecular Probes, Inc., Eugene, Oreg. Fluorescent dyes as well as labeling kits are commercially available from, for example, Amersham Biosciences, Inc. (Piscataway, N.J.), Molecular Probes Inc. (Eugene, Oreg.), and New England Biolabs Inc. (Berverly, Mass.).

As mentioned, identification of M 17 may be carried out using an amplification reaction.

As used herein, the term "amplification" refers to a process that increases the representation of a population of specific nucleic acid sequences in a sample by producing multiple (i.e., at least 2) copies of the desired sequences. Methods for nucleic acid amplification are known in the art and include, but are not limited to, polymerase chain reaction (PCR) and ligase chain reaction (LCR). In a typical PCR amplification reaction, a nucleic acid sequence of interest is often amplified at least fifty thousand fold in amount over its amount in the starting sample. A "copy" or "amplicon" does not necessarily mean perfect sequence complementarity or identity to the template sequence. For example, copies can include nucleotide analogs such as deoxyinosine, intentional sequence alterations (such as sequence alterations introduced through a primer comprising a sequence that is hybridizable but not complementary to the template), and/or sequence errors that occur during amplification.

A typical amplification reaction is carried out by contacting a forward and reverse primer (a primer pair) to the sample DNA together with any additional amplification reaction reagents under conditions which allow amplification of the target sequence.

The terms "forward primer" and "forward amplification primer" are used herein interchangeably, and refer to a primer that hybridizes (or anneals) to the target (template strand). The terms "reverse primer" and "reverse amplification primer" are used herein interchangeably, and refer to a primer that hybridizes (or anneals) to the complementary target strand. The forward primer hybridizes with the target sequence 5' with respect to the reverse primer.

The term "amplification conditions", as used herein, refers to conditions that promote annealing and/or extension of primer sequences. Such conditions are well-known in the art and depend on the amplification method selected. Thus, for example, in a PCR reaction, amplification conditions generally comprise thermal cycling, i.e., cycling of the reaction mixture between two or more temperatures. In isothermal amplification reactions, amplification occurs without thermal cycling although an initial temperature increase may be required to initiate the reaction. Amplification conditions encompass all reaction conditions including, but not limited to, temperature and temperature cycling, buffer, salt, ionic strength, and pH, and the like.

As used herein, the term "amplification reaction reagents", refers to reagents used in nucleic acid amplification reactions and may include, but are not limited to, buffers, reagents, enzymes having reverse transcriptase and/or polymerase activity or exonuclease activity, enzyme cofactors such as magnesium or manganese, salts, nicotinamide adenine dinuclease (NAD) and deoxynucleoside triphosphates (dNTPs), such as deoxyadenosine triphospate, deoxyguanosine triphosphate, deoxycytidine triphosphate and thymidine triphosphate. Amplification reaction reagents may readily be selected by one skilled in the art depending on the amplification method used.

According to this aspect of the present invention, the amplifying may be effected using techniques such as polymerase chain reaction (PCR), which includes, but is not limited to Allele-specific PCR, Assembly PCR or Polymerase Cycling Assembly (PCA), Asymmetric PCR, Helicase-dependent amplification, Hot-start PCR, Intersequence-specific PCR (ISSR), Inverse PCR, Ligation-mediated PCR, Methylation-specific PCR (MSP), Miniprimer PCR, Multiplex Ligation-dependent Probe Amplification, Multiplex-PCR,Nested PCR, Overlap-extension PCR, Quantitative PCR (Q-PCR), Reverse Transcription PCR (RT-PCR), Solid Phase PCR: encompasses multiple meanings, including Polony Amplification (where PCR colonies are derived in a gel matrix, for example), Bridge PCR (primers are covalently linked to a solid-support surface), conventional Solid Phase PCR (where Asymmetric PCR is applied in the presence of solid support bearing primer with sequence matching one of the aqueous primers) and Enhanced Solid Phase PCR (where conventional Solid Phase PCR can be improved by employing high Tm and nested solid support primer with optional application of a thermal 'step' to favour solid support priming), Thermal asymmetric interlaced PCR (TAIL-PCR), Touchdown PCR (Step-down PCR), PAN-AC and Universal Fast Walking.

The PCR (or polymerase chain reaction) technique is well-known in the art and has been disclosed, for example, in K. B. Mullis and F. A. Faloona, Methods Enzymol., 1987, 155: 350-355 and U.S. Pat. Nos. 4,683,202; 4,683,195; and 4,800,159 (each of which is incorporated herein by reference in its entirety). In its simplest form, PCR is an in vitro method for the enzymatic synthesis of specific DNA sequences, using two oligonucleotide primers that hybridize to opposite strands and flank the region of interest in the target DNA. A plurality of reaction cycles, each cycle comprising: a denaturation step, an annealing step, and a polymerization step, results in the exponential accumulation of a specific DNA fragment ("PCR Protocols: A Guide to Methods and Applications", M. A. Innis (Ed.), 1990, Academic Press: New York; "PCR Strategies", M. A. Innis (Ed.), 1995, Academic Press: New York; "Polymerase chain reaction: basic principles and automation in PCR: A Practical Approach", McPherson et al. (Eds.), 1991, IRL Press: Oxford; R. K. Saiki et al., Nature, 1986, 324: 163-166). The termini of the amplified fragments are defined as the 5' ends of the primers. Examples of DNA polymerases capable of producing amplification products in PCR reactions include, but are not limited to: E. coli DNA polymerase I, Klenow fragment of DNA polymerase I, T4 DNA polymerase, thermostable DNA polymerases isolated from Thermus aquaticus (Taq), available from a variety of sources (for example, Perkin Elmer), Thermus thermophilus (United States Biochemicals), Bacillus stereothermophilus (Bio-Rad), or Thermococcus litoralis ("Vent" polymerase, New England Biolabs). RNA target sequences may be amplified by reverse transcribing the mRNA into cDNA, and then performing PCR (RT-PCR), as described above. Alternatively, a single enzyme may be used for both steps as described in U.S. Pat. No. 5,322,770.

The duration and temperature of each step of a PCR cycle, as well as the number of cycles, are generally adjusted according to the stringency requirements in effect. Annealing temperature and timing are determined both by the efficiency with which a primer is expected to anneal to a template and the degree of mismatch that is to be tolerated. The ability to optimize the reaction cycle conditions is well within the knowledge of one of ordinary skill in the art. Although the number of reaction cycles may vary depending on the detection analysis being performed, it usually is at least 15, more usually at least 20, and may be as high as 60 or higher. However, in many situations, the number of reaction cycles typically ranges from about 20 to about 40.

The denaturation step of a PCR cycle generally comprises heating the reaction mixture to an elevated temperature and maintaining the mixture at the elevated temperature for a period of time sufficient for any double-stranded or hybridized nucleic acid present in the reaction mixture to dissociate. For denaturation, the temperature of the reaction mixture is usually raised to, and maintained at, a temperature ranging from about 85 °C. to about 100 °C, usually from about 90°C to about 98 °C, and more usually from about 93 °C. to about 96 °C. for a period of time ranging from about 3 to about 120 seconds, usually from about 5 to about 30 seconds.

Following denaturation, the reaction mixture is subjected to conditions sufficient for primer annealing to template DNA present in the mixture. The temperature to which the reaction mixture is lowered to achieve these conditions is usually chosen to provide optimal efficiency and specificity, and generally ranges from about 50 °C to about °C, usually from about 55 °C. to about 70 °C, and more usually from about 60 °C to about 68 °C. Annealing conditions are generally maintained for a period of time ranging from about 15 seconds to about 30 minutes, usually from about 30 seconds to about 5 minutes.

Following annealing of primer to template DNA or during annealing of primer to template DNA, the reaction mixture is subjected to conditions sufficient to provide for polymerization of nucleotides to the primer's end in a such manner that the primer is extended in a 5' to 3' direction using the DNA to which it is hybridized as a template, (i.e., conditions sufficient for enzymatic production of primer extension product). To achieve primer extension conditions, the temperature of the reaction mixture is typically raised to a temperature ranging from about 65°C to about 75 °C, usually from about 67 °C. to about 73 °C, and maintained at that temperature for a period of time ranging from about 15 seconds to about 20 minutes, usually from about 30 seconds to about 5 minutes.

The above cycles of denaturation, annealing, and polymerization may be performed using an automated device typically known as a thermal cycler or thermocycler. Thermal cyclers that may be employed are described in U.S. Pat. Nos. 5,612,473; 5,602,756; 5,538,871; and 5,475,610 (each of which is incorporated herein by reference in its entirety). Thermal cyclers are commercially available, for example, from Perkin Elmer-Applied Biosystems (Norwalk, Conn.), BioRad (Hercules, Calif.), Roche Applied Science (Indianapolis, Ind.), and Stratagene (La Jolla, Calif.).

Amplification products obtained using primers of the present invention may be detected using agarose gel electrophoresis and visualization by ethidium bromide staining and exposure to ultraviolet (UV) light or by sequence analysis of the amplification product.

According to one embodiment, the amplification and quantification of the amplification product may be effected in real-time (qRT-PCR). Typically, QRT-PCR methods use double stranded DNA detecting molecules to measure the amount of amplified product in real time.

As used herein the phrase "double stranded DNA detecting molecule" refers to a double stranded DNA interacting molecule that produces a quantifiable signal (e.g., fluorescent signal). For example such a double stranded DNA detecting molecule can be a fluorescent dye that (1) interacts with a fragment of DNA or an amplicon and (2) emits at a different wavelength in the presence of an amplicon in duplex formation than in the presence of the amplicon in separation. A double stranded DNA detecting molecule can be a double stranded DNA intercalating detecting molecule or a primer-based double stranded DNA detecting molecule.

A double stranded DNA intercalating detecting molecule is not covalently linked to a primer, an amplicon or a nucleic acid template. The detecting molecule increases its emission in the presence of double stranded DNA and decreases its emission when duplex DNA unwinds. Examples include, but are not limited to, ethidium bromide, YO-PRO-1, Hoechst 33258, SYBR Gold, and SYBR Green I. Ethidium bromide is a fluorescent chemical that intercalates between base pairs in a double stranded DNA fragment and is commonly used to detect DNA following gel electrophoresis. When excited by ultraviolet light between 254 nm and 366 nm, it emits fluorescent light at 590 nm. The DNA-ethidium bromide complex produces about 50 times more fluorescence than ethidium bromide in the presence of single stranded DNA. SYBR Green I is excited at 497 nm and emits at 520 nm. The fluorescence intensity of SYBR Green I increases over 100 fold upon binding to double stranded DNA against single stranded DNA. An alternative to SYBR Green I is SYBR Gold introduced by Molecular Probes Inc. Similar to SYBR Green I, the fluorescence emission of SYBR Gold enhances in the presence of DNA in duplex and decreases when double stranded DNA unwinds. However, SYBR Gold's excitation peak is at 495 nm and the emission peak is at 537 nm. SYBR Gold reportedly appears more stable than SYBR Green I. Hoechst 33258 is a known bisbenzimide double stranded DNA detecting molecule that binds to the AT rich regions of DNA in duplex. Hoechst 33258 excites at 350 nm and emits at 450 nm. YO-PRO-1, exciting at 450 nm and emitting at 550 nm, has been reported to be a double stranded DNA specific detecting molecule. In a particular embodiment of the present invention, the double stranded DNA detecting molecule is SYBR Green I.

A primer-based double stranded DNA detecting molecule is covalently linked to a primer and either increases or decreases fluorescence emission when amplicons form a duplex structure. Increased fluorescence emission is observed when a primer-based double stranded DNA detecting molecule is attached close to the 3' end of a primer and the primer terminal base is either dG or dC. The detecting molecule is quenched in the proximity of terminal dC-dG and dG-dC base pairs and dequenched as a result of duplex formation of the amplicon when the detecting molecule is located internally at least 6 nucleotides away from the ends of the primer. The dequenching results in a substantial increase in fluorescence emission. Examples of these type of detecting molecules include but are not limited to fluorescein (exciting at 488 nm and emitting at 530 nm), FAM (exciting at 494 nm and emitting at 518 nm), JOE (exciting at 527 and emitting at 548), HEX (exciting at 535 nm and emitting at 556 nm), TET (exciting at 521 nm and emitting at 536 nm), Alexa Fluor 594 (exciting at 590 nm and emitting at 615 nm), ROX (exciting at 575 nm and emitting at 602 nm), and TAMRA (exciting at 555 nm and emitting at 580 nm). In contrast, some primer-based double stranded DNA detecting molecules decrease their emission in the presence of double stranded DNA against single stranded DNA. Examples include, but are not limited to, rhodamine, and BODIPY-FI (exciting at 504 nm and emitting at 513 nm). These detecting molecules are usually covalently conjugated to a primer at the 5' terminal dC or dG and emit less fluorescence when amplicons are in duplex. It is believed that the decrease of fluorescence upon the formation of duplex is due to the quenching of guanosine in the complementary strand in close proximity to the detecting molecule or the quenching of the terminal dC-dG base pairs.

According to one embodiment, the primer-based double stranded DNA detecting molecule is a 5' nuclease probe. Such probes incorporate a fluorescent reporter molecule at either the 5' or 3' end of an oligonucleotide and a quencher at the opposite end. The first step of the amplification process involves heating to denature the double stranded DNA target molecule into a single stranded DNA. During the second step, a forward primer anneals to the target strand of the DNA and is extended by Taq polymerase. A reverse primer and a 5' nuclease probe then anneal to this newly replicated strand.

In this embodiment, at least one of the primer pairs or 5' nuclease probe should hybridize with a unique M 17 sequence. The polymerase extends and cleaves the probe from the target strand. Upon cleavage, the reporter is no longer quenched by its proximity to the quencher and fluorescence is released. Each replication will result in the cleavage of a probe. As a result, the fluorescent signal will increase proportionally to the amount of amplification product.

The present invention contemplates various scenarios that would lead to the amplification of a unique M17 sequence:
According to the first scenario, both the forward primer and the reverse primer hybridize to a unique M17 sequence.

In the second scenario, only one of the primers of the primer pair hybridizes to a unique M 17 sequence. The primer pair hybridizes to a non-unique M 17 sequence. The primer pair may or may not be capable of hybridizing to non M17 sequences.

In the third scenario, neither of the primers hybridize to a unique M17 sequence, but both hybridize with a sequence that flanks the unique sequence. In such a scenario, the amplified sequence may be detected due to its unique size.

As shown in Example 2, herein below, primer pair (SEQ ID NOs: 37 and 38) and primer pair (SEQ ID NOs: 39 and 40) which hybridized with Fragment 33 of M17 were capable of distinguishing between M17 and other E.coli. In addition, primer pair (SEQ ID NO: 45 and 46) which hybridized with Fragment 44 was capable of distinguishing between M17 and other E.coli. The three primer pairs could also successfully identify M17 in a spiked fecal sample.

Below is a table (Table 9) listing additional primers contemplated by the present invention that may be used to identify the sequence as set forth in SEQ ID NO: 1 (Fragment 1). In the Table, and subsequent Tables 10-43, the primer pairs which generate a 60-200 bp product are typically used for real-time PCR, whereas the primers which generate a 300-500 bp product are typically used for standard PCR.

**Table 9**

| | SEQ ID NO: | 5' primer | SEQ ID NO: | 3' primer | coordinates | coordinates | pcr product length |
|---|---|---|---|---|---|---|---|
| flanking region | 62 | CCAGCT CATCGT GTTTTCC | 63 | GGTGTC GATGCG ATAAAA CC | 4 | 1205 | 1201 |
| 60bp-200bp | 64 | AACCCT ATCGGT CAGGCT CT | 65 | GTTGCT CAAGTG TCGCCA TA | 78 | 269 | 191 |
| | 66 | CCTGAT GGCGGA AAAGAA TA | 67 | GGTGTC GATGCG ATAAAA CC | 1016 | 1205 | 189 |
| | 68 | TCCGGG TTGATA ACCATC AT | 69 | TGACAC CAACGC CAATAA GA | 207 | 305 | 98 |
| | 70 | TCGGTC AGGCTC TCTCAA AT | 71 | CGGAAG CTGCGA ATTTTAT T | 85 | 210 | 125 |
| | 72 | CCTGAT GGCGGA AAAGAA TA | 73 | GCTTTTA GGGCGC TGAGTT A | 1016 | 1111 | 95 |
| | 74 | ATCGGT CAGGCT CTCTCA AA | 75 | GTTGCT CAAGTG TCGCCA TA | 84 | 269 | 185 |
| | 76 | TCCGGG TTGATA ACCATC AT | 77 | ACCACT CTGGTC CTTCAT GC | 207 | 703 | 496 |
| 300bp-500bp | 78 | TCGGTC AGGCTC TCTCAA AT | 79 | GATCCC CATCAT GGAAAC AT | 85 | 586 | 501 |
| | 80 | TATGGC GACACT TGAGCA AC | 81 | TTCCGG GAAGAT AAATGG TG | 250 | 740 | 490 |
| | 82 | TCTTATT GGCGTT GGTGTC A | 83 | GCAGTA TCCGGT TTTTCAG C | 286 | 886 | 600 |
| | 84 | AACCCT ATCGGT CAGGCT CT | 85 | GCGTAG TGAATG CGGATG TA | 78 | 470 | 392 |

Table 10 lists additional primers contemplated by the present invention that may be used to identify the sequence as set forth in SEQ ID NO: 2 (Fragment 2).

**Table 10**

| | SEQ ID NO: | 5' primer | SEQ ID NO: | 3' primer | coordinates | coordinates | pcr product length |
|---|---|---|---|---|---|---|---|
| flanking region | 86 | GGCGCTC ATCGTAT TGTGTA | 87 | GCGGAA TAACGA GTCCAC AT | -19 | 186 | 205 |
| 60bp-200bp | 88 | AGAGCCT CGAAGAT GTTTGC | 89 | ACATTTT GCTGTG GACCTT G | 21 | 170 | 149 |
| | 90 | GCCTCGA AGATGTT TGCTCT | 91 | CGGGCA TAATGA CCTTTTT C | 24 | 148 | 124 |
| | 92 | AGAGCCT CGAAGAT GTTTGC | 93 | CTGTAG GCCAGT GAGCGT TT | 21 | 119 | 98 |

Table 11 lists additional primers contemplated by the present invention that may be used to identify the sequence as set forth in SEQ ID NO: 3 (Fragment 3).

**Table 11**

| | SEQ ID NO: | 5'primer | SEQ ID NO: | 3' primer | coordinates | coordinates | pcr product length |
|---|---|---|---|---|---|---|---|
| flanking region | 94 | CGCAGAC CTACAGG AAGCAT | 95 | GAGGGG AGGCAA AAGAAA AC | -25 | 1718 | 1743 |
| 60bp-200bp | 96 | CCGGAAA AATTAGC GTTGAA | 97 | CAATTC TCCGGC ATCAAG TT | 1030 | 1133 | 103 |
| | 98 | TGTGGTT GGACTCA TGCAAT | 99 | TTGGCA CTAATC GCCTAA CC | 1286 | 1401 | 115 |
| | 100 | CAAAAGG AGGGCGA TAATGA | 101 | AGCCCA ATGCTG ACTTGA AC | 1151 | 1248 | 97 |
| | 102 | CGGGTGT TGTCCTA ACTGCT | 103 | GAGTGG TCATTG GCCTCA TT | 1258 | 1363 | 105 |
| 300bp-500bp | 104 | ATACCGC CCAATAG GGAAAG | 105 | GAGTGG TCATTG GCCTCA TT | 770 | 1363 | 593 |
| | 106 | ATACCGC CCAATAG GGAAAG | 107 | TCATTAT CGCCCT CCTTTTG | 770 | 1170 | 400 |
| | 108 | GGGGAAA TAACGGG AAAAGA | 109 | GGGCTT GATCAT TTGTGCT T | 999 | 1506 | 507 |
| | 110 | GCGATAA CTGGGCA AATGAT | 111 | ATTGCA TGAGTC CAACCA CA | 817 | 1305 | 488 |

Table 12 lists additional primers contemplated by the present invention that may be used to identify the sequence as set forth in SEQ ID NO: 4 (Fragment 4).

**Table 12**

| | SEQ ID NO: | 5' primer | SEQ ID NO: | 3' primer | coordinates | coordinates | pcr product length |
|---|---|---|---|---|---|---|---|
| flanking region | 112 | TCTTTCCG ATGGCTC AGTCT | 113 | CAGACC CGTTTTG CAGAGA T | -26 | 215 | 241 |
| | 114 | GATGGCT CAGTCTG GAAAGG | 115 | TTGAGG CTCCCG TAACAT TC | -19 | 239 | 258 |
| 60bp-200bp | 116 | GGAATGG TGCTGTTT CCATT | 117 | CAGACC CGTTTTG CAGAGA T | 114 | 215 | 101 |
| | 118 | GCACGTG TCACACT GAAAAA | 119 | AAACAG CACCAT TCCACA CA | 29 | 128 | 99 |
| | 120 | AAAACTC CAGCTGG GATGG | 121 | CGTTAA TGGAAA CAGCAC CA | 45 | 137 | 92 |
| | 122 | CGTAGGT AAAGGTC TGGATGG | 123 | GAACCG AGCCCA TTGGTA CT | 4 | 176 | 172 |
| | 124 | GCTGGGA TGGTGAT GTCAAT | 125 | GAACCG AGCCCA TTGGTA CT | 54 | 176 | 122 |
| | 126 | TGCTGTTT TTCTGAC GGATG | 127 | GAACCG AGCCCA TTGGTA CT | 85 | 176 | 91 |
| | 128 | AAAACTC CAGCTGG GATGG | 129 | AAACAG CACCAT TCCACA CA | 45 | 128 | 83 |

Table 13 lists additional primers contemplated by the present invention that may be used to identify the sequence as set forth in SEQ ID NO: 5 (Fragment 5).

**Table 13**

| | SEQ ID NO: | 5' primer, | SEQ ID NO: | 3' primer | coordinates | coordinates | pcr product length |
|---|---|---|---|---|---|---|---|
| flanking region | 130 | AGAGTCC GTCTCCC ATAGCC | 131 | ACTTGC CCAGGT TCTTGA AA | -31 | 432 | 463 |
| | 132 | GTCCGTC TCCCATA GCCTTA | 133 | ACTTGC CCAGGT TCTTGA AA | -28 | 432 | 460 |
| | 134 | AAGTGTG CCATTGC CTTTCT | 135 | TATTAA GGCGCC ACAACT GG | 210 | 327 | 117 |
| 60bp-200bp | 136 | CAACTGG TACTGAG CGTTCG | 137 | GAAAAA GAGCGG GTGAAC AA | 153 | 251 | 98 |
| | 138 | GTTAATA TCGCGCG TCCATC | 139 | CTATCCT TGACCC GACGAA C | 96 | 187 | 91 |
| | 140 | CCATTGC CTTTCTGC TTGTT | 141 | TATTAA GGCGCC ACAACT GG | 217 | 327 | 110 |
| 300bp-500bp | 142 | TTGCTTCA TCATCGC CATT | 143 | CGCAAT TAGTGA CCAGAT CG | 8 | 411 | 403 |
| | 144 | CAACTGG TACTGAG CGTTCG | 145 | ACTTGC CCAGGT TCTTGA AA | 153 | 432 | 279 |

Table 14 lists additional primers contemplated by the present invention that may be used to identify the sequence as set forth in SEQ ID NO: 6 (Fragment 6).

**Table 14**

| | SEQ ID NO: | 5' primer | SEQ ID NO: | 3' primer | coordinates | coordinates | pcr product length |
|---|---|---|---|---|---|---|---|
| flanking region | 146 | ATGGGCA TCACGCA AGATA | 147 | AATCGT GTCCAG CCATTTT G | -43 | 579 | 622 |
| 60bp-200bp | 148 | GTTGTGG AGCAGCT TGAACA | 149 | GATATC CTGCGG ACGCTC TA | 264 | 374 | 110 |
| | 150 | GTTGTGG AGCAGCT TGAACA | 151 | GCTCTA TCCCTG CTGAAT GC | 264 | 360 | 96 |
| 300bp-500bp | 152 | CCAACTA CCCACCC TGTGTC | 153 | CCGAAT CGTTGA CTCGTA TG | 49 | 512 | 463 |
| | 154 | CCAACTA CCCACCC TGTGTC | 155 | CAACCG CTCGAA CACCTT AG | 49 | 435 | 386 |

Table 15 lists additional primers contemplated by the present invention that may be used to identify the sequence as set forth in SEQ ID NO: 7 (Fragment 7).

**Table 15**

| | SEQ ID NO: | 5' primer | SEQ ID NO: | 3' primer | coordinates | coordinates | pcr product length |
|---|---|---|---|---|---|---|---|
| flanking region | 156 | CCACGGT ATATCCC TCGGTA | 157 | CTTCGG GATTCA TGGTTA GC | -44 | 1366 | 1410 |
| | 158 | GGCAGTC TTTCTGGC ATAGG | 159 | ATTGGG TGAGCC TGATTG AA | 759 | 1389 | 630 |
| 60bp-200bp | 160 | GGTGCTA GACTCTG GGCTTG | 161 | GATTTC CCACGC TGTCAC TT | 428 | 551 | 123 |
| | 162 | TGATCAG TGATTGC GTGACA | 163 | AAGCCC AGAGTC TAGCAC CA | 346 | 446 | 100 |
| 300bp-500bp | 164 | GGTGCTA GACTCTG GGCTTG | 165 | AGCATA CCCAAA ATGGCA AC | 428 | 926 | 498 |
| | 166 | AGGTCGA TCTACGC GAAAAA | 167 | GCAAAG CCTATG CCAGAA AG | 188 | 784 | 596 |
| | 168 | ATCAGAA CCCGACG ACAAAG | 169 | TCCAGG CTTCGA GGAGAG TA | 456 | 854 | 398 |

Table 16 lists additional primers contemplated by the present invention that may be used to identify the sequence as set forth in SEQ ID NO: 8 (Fragment 8).

**Table 16**

| | SEQ ID NO: | 5' primer | SEQ ID NO: | 3' primer | coordinates | coordinates | pcr product length |
|---|---|---|---|---|---|---|---|
| flanking region | 170 | AACAAGTC CAGTGCAT CACG | 171 | GTCAGG ACGATT TCGTTG GT | -34 | 827 | 861 |
| 60bp-200bp | 172 | CAGGTATG GCAAGGAC GATT | 173 | ATGAAC CCTTGC GAATCA AG | 10 | 128 | 118 |
| | 174 | CTTGATTC GCAAGGGT TCAT | 175 | GGAGTT ACGCGA GTTGCTT T | 109 | 208 | 99 |
| 300bp-500bp | 176 | TTCCATGA CTCGTCAG CAAG | 177 | ATGTCC CATATA GCCCGT TG | 29 | 537 | 508 |
| | 178 | CTTGATTC GCAAGGGT TCAT | 179 | CTTTCTC GGACGA ACGATT T | 109 | 713 | 604 |
| | 180 | TTGCCAAC GATACAAA TCCA | 181 | TCGAAA TTGACC CGAAAC TC | 250 | 653 | 403 |

Table 17 lists additional primers contemplated by the present invention that may be used to identify the sequence as set forth in SEQ ID NO: 9 (Fragment 9).

**Table 17**

| | SEQ ID NO: | 5' primer | SEQ ID NO: | 3' primer | coordinates | coordinates | pcr product length |
|---|---|---|---|---|---|---|---|
| flanking region | 182 | TTTTtGGAA TTGGCAGC ATC | 183 | GCGTTG TGTTCTT CTGTGG A | -24 | 377 | 401 |
| 60bp-200bp | 184 | GCTTTGGC TTAAGGGC TTTT | 185 | AATTGA GCGTGA GGTTTTC G | 150 | 271 | 121 |
| | 186 | CCACATGA AAGAGACG GTCA | 187 | GGGCGC TATTGA TACTCA GG | 39 | 139 | 100 |
| 300bp-500bp | 188 | AAGCCTGG CCTGTACG TTTA | 189 | ACCTGG TTTCAA AGGGTT GG | 15 | 331 | 316 |

Table 18 lists additional primers contemplated by the present invention that may be used to identify the sequence as set forth in SEQ ID NO: 10 (Fragment 10).

**Table 18**

| | SEQ ID NO: | 5' primer | SEQ ID NO: | 3' primer | coordinates | coordinates | pcr product length |
|---|---|---|---|---|---|---|---|
| flanking region | 190 | GCAGGGA TCCAGCT AATTGA | 191 | AACAGG GTTTGG GACATT TTT | 26 | 576 | 550 |
| 60bp-200bp | 192 | CGATTCG CAAGAAT CTGGA | 193 | TTTTGCC TCTGTC ACCATC A | -34 | 62 | 96 |
| | 194 | AAAATGC AGGGATC CAGCTA | 195 | GCGGAT AAGAAA AGACAA TAGCC | 21 | 117 | 96 |
| | 196 | CGAGCTG ATAATAA ATTATGG AACC | 197 | GCACTG CAGAGC CAGAGA TA | 317 | 419 | 102 |
| | 198 | CGAGCTG ATAATAA ATTATGG AACC | 199 | ATCTCTC GCGGGT AGTTGA G | 317 | 439 | 122 |
| 300bp-500bp | 200 | TTGATGG TGACAGA GGCAAA | 201 | ATCTCTC GCGGGT AGTTGA G | 42 | 439 | 397 |

Table 19 lists additional primers contemplated by the present invention that may be used to identify the sequence as set forth in SEQ ID NO: 11 (Fragment 11).

**Table 19**

| | SEQ ID NO: | 5' primer | SEQ ID NO: | 3' primer | coordinates | coordinates | pcr product length |
|---|---|---|---|---|---|---|---|
| flanking region | 202 | CTCGCTTT TCCCAAG TGAAT | 203 | GAGATG AGTTTC GGGAGC AG | -48 | 497 | 545 |
| 60bp-200bp | 204 | GGAAACA AACCGAC TGGAAA | 205 | CACCCC ACCAGA ACCATA AA | 9 | 114 | 105 |
| | 206 | GAATACT GATGCGG CAGTCC | 207 | TGGGCA ATGATT GTTTGT GT | 306 | 392 | 86 |
| 300bp-500bp | 208 | GGCTTAT GGGAAAG CACTCA | 209 | TGGGCA ATGATT GTTTGT GT | 42 | 392 | 350 |

Table 20 lists additional primers contemplated by the present invention that may be used to identify the sequence as set forth in SEQ ID NO: 12 (Fragment 12).

**Table 20**

| | SEQ ID NO: | 5' primer | SEQ ID NO: | 3' primer | coordinates | coordinates | pcr product length |
|---|---|---|---|---|---|---|---|
| flanking region | 210 | CGATTGT GGCTGAA ATTGAA | 211 | AGCTAG ATCGCC ACTGGA AA | -34 | 464 | 498 |
| 60bp-200bp | 212 | GCGACAA CATCAGA AAACGA | 213 | TTGCTC ATGGCG AAATAA CA | 255 | 356 | 101 |
| | 214 | GCCCACT TTTTCCAG TCAAA | 215 | GCTAGA TCGCCA CTGGAA AC | 372 | 463 | 91 |
| | 216 | AGGTGCA GAAATGA GCGAGT | 217 | TTTGCC AATATT CCCCAG AG | -7 | 80 | 87 |
| 300bp-500bp | 218 | GCGACAA CATCAGA AAACGA | 219 | AGCTAG ATCGCC ACTGGA AA | 255 | 464 | 209 |
| | 220 | AGGTGCA GAAATGA GCGAGT | 221 | GGTTTT ACGACA TCCTCAT CG | -7 | 292 | 299 |

Table 21 lists additional primers contemplated by the present invention that may be used to identify the sequence as set forth in SEQ ID NO: 13 (Fragment 13).

**Table 21**

| | SEQ ID NO: | 5' primer | SEQ ID NO: | 3' primer | coordinates | coordinates | pcr product length |
|---|---|---|---|---|---|---|---|
| flanking region | 222 | ACTTTGG GAGAAGT GGCTCA | 223 | TTGGTC AGGGTG TCGAAT TT | -17 | 443 | 460 |
| 60bp-200bp | 224 | TGCAAAA CAGAGCA GGAAAA | 225 | GGCGGA TATTCCC AATCAA T | 71 | 169 | 98 |
| | 226 | GTGGAAC AAATGGC GATGTA | 227 | CGGCAT TTTTGCT CCTTTA G | 206 | 339 | 133 |
| | 228 | TGCAAAA CAGAGCA GGAAAA | 229 | CACCGA TACAAT CTGCTT GC | 71 | 208 | 137 |
| 300bp-500bp | 230 | ATCTGCT GCAAAAC AGAGCA | 231 | GAGTGG CGGTAC AGGGAT T | 65 | 390 | 325 |

Table 22 lists additional primers contemplated by the present invention that may be used to identify the sequence as set forth in SEQ ID NO: 14 (Fragment 14).

**Table 22**

| | SEQ ID NO: | 5' primer | SEQ ID NO: | 3' primer | coordinates | coordinates | pcr product length |
|---|---|---|---|---|---|---|---|
| flanking region | 232 | GCAGGGC TTGGAGA TCATT | 233 | GAGGGC GGGATT TCTACTT C | -29 | 669 | 698 |
| 60bp-200bp | 234 | AGCCAGG CAAAAGG ATACAA | 235 | CATGCC AATCCA TCACTG AA | 538 | 637 | 99 |
| | 236 | CATTCGT GCAAGCA AGAGAA | 237 | TTTCAA GACCTG CACCTT CA | 153 | 254 | 101 |
| | 238 | AATTATG GGAGCAA GGCAGA | 239 | CCTCTA GGATCC GGCTCA AT | 126 | 213 | 87 |
| 300bp-500bp | 240 | TGAAGGT GCAGGTC TTGAAA | 241 | GGATTC CTCAGC GCTAAC TG | 235 | 688 | 453 |

Table 23 lists additional primers contemplated by the present invention that may be used to identify the sequence as set forth in SEQ ID NO: 15 (Fragment 15).

**Table 23**

| | SEQ ID NO: | 5' primer | SEQ ID NO: | 3' primer | coordinates | coordinates | pcr product length |
|---|---|---|---|---|---|---|---|
| flanking region | 242 | GCGCAAC GGATAAG GATAAA | 243 | CCGCCA TATCGTT GTTATA CG | -42 | 846 | 888 |
| 60bp-200bp | 244 | GGCATTA ACCCGTC TTCTGA | 245 | CTGTTTT CGGAAA TGCCTG T | 580 | 695 | 115 |
| | 246 | GGGCAGA CTATCAG GCAGAG | 247 | TGCTGT GCGTAA TTGTGG AT | 132 | 235 | 103 |
| 300bp-500bp | 248 | GGGCAGA CTATCAG GCAGAG | 249 | CGAGTT TGATAC GCCCTT CT | 132 | 620 | 488 |
| | 250 | GGAGGTT CAGCAAC AACGAT | 251 | GCTCGT CAACAC TTCCTTC C | 53 | 659 | 606 |
| | 252 | CAATTAC GCACAGC AACTGG | 253 | CGAGTT TGATAC GCCCTT CT | 221 | 620 | 399 |

Table 24 lists additional primers contemplated by the present invention that may be used to identify the sequence as set forth in SEQ ID NO: 16 (Fragment 16).

**Table 24**

| | SEQ ID NO: | 5' primer | SEQ ID NO: | 3' primer | coordinates | coordinates | pcr product length |
|---|---|---|---|---|---|---|---|
| flanking region | 254 | ACGcTCCC CCTGTAA AACA | 255 | GGATGT GATCCA TCTGGT GA | -14 | 387 | 401 |
| 60bp-200bp | 256 | GAGGCAT AAACCCA TGCTGT | 257 | ATGGAT TTGCCT GTCTGA CC | 24 | 122 | 98 |
| | 258 | TGGCATA ACCGATG AACAGA | 259 | AGACAA CGGGCT GTAGCA TT | 201 | 318 | 117 |
| 300bp-500bp | 260 | CGTACCT GGAGGCA TAAACC | 261 | GGATGT GATCCA TCTGGT GA | 16 | 387 | 371 |

Table 25 lists additional primers contemplated by the present invention that may be used to identify the sequence as set forth in SEQ ID NO: 17 (Fragment 17).

**Table 25**

| | SEQ ID NO: | 5' primer | SEQ ID NO: | 3' primer | coordinates | coordinates | pcr product length |
|---|---|---|---|---|---|---|---|
| flanking region | 262 | AACAGTT CCTGAGT GTAATCA CCA | 263 | ACTAAT GCGAGT GCTTGC TG | -46 | 316 | 362 |
| 60bp-200bp | 264 | ATGTTCC GATTCGC AATGTT | 265 | CCAGCA ATCGTTT GTGTTT G | 0 | 98 | 98 |
| | 266 | ATTCGCC CGAACAT ACAAAC | 267 | GCCAAT ATGACT CACCCA CA | 64 | 162 | 98 |
| | 268 | AATATAC CCGCTGG TCCAAA | 269 | CCGGGT ATGGAA ATCACT TG | 209 | 283 | 74 |
| 300bp-500bp | 270 | ATGTTCC GATTCGC | 271 | ACTAAT GCGAGT | 0 | 316 | 316 |
| | | AATGTT | | GCTTGC TG | | | |
| | 272 | ATTCGCC CGAACAT ACAAAC | 273 | ACTAAT GCGAGT GCTTGC TG | 64 | 316 | 252 |

Table 26 lists additional primers contemplated by the present invention that may be used to identify the sequence as set forth in SEQ ID NO: 18 (Fragment 18).

**Table 26**

| | SEQ ID NO: | 5' primer | SEQ ID NO: | 3' primer | coordinates | coordinates | pcr product length |
|---|---|---|---|---|---|---|---|
| flanking region | 274 | GTACTTTC ACCTCCC GACCA | 275 | CAATGA CATGGA CGCTGG TA | -27 | 510 | 537 |
| 60bp-200bp | 276 | GCACCCT ATCCATT CACCAT | 277 | GCATCG CCAGCG TTATTAT T | 299 | 399 | 100 |
| | 278 | TGCCAGT CAGTGAG CTATGG | 279 | AAAGCC ATTAAG GCGTAG GG | 442 | 547 | 105 |
| | 280 | AATAATA ACGCTGG CGATGC | 281 | TGCCAT AGCTCA CTGACT GG | 380 | 463 | 83 |
| | 282 | GGTAGCA CCAGTCA GGCTGT | 283 | CGGAAT TGAAAA CCTCTG CT | 132 | 236 | 104 |
| 300bp-500bp | 284 | TGAAAGT TCGTTCA GCTTGC | 285 | GCATCG CCAGCG TTATTAT T | 3 | 399 | 396 |
| | 286 | GGTAGCA CCAGTCA GGCTGT | 287 | GCCCAG TATGAT GTCCAG AAA | 132 | 431 | 299 |

Table 27 lists additional primers contemplated by the present invention that may be used to identify the sequence as set forth in SEQ ID NO: 19 (Fragment 19).

**Table 27**

| | SEQ ID NO: | 5' primer | SEQ ID NO: | 3' primer | coordinates | coordinates | pcr product length |
|---|---|---|---|---|---|---|---|
| flanking region | 288 | CATCCTG GAACAGA CTGGGTA | 289 | ATAAAA CCAATC GGCCCA AC | -19 | 194 | 213 |
| 60bp-200bp | 290 | CATGGCA ACTTACG GCATTA | 291 | CGTAAC AGGGAG GAAAGA CG | 50 | 140 | 90 |
| | 292 | ACGCATG GGAGAAG AAAGAG | 293 | CGATAA TGCTGC AAGCAA AC | 69 | 165 | 96 |
| | 294 | ACAGCAA CTGCGTC TTTCCT | 295 | ATAAAA CCAATC GGCCCA AC | 111 | 194 | 83 |

Table 28 lists additional primers contemplated by the present invention that may be used to identify the sequence as set forth in SEQ ID NO: 20 (Fragment 20).

**Table 28**

| | SEQ ID NO: | 5' primer | SEQ ID NO: | 3' primer | coordinates | coordinates | pcr product length |
|---|---|---|---|---|---|---|---|
| flanking region | 296 | CCAATAG GCATCGT CACCTC | 297 | GCCAGA TGTTCTA TCCCCA GT | -41 | 421 | 462 |
| 60bp-200bp | 298 | AATGGTT GCGGTAA ATCGAC | 299 | TGGCAT TTGCATT AGGTTG A | 150 | 248 | 98 |
| | 300 | ATTCTGG GACCAAA TGGTTG | 301 | TGGCAT TTGCATT AGGTTG A | 138 | 248 | 110 |
| | 302 | TACCTGA ACTGCAA CGAGGA | 303 | AATGTC GATTTA CCGCAA CC | 106 | 172 | 66 |
| 300bp-500bp | 304 | CGCTATC GCAGGAG TTTGTT | 305 | CGCGTA GGGAAA CCAGAA TA | 16 | 345 | 329 |
| | 306 | CGCTATC GCAGGAG TTTGTT | 307 | ATCTTTG ACGCGC GTAGG | 16 | 355 | 339 |
| | 308 | CGCTATC GCAGGAG TTTGTT | 309 | ATGTTTC CCGCCC ATCTT | 16 | 368 | 352 |

Table 29 lists additional primers contemplated by the present invention that may be used to identify the sequence as set forth in SEQ ID NO: 21 (Fragment 21).

**Table 29**

| | SEQ ID NO: | 5' primer | SEQ ID NO: | 3' primer | coordinates | coordinates | pcr product length |
|---|---|---|---|---|---|---|---|
| flanking region | 310 | CCATCAGT TCCATGTT ATGGATT | 311 | CTCTGA TGTATC CGGTGT GC | -20 | 1137 | 1157 |
| | 312 | TACAATGA ATGCCCTG TTGG | 313 | ATGGCC AGTGAG CGTAAA AA | 451 | 1100 | 649 |
| | 314 | CCATCAGT TCCATGTT ATGGATT | 315 | GGTGCA TTGATTC CACGTC | -20 | 623 | 643 |
| 60bp-200bp | 316 | CCGTGGA CGAATAG AGCATT | 317 | TGAGAA GTTCCG GGAGAG AA | 178 | 284 | 106 |
| | 318 | AGTTGCTG CTGACGA CCTTC | 319 | TTTTTGG TGCATT GATTCC A | 544 | 628 | 84 |
| | 320 | CTGTGGTT GAGGTTTG AGCA | 321 | ATGGCC AGTGAG CGTAAA AA | 1016 | 1100 | 84 |
| | 322 | CCTCTAGT TGCTGCTG ACGA | 323 | TTTTTGG TGCATT GATTCC A | 539 | 628 | 89 |
| | 324 | CGGTTTCG ATACGCTC TTTT | 325 | ATGGGT TTTGGA TCTGAA CG | 515 | 603 | 88 |
| | 326 | GGTCTCGG AAGATCG AGAAA | 327 | CCGGGC AAAAGT ATCAAA AA | 895 | 1005 | 110 |
| 300bp-500bp | 328 | TGGAATCA ATGCACC AAAAA | 329 | CCGGGC AAAAGT ATCAAA AA | 609 | 1005 | 396 |
| | 330 | TTCTCTCC CGGAACTT CTCA | 331 | AAATAC GGGGAA TTGTGT GG | 265 | 666 | 401 |
| | 332 | GTGGAATC AATGCAC CAAAA | 333 | CCGGGC AAAAGT ATCAAA AA | 608 | 1005 | 397 |

Table 30 lists additional primers contemplated by the present invention that may be used to identify the sequence as set forth in SEQ ID NO: 22 (Fragment 22).

**Table 30**

| | SEQ ID NO: | 5' primer | SEQ ID NO: | 3' primer | coordinates | coordinates | pcr product length |
|---|---|---|---|---|---|---|---|
| flanking region | 334 | CCGTAAC GGTCAAA AATCGT | 335 | TTTTGTT CACCAC GACCTC A | -29 | 591 | 620 |
| | 336 | AAAAATC GTGGCGTT GACAC | 337 | TTTTGTT CACCAC GACCTC A | -18 | 591 | 609 |
| 60bp-200bp | 338 | GTATCGAC TGGTGGCA TCTG | 339 | TGACTG CCTTTCT CCCACT T | 36 | 111 | 75 |
| | 340 | GGCATCTG GAGATAC GCTTT | 341 | TGACTG CCTTTCT CCCACT T | 48 | 111 | 63 |
| | 342 | TGCTTTTT GATGATG GAAACA | 343 | CAAACG GGGAAT GTAGCA AT | 447 | 508 | 61 |
| | 344 | TGCATTGC GGTTTTAA TCTTT | 345 | AGCCAA GCCAAT TTATTTC AA | 241 | 332 | 91 |
| | 346 | GTATCGAC TGGTGGCA TCTG | 347 | CAGCAT GACTGC CTTTCTC C | 36 | 116 | 80 |
| | 348 | AATGCATT GCGGTTTT AATCTT | 349 | TTGAAT AGCCAA GCCAAT TT | 239 | 338 | 99 |
| 300bp-500bp | 350 | CTGCAGC ATTGACGA TTTGT | 351 | CAAACG GGGAAT GTAGCA AT | 114 | 508 | 394 |
| | 352 | TGCAGCAT TGACGATT TGTT | 353 | TATCAC CCAAAC GGGGAA T | 115 | 515 | 400 |
| | 354 | CGGACAT AAATATCT CAAAATG ACA | 355 | CAAACG GGGAAT GTAGCA AT | 192 | 508 | 316 |

Table 31 lists additional primers contemplated by the present invention that may be used to identify the sequence as set forth in SEQ ID NO: 23 (Fragment 23).

**Table 31**

| | SEQ ID NO: | 5' primer, | SEQ ID NO: | 3' primer | coordinates | coordinates | pcr product length |
|---|---|---|---|---|---|---|---|
| flanking region | 356 | GAGCGTCT GCTCAAA CAGGT | 357 | GTAGTG GGTGAG GGGCTG T | -16 | 322 | 338 |
| | 358 | TCAAACA GGTTATCC GTCAGG | 359 | GTAGTG GGTGAG GGGCTG T | -6 | 322 | 328 |
| 60bp-200bp | 360 | TCCGTCAG GAAGAGG AAAAA | 361 | CAGAGC GTCAAG CAACCT TT | 6 | 108 | 102 |
| 300bp-500bp | 362 | TCCGTCAG GAAGAGG AAAAA | 363 | CCTTAC CTATTTC CGCTGG T | 6 | 263 | 257 |

Table 32 lists additional primers contemplated by the present invention that may be used to identify the sequence as set forth in SEQ ID NO: 24 (Fragment 24).

**Table 32**

| | SEQ ID NO: | 5' primer, | SEQ ID NO: | 3' primer | coordinates | coordinates | pcr product length |
|---|---|---|---|---|---|---|---|
| flanking region | 364 | TTGAAAAC CAGAGCC TTGCT | 365 | CGTTGA CCATAA GAAAAC CTCA | -11 | 1241 | 1252 |
| | 366 | AATGAAA AAGGAAA CGCCATA | 367 | CGTTGA CCATAA GAAAAC CTCA | 694 | 1241 | 547 |
| 60bp-200bp | 368 | TTTtCTGCT GCAAGCA CTTC | 369 | GGCCCG TTTATCA GAAAGG T | 205 | 347 | 142 |
| | 370 | AAAAGGG AAGGCCTT ATGATG | 371 | TCCAAA ATGGGA AAGAAG AGG | 528 | 619 | 91 |
| | 372 | TTTCATAG GAAGTGG AGGTGGT | 373 | AAAATG GCCCGT TTATCA GA | 272 | 352 | 80 |
| 300bp-500bp | 374 | TTTtCTGCT GCAAGCA CTTC | 375 | CCAAAA TGGGAA AGAAGA GG | 205 | 618 | 413 |
| | 376 | CCTTGCTT AGACCTGT GTCCA | 377 | GGCCCG TTTATCA GAAAGG T | 2 | 347 | 345 |
| | 378 | ACGTGGGT TGGATCAT TGTT | 379 | AAAATG GCCCGT TTATCA GA | 27 | 352 | 325 |

Table 33 lists additional primers contemplated by the present invention that may be used to identify the sequence as set forth in SEQ ID NO: 25 (Fragment 25).

**Table 33**

| | SEQ ID NO: | 5'primer | SEQ ID NO: | 3' primer | coordinates | coordinates | pcr product length |
|---|---|---|---|---|---|---|---|
| flanking region | 380 | GGGTCTCG ATTTGATG ATTGA | 381 | GGCTTT ACCGTG GTAGTA CTGG | -39 | 827 | 866 |
| | 382 | TCTTGGTA GGGACGT GGTTT | 383 | GGCTTT ACCGTG GTAGTA CTGG | -10 | 827 | 837 |
| 60bp-200bp | 384 | CTTCATTT TGGCCTCT TTGC | 385 | TTGGGT AGCCAC ATCCCTT A | 299 | 395 | 96 |
| | 386 | GATGTGGC TACCCAA GCAAT | 387 | CACCCA AGGACT GAAGGA AG | 381 | 535 | 154 |
| | 388 | GGGCATG GGCATACT TATCA | 389 | ATTTCCC CTCAAT TCCTTCG | 535 | 685 | 150 |
| 300bp-500bp | 390 | TGTGCACC TTAGTCGC TTTG | 391 | ATTTCCC CTCAAT TCCTTCG | 226 | 685 | 459 |
| | 392 | CTTCATTT TGGCCTCT TTGC | 393 | TCTGAG CGATTTT TCTTGA GC | 299 | 767 | 468 |
| | 394 | ATGGCGGT AGCTCATA CCTTT | 395 | ATTTCCC CTCAAT TCCTTCG | 343 | 685 | 342 |

Table 34 lists additional primers contemplated by the present invention that may be used to identify the sequence as set forth in SEQ ID NO: 26 (Fragment 26).

**Table 34**

| | SEQ ID NO: | 5' primer | SEQ ID NO: | 3' primer | coordinates | coordinates | pcr product length |
|---|---|---|---|---|---|---|---|
| flanking region | 396 | CGCCCCAT AGTATATT GGACAT | 397 | ACGATA TCAGCG AAGGTG CT | -21 | 1478 | 1499 |
| | 398 | TCCAGGTC GCCCCATA GTA | 399 | GCTGCA CTGGTA TCCGAT TT | -28 | 384 | 412 |
| | 400 | GGGGCTC CTGATTCA TAACC | 401 | ACGATA TCAGCG AAGGTG CT | 1052 | 1478 | 426 |
| 60bp-200bp | 402 | CAAGCCA AATGCTGA CAAAA | 403 | ATTAAG AACCGA CGCCAG TG | 430 | 535 | 105 |
| | 404 | CGGTTCTG GCTCAGGT AGTT | 405 | TGAATC CCACAG CGTCAT TA | 397 | 496 | 99 |
| | 406 | TTATTTAG AGCCGCG CTGAC | 407 | GCGAAG ATCCTCT GGTAAC G | 902 | 1007 | 105 |
| | 408 | AGCATCCC CCTTGTTA TTGA | 409 | GCACCA GCAGTA CATCGA GA | 732 | 819 | 87 |
| | 410 | ATACCCGC TTTCTCAA GTGC | 411 | GCTACG TGCTGG GGTATC TC | 1316 | 1421 | 105 |
| 300bp-500bp | 412 | CGGGCCA TACATCCA GTAAT | 413 | GTGTTC GGCTTG CAGCTA TC | 864 | 1265 | 401 |
| | 414 | TTCTTTAG CTTCGGGC CATA | 415 | CTATCG GGGGCG TAGAGA A | 853 | 1250 | 397 |
| | 416 | TAATGACG CTGTGGGA TTCA | 417 | GATGTA TGGCCC GAAGCT AA | 477 | 876 | 399 |
| | 418 | GTCAAGC CAAATGCT GACAA | 419 | GCACCA GCAGTA CATCGA GA | 428 | 819 | 391 |

Table 35 lists additional primers contemplated by the present invention that may be used to identify the sequence as set forth in SEQ ID NO: 27 (Fragment 27).

**Table 35**

| | SEQ ID NO: | 5' primer | SEQ ID NO: | 3' primer | coordinates | coordinates | pcr product length |
|---|---|---|---|---|---|---|---|
| flanking region | 420 | CGGGCAA AGACTAC ACACAG | 421 | CAGATC ATAGGT GTAATG ATCGAA | -33 | 570 | 603 |
| 60bp-200bp | 422 | GTATGCAG GAAAGCA CCACA | 423 | AAACCG CTCAAA GGTGAA TG | 67 | 185 | 118 |
| | 424 | ATACGTTT TCACGCCG TTTC | 425 | CGGCAA TACGGG TCAGTA AG | 186 | 302 | 116 |
| 300bp-500bp | 426 | ATCGTTTT GGCTTTGG TGTC | 427 | CGTAAA TATCGG GAGGCG TA | 122 | 510 | 388 |
| | 428 | TGGTATTG TGCGTACG TGGT | 429 | CGTAAA TATCGG GAGGCG TA | 8 | 510 | 502 |

Table 36 lists additional primers contemplated by the present invention that may be used to identify the sequence as set forth in SEQ ID NO: 28 (Fragment 28).

**Table 36**

| | SEQ ID NO: | 5' primer | SEQ ID NO: | 3' primer | coordinates | coordinates | pcr product length |
|---|---|---|---|---|---|---|---|
| flanking region | 430 | CCCTGGTG CAGGACA TAGAT | 431 | AGCTCC CCATGG TTTGCTA T | -37 | 760 | 797 |
| | 432 | GACAACA AACTCCCC TGGTG | 433 | TTGCTAT CGGACA TGGGTT A | -49 | 747 | 796 |
| 60bp-200bp | 434 | CTGGTTCG TCCACTTT CGAT | 435 | GCACGT CGTTTG GAATTA GG | 276 | 373 | 97 |
| | 436 | AGCTCTCC TGCCTGAA CGTA | 437 | AGCCCA GACTGG CTACTG AA | 135 | 242 | 107 |
| | 438 | CGACCCG AGTAAAA GAACGA | 439 | GCCGAG CAATAA CACCAC TT | 528 | 644 | 116 |
| 300bp-500bp | 440 | AGCCAGA TCCAGAA GATTGC | 441 | GCCGAG CAATAA CACCAC TT | 248 | 644 | 396 |
| | 442 | AGCTCTCC TGCCTGAA CGTA | 443 | GGTCGC TAAGGT CATTGC TT | 135 | 532 | 397 |
| | 444 | TCGATACG TAATGCGA AGCA | 445 | TTTTACT CGGGTC GCTAAG G | 39 | 541 | 502 |

Table 37 lists additional primers contemplated by the present invention that may be used to identify the sequence as set forth in SEQ ID NO: 29 (Fragment 29).

**Table 37**

| | SEQ ID NO: | 5' primer | SEQ ID NO: | 3' primer | coordinates | coordinates | pcr product length |
|---|---|---|---|---|---|---|---|
| flanking region | 446 | GGCGTGGT ACATGGAT ATGA | 447 | GGGCGA CGAATG TATTCA AA | -39 | 284 | 323 |
| | 448 | CCAGGCG TGGTACAT GGATA | 449 | GGGCGA CGAATG TATTCA AA | -42 | 284 | 326 |
| 60bp-200bp | 450 | GCGGTGG CTACACTA TGGTT | 451 | ATTGGT ACCCAG TTCGGT GA | 116 | 210 | 94 |
| | 452 | ATGGCTTC ACGGTTAA ATGC | 453 | TGGTAC CCAGTT CGGTGA TT | 98 | 208 | 110 |
| | 454 | GGCGGAT CTGTATAC GCAAT | 455 | ACGCAG AAAAGG CAGCTA AC | 0 | 82 | 82 |
| 300bp-500bp | 456 | TGTATACG CAATCGG CTTTG | 457 | TGGTAC CCAGTT CGGTGA TT | 8 | 208 | 200 |

Table 38 lists additional primers contemplated by the present invention that may be used to identify the sequence as set forth in SEQ ID NO: 30 (Fragment 30).

**Table 38**

| | SEQ ID NO: | 5' primer | SEQ ID NO: | 3' primer | coordinates | coordinates | pcr product length |
|---|---|---|---|---|---|---|---|
| flanking region | 458 | TCATCGGA TCTTTCCC TTGT | 459 | AGCGGG TTTGAA AGGATG TA | -27 | 935 | 962 |
| | 460 | AGTTGCCA ACCTTGAT CCTG | 461 | AAGCGG GTTTGA AAGGAT GT | 494 | 936 | 442 |
| | 462 | TCATCGGA TCTTTCCC TTGT | 463 | GAATCA GGAACG GCTTTTT G | -27 | 421 | 448 |
| 60bp-200bp | 464 | CAGTTGCC AACCTTGA TCCT | 465 | CCTGCT GAAACA TGGCAA TA | 493 | 591 | 98 |
| | 466 | ACCGAAA AAGAGCA AGAGCA | 467 | CAATGC AAAATC CCATCC AT | 681 | 780 | 99 |
| | 468 | ATTCGGAT GGTATCGA CGAA | 469 | AGGATC AAGGTT GGCAAC TG | 418 | 512 | 94 |
| 300bp-500bp | 470 | TGCCGGTA GTGTTATG GACA | 471 | CCCTGA AAAGAC TCCTGCT G | 211 | 604 | 393 |
| | 472 | AAGGTAA ACTCTGCG CTCCA | 473 | AGGATC AAGGTT GGCAAC TG | 135 | 512 | 377 |
| | 474 | TGCCGGTA GTGTTATG GACA | 475 | TCCCTG AAAAGA CTCCTG CT | 211 | 605 | 394 |
| | 476 | GCTGCCG GTAGTGTT ATGGA | 477 | CCCTGA AAAGAC TCCTGCT G | 209 | 604 | 395 |
| | 478 | AAACAAA GCGTCGC AAAAAG | 479 | CAATGC AAAATC CCATCC AT | 389 | 780 | 391 |

Table 39A lists additional primers contemplated by the present invention that may be used to identify the sequence as set forth in SEQ ID NO: 31 (Fragment 31).

**Table 39A**

| | SEQ ID NO: | 5' primer | SEQ ID NO: | 3' primer | coordinates | coordinates | pcr product length |
|---|---|---|---|---|---|---|---|
| flanking region | 480 | TACGGTGA AAGAGTG GCATT | 481 | CTGCAA CAATGG CTTTTTG T | -15 | 830 | 845 |
| | 482 | TGTCTGCA TAATTGGC TTTACC | 483 | CTCGTTC CCTGCA ACAATG | 436 | 838 | 402 |
| | 484 | ACGGTGA AAGAGTG GCATTG | 485 | TGGGCT TTCTGTA GGTTTT GA | -14 | 407 | 421 |
| 60bp-200bp | 486 | GGCTACTT CTCCCCAC CATT | 487 | GGGACG TTACGC AAATTT CT | 77 | 161 | 84 |
| | 488 | TTCTCGTC AGGCATTT TTCC | 489 | TGGGCT TTCTGTA GGTTTT GA | 318 | 407 | 89 |
| | 490 | TGATCAAA CCAGCCAT CAAC | 491 | CTGCAA CAATGG CTTTTG T | 723 | 830 | 107 |
| | 492 | TCGTCAGG CATTTTTC CTTT | 493 | TGGGCT TTCTGTA GGTTTT GA | 321 | 407 | 86 |
| 300bp-500bp | 494 | GGCTACTT CTCCCCAC CATT | 495 | GGGCAA CAGCCA TAGGTA AA | 77 | 471 | 394 |
| | 496 | TCTCGTCA GGCATTTT TCCT | 497 | TCAGTT GATGGC TGGTTT GA | 319 | 745 | 426 |

Table 39B lists additional primers contemplated by the present invention that may be used to identify the sequence as set forth in SEQ ID NO: 32 (Fragment 32).

**Table 39B**

| | SEQ ID NO: | 5' primer | SEQ ID NO: | 3' primer | coordinates | coordinates | pcr product length |
|---|---|---|---|---|---|---|---|
| flanking region | 498 | TGAAAAA TAACTTAA ATAAGGG ATGG | 499 | GCGAAT TATTTA GTACAA AAAGCG TA | 132 | 231 | 99 |
| 60bp-200bp | 500 | TTTCATTA TGCTATTT AAGATGT GA | 501 | AATATA AATATA TCCCAT CCCTTAT TT | 110 | 171 | 61 |
| | 502 | CAGAGTA AAAATGT AACGCTG A | 503 | CCCATC CCTTATT TAAGTT ATTTTTC | 4 | 158 | 154 |

Table 40 lists additional primers contemplated by the present invention that may be used to identify the sequence as set forth in SEQ ID NO: 33 (Fragment 33).

**Table 40**

| | SEQ ID NO: | 5' primer | SEQ ID NO: | 3' primer | coordinates | coordinates | per product length |
|---|---|---|---|---|---|---|---|
| flanking region | 504 | GCAACAT CAAAATG GCTGAG | 505 | TGATTTT CGTCAG ACTGCA AG | -30 | 2460 | 2490 |
| | 506 | GCAACAT CAAAATG GCTGAG | 507 | AACCTT TCAACC GGAGTT CA | -30 | 252 | 282 |
| | 508 | TGGCCTTG TACCAATT CCTT | 509 | TGATTTT CGTCAG ACTGCA AG | 1536 | 2460 | 924 |
| 60bp-200bp | 510 | TCCGATGA AACATCA CCATC | 511 | CAATTG AAAACA TGGCCA GA | 66 | 154 | 88 |
| | 512 | GATGTCCG ATGAAAC ATCACC | 513 | CAATTG AAAACA TGGCCA GA | 62 | 154 | 92 |
| | 514 | TGGCCTTG TACCAATT CCTT | 515 | TTTTTCA GTAAGC TCAGAC AAATCA | 1536 | 1671 | 135 |
| 300bp-500bp | 516 | TGGCCTTG TACCAATT CCTT | 517 | AAACAG ATGTCC CGAAAA TCA | 1536 | 1940 | 404 |

Table 41 lists additional primers contemplated by the present invention that may be used to identify the sequence as set forth in SEQ ID NO: 34 (Fragment 34).

**Table 41**

| | SEQ ID NO: | 5' primer | SEQ ID NO: | 3' primer | coordinates | coordinates | pcr product length |
|---|---|---|---|---|---|---|---|
| flanking region | 518 | CCTGCTGA TAGTGCCA TGAA | 519 | TAATCA GGAACA GCCGGA AG | -49 | 1410 | 1459 |
| | 520 | CCTGCTGA TAGTGCCA TGAA | 521 | CGGTAA GACACC AGCCTT GA | -49 | 473 | 522 |
| 60bp-200bp | 522 | TCCTTGCC TCATGTGT TCTG | 523 | GAGATC AAGCGT TTCCCA AG | 1335 | 1436 | 101 |
| | 524 | GGAATTGC GAGTGAG GTCTT | 525 | AGCGAA CGAACA GCTCAG AT | 496 | 592 | 96 |
| | 526 | AGGTCTTA CCATTGGC TGGA | 527 | TGTCGA GAATAA GCGAAC GA | 509 | 603 | 94 |
| 300bp-500bp | 528 | CACTTTGC ATGAAAG GGCTTA | 529 | AGCGAA CGAACA GCTCAG AT | 190 | 592 | 402 |
| | 530 | AGGATGCT GGATCAA AATGC | 531 | TTGATA GCTTAG CGCCCA AT | 9 | 366 | 357 |
| | 532 | TCTCACCT TCACTTTG | 533 | AGCGAA CGAACA | 181 | 592 | 411 |

Table 42 lists additional primers contemplated by the present invention that may be used to identify the sequence as set forth in SEQ ID NO: 35 (Fragment 35).

**Table 42**

| | SEQ ID NO: | 5' primer | SEQ ID NO: | 3' primer | coordinates | coordinates | pcr product length |
|---|---|---|---|---|---|---|---|
| flanking region | 534 | TCACATGT GTTGCACC TTTACA | 535 | GCAACC TTTTGCT TTAATGt TTTT | -14 | 1498 | 1512 |
| | 536 | TGCACAA CTGGCCTA TGTTT | 537 | GCAACC TTTTGCT TTAATGt TTTT | 1086 | 1498 | 412 |
| | 538 | GGAGAGT GCGGGGT ATTTTA | 539 | AGTTCA TTCGCA ACCGTT TT | -49 | 351 | 400 |
| 60bp-200bp | 540 | TGCTATTG GTGAATG GCAAA | 541 | CCCGCT TCATCT GATGGT AT | 1245 | 1346 | 101 |
| | 542 | GTTGGCAG GTTGCTCA ATTC | 543 | CTCCAC CATAAT TCCGCTT G | 866 | 981 | 115 |
| | 544 | TGAGCAA GAGCATA GGTTTGA | 545 | CGCAAC CGTTTTG TTTTCtT | 239 | 343 | 104 |
| 300bp-500bp | 546 | GTTGGCAG GTTGCTCA ATTC | 547 | TGCCAT TCACCA ATAGCA AA | 866 | 1262 | 396 |
| | 548 | GCGGAATT ATGGTGG AGAAA | 549 | ATAGAC GTCGCC AGATTT CC | 965 | 1364 | 399 |
| | 550 | TACAAAG GCTGTTGG CAGGT | 551 | TGCCAT TCACCA ATAGCA AA | 856 | 1262 | 406 |
| | 552 | GGATGAT GAATCAAT GCCAAA | 553 | CACTTC CGAATT GAGCAA CC | 505 | 892 | 387 |

Table 43 lists additional primers contemplated by the present invention that may be used to identify the sequence as set forth in SEQ ID NO: 36 (Fragment 36).

**Table 43**

| | SEQ ID NO: | 5' primer | SEQ ID NO: | 3' primer | coordinates | coordinates | pcr product length |
|---|---|---|---|---|---|---|---|
| flanking region | 554 | CGAGCAC TGTTATAG TAATTTCA GAAG | 555 | GAGTGG TCGGGG TATTGT GT | -47 | 758 | 805 |
| | 556 | TGAAGTTT GTGCCAC GGTAA | 557 | GATGAG TGGTCG GGGTAT TG | 371 | 761 | 390 |
| | 558 | CGAGCAC TGTTATAG TAATTTCA GAAG | 559 | CGACCT GAAAAG CCCAAA TA | -47 | 345 | 392 |
| 60bp-200bp | 560 | ACAGTCG AGCCAGC TTCAAT | 561 | CGACCT GAAAAG CCCAAA TA | 235 | 345 | 110 |
| | 562 | ACAGTCG AGCCAGC TTCAAT | 563 | CCCAAA TACTTC GGGAGC TA | 235 | 333 | 98 |
| | 564 | CCACCATC ACCCTCAA GTTC | 565 | TGACAT GGTTGA CAACAG CA | 415 | 523 | 108 |
| | 566 | CCCGAAG TATTTGGG CTTTT | 567 | GAGGGT GATGGT GGTGAT GT | 319 | 428 | 109 |
| 300bp-500bp | 568 | TTGAATCA AAAATGC ACGACA | 569 | TGAACT TGAGGG TGATGG TG | 33 | 435 | 402 |
| | 570 | CCCGAAG TATTTGGG CTTTT | 571 | CAAATA GTCCCC GCCCTTT A | 319 | 715 | 396 |
| | 572 | CCGAAAA GAGGAGT TGAACG | 573 | TGACAT GGTTGA CAACAG CA | 104 | 523 | 419 |

Another method based on single nucleotide primer (SNuPe) extension may also be used to detect the M17 sequences of the present invention.

A number of primer extension-based characterizations of bacteria have been reported, including the phylotyping of *Listeria monocytogenes* [Rudi et al, 2003, FEMS Microbiol. Lett. 220, 9-14; Ducey et al, 2007, Microbiol. 73, 133-147] and *Escherichia coli* strains [Hommais et al, 2005, Appl. Environ. Microbiol. 71, 4784-4792] and the rapid identification of *Brucella* isolates [Scott et al, 2007, Appl. Environ. Microbiol. 73, 7331-7337]. These studies demonstrated the good discrimination potential and high taxonomical resolution of SNuPe analyses with primer extension.

The principle of SNuPE is described herein below and in Nikolausz et al [Biochemical Society Transactions (2009) Volume 37, part 2], incorporated herein by reference. The method benefits from the high fidelity of DNA polymerases while incorporating nucleotides or nucleotide analogues, resulting in a highly specific distinction of sequence variants. When a specific primer hybridizes upstream from the target nucleotide position, a DNA polymerase incorporates a labelled nucleoside triphosphate, which terminates the reaction and results in a labelled extended primer.

As used herein the term "about" refers to ± 10 %.

The terms "comprises", "comprising", "includes", "including", "having" and their conjugates mean "including but not limited to".

The term "consisting of means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following examples.

### EXAMPLES

Reference is now made to the following examples, which together with the above descriptions illustrate some embodiments of the invention in a non limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Culture of Animal Cells - A Manual of Basic Technique" by Freshney, Wiley-Liss, N. Y. (1994), Third Edition; "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996); all of which are incorporated by reference as if fully set forth herein. Other general references are provided throughout this document. The procedures therein are believed to be well known in the art and are provided for the convenience of the reader. All the information contained therein is incorporated herein by reference.

### EXAMPLE 1

### Sequencing of M17

### MATERIALSAND METHODS

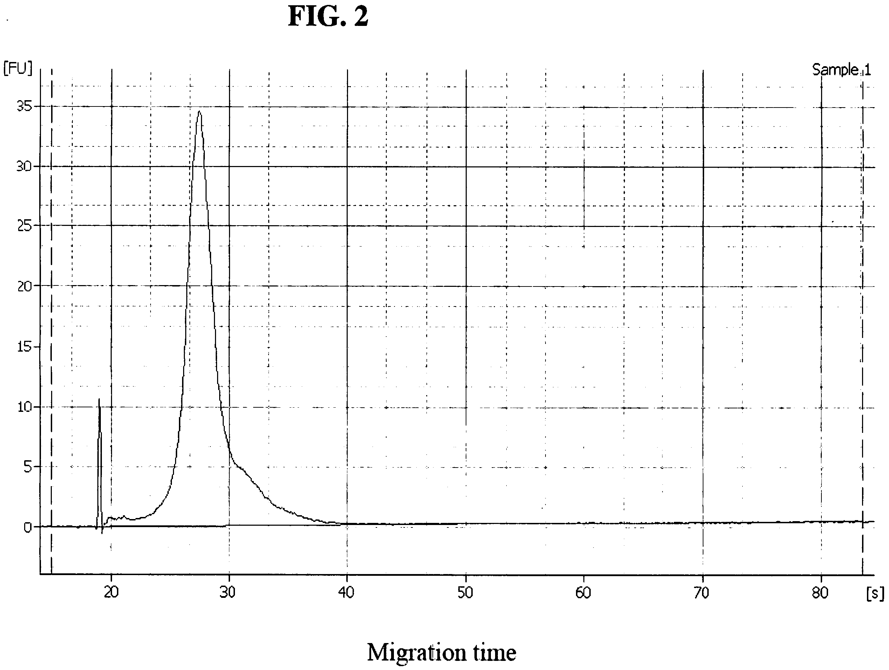

***454 Genomic Sequencing of M17p:*** M17p gDNA was fragmented and an 8 kb paired-end library suitable for 454 platform sequencing was prepared (following manufacturer's instructions. QC analysis of the generated paired-end library using an Agilent 2100 Bioanalyzer (Figure 2) indicated that the library was of acceptable quality, containing the expected fragment size and yield, for continued sample processing.

The paired-end library was used in emulsion PCR (GS Titanium LV emPCR Kit (Lib-L), Roche) following manufacturers instructions. The generated DNA beads passed QC analysis with an enrichment of 20.3. These DNA beads were used in ½ Titanium plate 454 sequencing (following manufacturers instructions), using the GS Titanium Sequencing Kit XLR70.

The raw 454 sequence data was then assembled with the Roche Newbler (2.0.00.22) software.

***Unique fragment identification:*** Sequence data available from public sources was compared to the M17p 454 sequence data in the Cross-Match software package (using the default screening settings) and 36 fragments were identified that are unique to the M17p strain.

### RESULTS

The sequencing results summary is provided in Table 44, herein below.

**Table 44**

| ***Sample Name*** | ***Read Number (No. of bases)*** | ***Average Read Length (No. of bases)*** | ***Total Bases*** |
|---|---|---|---|
| M17p | 529,038 | 378 | 200,066,401 |

The assembly results are summarized in Table 45, herein below.

**Table 45**

| ***Sample Name*** | ***Number of Scaffolds**** | ***Number of Bases*** | ***Largest Scaffold Size*** | ***Number of Large Contigs*** | ***Largest Contig Size*** |
|---|---|---|---|---|---|
| M17p | 5 | 5,010,882 | 5,005,431 | 78 | 440,798 |

| | | | | | |
|---|---|---|---|---|---|
| *A Scaffold is a collection of sequence contigs that have been oriented and spaced with respect to one another based on paired-end sequencing data. Scaffold sequences are represented in FASTA format and contain stretches of N's to represent the gaps between contigs. | | | | | |

The sequences were then analyzed using the Blastn algorithm (the NCBI website) to search the nr Database collection: All GenBank+EMBL+DDBJ+PDB sequences under default settings.

The sequence of the 36 fragments identified as being unique according to the Cross-Match software package and Blast analysis may be identified as set forth in Table 46, herein below.

**Table 46**

| frag. | SEQ ID NO: | contig | start | end | length |
|---|---|---|---|---|---|
| 1 | 1 | contig00078 | 62663 | 63843 | 1181 |
| 2 | 2 | contig00078 | 66656 | 66825 | 170 |
| 3 | 3 | contig00084 | 21765 | 23445 | 1681 |
| 4 | 4 | contig00095 | 18923 | 19111 | 189 |
| 5 | 5 | contig00097 | 11127 | 11542 | 416 |
| 6 | 6 | contig00100 | 28867 | 29421 | 555 |
| 7 | 7 | contig00100 | 29604 | 30960 | 1357 |
| 8 | 8 | contig00100 | 31120 | 31914 | 795 |
| 9 | 9 | contig00100 | 32458 | 32794 | 337 |
| 10 | 10 | contig00100 | 63457 | 63982 | 526 |
| 11 | 11 | contig00100 | 64229 | 64692 | 464 |
| 12 | 12 | contig00101 | 13634 | 14112 | 479 |
| 13 | 13 | contig00101 | 14323 | 14718 | 396 |
| 14 | 14 | contig00112 | 412197 | 412869 | 673 |
| 15 | 15 | contig00112 | 419061 | 419866 | 806 |
| 16 | 16 | contig00112 | 428236 | 428583 | 348 |
| 17 | 17 | contig00112 | 428632 | 428917 | 286 |
| 18 | 18 | contig00112 | 429912 | 430417 | 506 |
| 19 | 19 | contig00112 | 430856 | 431018 | 163 |
| 20 | 20 | contig00112 | 431234 | 431635 | 402 |
| 21 | 21 | contig00112 | 431796 | 432887 | 1092 |
| 22 | 22 | contig00112 | 434683 | 435243 | 561 |
| 23 | 23 | contig00113 | 240 | 518 | 279 |
| 24 | 24 | contig00113 | 15607 | 16798 | 1192 |
| 25 | 25 | contig00115 | 32035 | 32811 | 777 |
| 26 | 26 | contig00115 | 35651 | 37087 | 1437 |
| 27 | 27 | contig00115 | 40119 | 40664 | 546 |
| 28 | 28 | contig00115 | 40865 | 41583 | 719 |
| 29 | 29 | contig00115 | 61928 | 62161 | 234 |
| 30 | 30 | contig00115 | 62302 | 63238 | 937 |
| 31 | 31 | contig00116 | 630 | 1517 | 888 |
| 32 | 32 | contig00117 | 317892 | 318076 | 185 |
| 33 | 33 | contig00122 | 68537 | 70960 | 2424 |
| 34 | 34 | contig00122 | 72789 | 74195 | 1407 |
| 35 | 35 | contig00122 | 76097 | 77545 | 1449 |
| 36 | 36 | contig00122 | 80315 | 81044 | 730 |

A summary of the ten longest unique identified fragments is shown in Table 47.

**Table 47**

| ***Fragment*** | ***Fragment length*** |
|---|---|
| frag-33.fasta | 2,424 |
| frag-3.fasta | 1,681 |
| frag-35.fasta | 1,449 |
| frag-26.fasta | 1,437 |
| frag-34.fasta | 1,407 |
| frag-7.fasta | 1,357 |
| frag-24.fasta* | 1,192 |
| frag-1.fasta | 1,181 |
| frag-21.fasta | 1,092 |
| frag-30.fasta | 937 |

### EXAMPLE 2

### PCR Based Unique Fragments Confirmation Assay MATERIALS AND METHODS

***Samples:*** A total of 74 samples were processed during this project:
• 72 *E.coli* samples from the ECOR culture collection.
*• E.coli* M17p (M17 parent) - ATCC Deposit No. ATCC Deposit No. 202226 (DSM 12799).
*• E.coli* M17SNAR (nalidixic acid-resistant strain) - ATCC Deposit No. 7295.

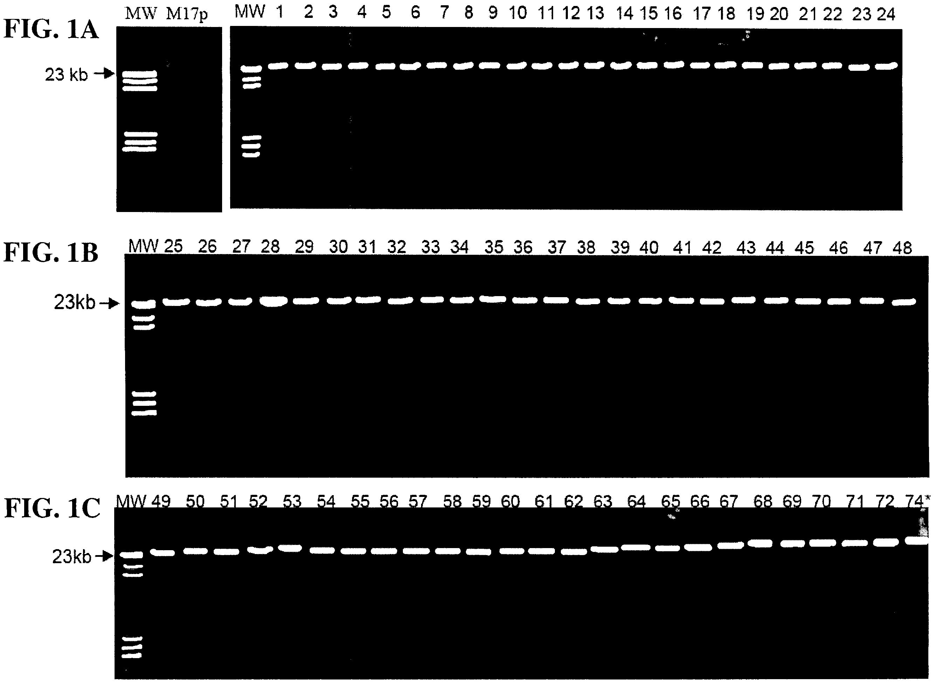

***DNA Purification:*** SeqWright extracted the *E*. *coli* gDNA from M17p, M17SNAR and the 72 ECOR collection *E*. *coli* culture samples with the Promega Wizard™ Genomic DNA Purification Kit (following manufacturer's instructions). A quality control (QC) inspection and rough quantitation of the extracted gDNA samples was performed by agarose gel electrophoresis and UV-induced ethidium bromide fluorescence (Figure 1). Sample quality was compared visually on the gel against a λ DNA-Hind III Digest and ΦX-174-RF DNA, Hae III digest molecular weight (MW) size marker. All 74 *E*. *coli* gDNA samples were of acceptable quality for continued processing. Sample quality was considered to be acceptable if the extracted gDNA supplied a single visible band while lacking any significant degradation products (degraded DNA seen as smear of small fragments).

***PCR Based Unique Fragments Confirmation Assay:*** The 10 unique fragments highlighted in Table 7 were selected for the development of a PCR based assay with an amplicon size range of 400-550 bp. Primers were designed for the assay using Primer3 software from MIT. Fragments 24 and 35 from Table 7 were not further processed because higher quality amplicons were obtained from the other eight unique fragments. Additionally, the larger size of the unique regions of Fragments 33 and 34 allowed for two non-overlapping amplicons to be designed for each fragment. For the PCR assay, a total of 10 Primer3 designed primer pairs were selected (Table 48, herein below). Detailed information on the selected primers can be found in Table 49, herein below.

**Table 48**

| ***Unique Fragment*** | ***Primer Name*** | ***Sequence*** | ***SEQ ID NO:*** | ***Amplicon*** |
|---|---|---|---|---|
| frag30 | CP1 | AAGGTAAACT CTGCGCTCCA | 52 | 470 bp |
| | CP2 | CCCTGAAAAG ACTCCTGCTG | 53 | |
| frag7 | CP3 | TGGTGCTAGAC TCTGGGCTT | 54 | 516 bp |
| | CP4 | TGACGGAAAT ATCCACAGCA | 55 | |
| frag34 | CP5 | CGCTGTGGAA AGTGACAGAA | 56 | 536 bp |
| | CP6 | AATGAATGAG CAAACCGAGG | 57 | |
| frag3 | CP7 | GCGATAACTG GGCAAATGAT | 58 | 489 bp |
| | CP8 | ATTGCATGAG TCCAACCACA | 59 | |
| frag26 | CP9 | AAATCGGATA CCAGTGCAGC | 60 | 455 bp |
| | CP10 | GCACCAGCAG TACATCGAGA | 61 | |
| frag33 | CP 11 | TGCGAATCGAT GATCTCAAG | 37 | 516 bp |
| | CP12 | TTGGTACAAG GCCATGTTGA | 38 | |
| frag33 | CP13 | GCTGTTTCATG AACTCCGGT | 39 | 545 bp |
| | CP14 | TGGGGACGAA ATATCACCAT | 40 | |
| frag1 | CP15 | TCCGGGTTGAT AACCATCAT | 41 | 497 bp |
| | CP16 | ACCACTCTGG TCCTTCATGC | 42 | |
| frag 21 | CP17 | CCGTGGACGA ATAGAGCATT | 43 | 451 bp |
| | CP18 | TTTTTGGTGCA TTGATTCCA | 44 | |
| frag34 | CP19 | ATCTGAGCTG TTCGTTCGCT | 45 | 451 bp |
| | CP20 | TACCGGGAAA AATGGTCAAA | 46 | |

**Table 49**

| ***Fragment of origin*** | ***Pair score (lower is better)*** | ***Left primer location, length*** | ***Right primer location, length*** | ***Left primer Tm*** | ***Right primer Tm*** |
|---|---|---|---|---|---|
| frag-30.fasta | 0.033 | 134,20 | 603,20 | 60.015 | 59.982 |
| frag-7.fasta | 0.0851 | 426,20 | 941,20 | 60.012 | 60.073 |
| frag-34.fasta | 0.0983 | 36,20 | 571,20 | 60.025 | 60.074 |
| frag-3.fasta | 0.1037 | 816,20 | 1304,20 | 59.929 | 59.967 |
| frag-26.fasta | 0.1191 | 364,20 | 818,20 | 60.103 | 60.016 |
| frag-33.fasta | 0.1282 | 1033,20 | 1548,20 | 59.907 | 59.964 |
| frag-33.fasta | 0.1324 | 223,20 | 767,20 | 60.119 | 60.014 |
| frag-1.fasta | 0.1334 | 206,20 | 702,20 | 60.014 | 60.12 |
| frag-21.fasta | 0.1886 | 177,20 | 627,20 | 60.096 | 59.907 |
| frag-34.fasta | 0.3238 | 572,20 | 1022,20 | 60.164 | 60.16 |

The PCR reactions in 25 ul contained 12.5 ul of AmpliTaq Gold 2x Master Mix, 0.2-1.0 uM of primers, and 50 ng of template DNA (with H₂O as negative control), and were performed for 30 cycles consisting of the following steps: denaturation at 95 °C for 30s; annealing at 50 °C for 30s; and extension at 72 °C for 60s. The generated PCR products were checked on agarose gel.

### RESULTS

Three primer pairs (CP11 and CP12, CP13 and CP14, and CP19 and CP20) generated PCR products (single band on the gel) for M17p and M17SNAR but not for the 72 ECOR collection *E*. *coli* culture samples as shown in Figures 3-5, with summary for all 10 primer pairs provided in Table 50.

**Table 50**

| **Unique Fragment** | **30** | **7** | **34** | **3** | **26** | **33** | **33** | **1** | **21** | **34** |
|---|---|---|---|---|---|---|---|---|---|---|
| **Primer Pair** | CP1 + CP2 | CP3 + CP4 | CP5 + CP6 | CP7 + CP8 | CP9 + CP10 | CP11 + CP12 | CP13 + CP14 | CP15 + CP16 | CP17 + CP18 | CP19 + CP20 |
| H₂O (neg. control) | - | - | - | - | - | - | - | - | - | - |
| M17p | + | + | + | + | + | + | + | + | + | + |
| M17 SNAR | + | + | + | + | + | + | + | + | + | + |
| ECOR-1 | - | - | - | - | - | - | - | + | - | - |
| ECOR-2 | + | - | - | - | - | - | - | + | - | - |
| ECOR-3 | - | - | - | - | - | - | - | + | - | - |
| ECOR-4 | + | - | - | - | + | - | - | + | - | - |
| ECOR-5 | - | - | - | - | + | - | - | + | - | - |
| ECOR-6 | + | - | - | - | + | - | - | + | - | - |
| ECOR-7 | - | - | - | - | + | - | - | + | - | - |
| ECOR-8 | - | + | + | - | - | - | - | + | - | - |
| ECOR-9 | - | - | - | - | + | - | - | + | - | - |
| ECOR-10 | + | + | - | - | + | - | - | + | - | - |
| ECOR-11 | + | - | - | - | + | - | - | + | - | - |
| ECOR-12 | + | - | - | - | + | - | - | + | - | - |
| ECOR-13 | + | - | - | - | + | - | - | + | - | - |
| ECOR-14 | + | - | - | - | + | - | - | + | - | - |
| ECOR-15 | - | - | - | - | + | - | - | + | - | - |
| ECOR-16 | - | - | - | - | + | - | - | + | - | - |
| ECOR-17 | - | + | - | - | + | - | - | + | - | - |
| ECOR-18 | + | - | - | - | + | - | - | + | - | - |
| ECOR-19 | + | - | - | - | + | - | - | + | - | - |
| ECOR-20 | + | - | - | - | + | - | - | + | - | - |
| ECOR-21 | - | - | - | - | + | - | - | + | - | - |
| ECOR-22 | - | - | - | - | + | - | - | + | - | - |
| ECOR-23 | - | - | - | - | + | - | - | + | - | - |
| | | | | | | | | | | |
| ECOR-24 | - | - | + | - | + | - | - | + | - | - |
| ECOR-25 | - | - | - | - | - | - | - | + | - | - |
| ECOR-26 | - | - | - | - | - | - | - | + | - | - |
| ECOR-27 | - | - | - | - | + | - | - | + | - | - |
| ECOR-28 | - | - | - | - | - | - | - | + | - | - |
| ECOR-29 | - | - | + | - | - | - | - | - | - | - |
| ECOR-30 | - | - | - | - | - | - | - | - | - | - |
| ECOR-31 | - | - | + | - | + | - | - | - | - | - |
| ECOR-32 | - | - | - | - | - | - | - | - | - | - |
| ECOR-33 | + | - | - | - | + | - | - | - | - | - |
| ECOR-34 | + | - | - | - | + | - | - | - | - | - |
| ECOR-35 | - | - | - | - | + | - | - | + | - | - |
| ECOR-36 | - | - | - | - | + | - | - | - | - | - |
| ECOR-37 | + | - | - | - | + | - | - | - | - | - |
| ECOR-38 | - | + | - | - | + | - | - | - | - | - |
| ECOR-39 | - | + | - | - | + | - | - | - | - | - |
| ECOR-40 | + | + | - | - | + | - | - | - | - | - |
| ECOR-41 | + | + | - | - | - | - | - | - | - | - |
| ECOR2 | + | - | - | - | + | - | - | - | - | - |
| ECOR-43 | + | + | + | - | + | - | - | - | - | - |
| ECOR-44 | - | - | + | - | - | - | - | + | - | - |
| ECOR-45 | - | - | - | - | - | - | - | + | - | - |
| ECOR-46 | - | - | - | - | + | - | - | + | - | - |
| ECOR-47 | - | + | - | - | + | - | - | + | - | - |
| ECOR-48 | - | - | - | - | + | - | - | - | - | - |
| ECOR-49 | - | + | - | - | + | - | - | + | - | - |
| ECOR-50 | - | + | - | - | + | - | - | + | - | - |
| ECOR-51 | - | + | + | - | - | - | - | - | - | - |
| ECOR-52 | - | + | + | - | - | - | - | - | - | - |
| ECOR-53 | - | + | - | - | - | - | - | - | + | - |
| ECOR-54 | - | + | + | - | - | - | - | - | - | - |
| ECOR-55 | - | + | + | - | - | - | - | - | - | - |
| ECOR-56 | - | + | + | - | - | - | - | - | - | - |
| ECOR-57 | - | + | + | - | + | - | - | - | - | - |
| ECOR-58 | - | - | - | - | + | - | - | - | - | - |
| ECOR-59 | - | + | + | - | + | - | - | - | - | - |
| ECOR-60 | - | + | + | - | + | - | - | - | - | - |
| ECOR-61 | - | + | + | - | + | - | - | - | - | - |
| ECOR-62 | - | + | + | - | - | - | - | - | - | - |
| ECOR-63 | - | + | + | - | - | - | - | - | - | - |
| ECOR-64 | - | + | + | - | - | - | - | - | - | - |
| ECOR-65 | - | - | + | + | - | - | - | - | + | - |
| | | | | | | | | | | |
| ECOR-66 | - | + | + | - | - | - | - | - | - | - |
| ECOR-67 | - | - | + | - | - | - | - | - | - | - |
| ECOR-68 | - | - | + | - | - | - | - | - | - | - |
| ECOR-69 | - | - | + | - | - | - | - | - | - | - |
| ECOR-70 | - | - | - | - | - | - | - | + | - | - |
| ECOR-71 | - | - | - | - | + | - | - | + | - | - |
| ECOR-72 | - | - | - | - | - | - | - | + | - | - |

### EXAMPLE 3

### Detection of E. coli M17p Strain Spiked in Biological Stool Samples

The following experiments were performed to test if the three unique M17 detection PCR assays identified as shown in Example 2 would work with DNA extracted from M17p cell spiked biological stool samples.

### MATERIALS AND METHODS

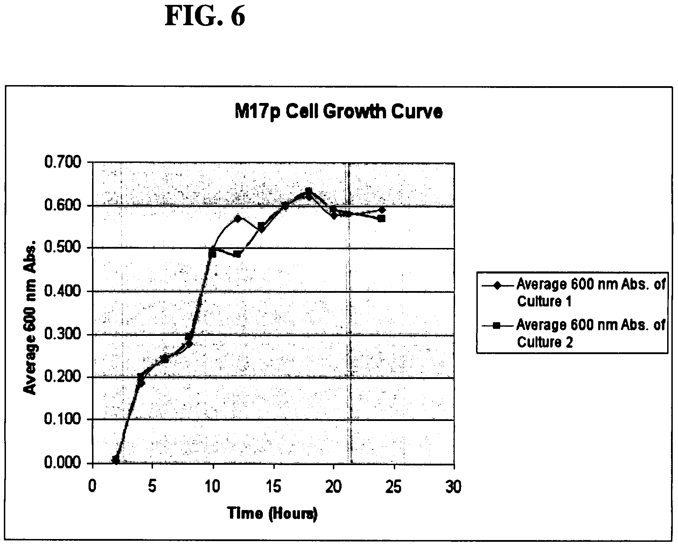

The *E. coli* M17p Strain Growth Curve (Figure 6) was determined from the average 600 nm absorbance readings measured by Nanodrop ND-1000 Spectrophotometer over a time course of 2-24 hours as described below. A single colony of the *E*. *coli* M17p Strain was used to inoculate 5 mL of LB growth media in duplicate (Culture 1 and Culture 2) and the cell cultures were grown at 37 °C and 250 rpm. A sample of 100 µL cell culture was obtained from each of Culture 1 and Culture 2 at time points 2, 4, 6, 8, 10, 12, 14, 16, 18, 20, and 24 hours. Each sample was used to measure the 600 nm absorbance readings three times on a Nonodrop ND-1000 Spectrophotometer and the average reading for each sample at each time point was used to create the growth curve.

The growth curve (see Figure 6) indicated that the 600 nm absorbance reading 0.5 by Nanodrop ND-1000 Spectrophotometer was approaching to the end of the logarithmic growth phase of the M17p cell cultures. (It should be appreciated that the OD600 reading measured by Nanodrop Spectrophotometer is approximately 10 fold less than other conventional spectrophotometers due to the use of shorter path length (1 mm vs. 10 mm) by Nanodrop Spectrophotometer. Therefore, e.g., the cell density from an OD600 absorbance reading 0.2 by Nanodrop is approximately equivalent to the cell density from an OD600 absorbance reading 2.0 by other conventional spectrophotometers.

***DNA Extraction from M17p Cell Spiked Stool Samples:*** Serial cell dilutions were made by picking a single colony from an agar plate and mixing it well with 5 mL LB broth (reference as undiluted or 100). From this initial dilution, subseqent 10-1, 10-2, and 10-3 dilutions were made. One hundred microliters of each dilution was used to inoculate a 5 mL-LB culture. The cultures were grown at 37 °C and 250 rpm for 14 hours. The 600 nm Absorbance of the four 14 hour old cell cultures were measured three times for each by Nanodrop ND-1000 Spetrophotometer and the average 600 nm absorbance for each culture is listed in Table 51. The M17p cell culture from 10-1 inoculation listed in Table 51 was used for stool spiking experiment.

**Table 51**

| ***Cell culture*** | ***Average 600 nm Abs.*** |
|---|---|
| M17p Cell Culture from 10⁰ inoculation | 0.24 |
| M17p Cell Culture from 10⁻¹ inoculation | 0.21 |
| M17p Cell Culture from 10⁻² inoculation | 0.19 |
| M17p Cell Culture from 10⁻³ inoculation | 0.15 |

The M17p Cell Culture from 10⁻¹ inoculation with an average 600 nm absorbance reading 0.21 was selected for spiking experiment since it was well within the logarithmic growth phase. A serial dilution was made from this culture: undiluted, 10⁻¹, 10⁻², 10⁻³, 10⁻⁴, 10⁻¹, 10⁻⁶, 10⁻⁷, 10⁻⁸, 10⁻⁹, 10⁻¹⁰, 10⁻¹¹, 10⁻¹², and 10⁻¹³.

Aliquots of 180 mg biological stool samples were made in 2-mL tubes and spiked with 50 µL of cell dilutions listed above respectively. The spiked stool samples were mixed thoroughly.

DNAs were extracted from the spiked stool samples using QIAamp Stool DNA mini kit, including a no-spike stool sample control, a spike-buffer control (180 µL PBS buffer + 50 µL of 10⁻³ cell dilution), and 10¹⁰ spike control (500 µL undiluted culture spun down and resuspended in 50 µL for spiking). 50 µL of the cell dilutions from the 10⁻⁶, 10⁻⁷, 10⁻⁸, and 10⁻⁹ serial dilutions (the same dilutions that were used to spike the stool samples) were plated on LB Agar plates in duplicate and grown overnight at 37 °C to determine the colony formation units per mL (CFU/mL). The colony counts from each dilution are listed in Table 52.

**Table 52**

| ***Dilutions*** | ***10⁻⁶*** | ***10⁻⁷*** | ***10⁻⁸*** | ***10⁻⁹*** |
|---|---|---|---|---|
| # of Colonies from Plate A | 65 | 11 | 0 | 0 |
| # of Colonies from Plate B | 56 | 6 | 0 | 0 |

Average from the 10⁻⁶culture is ∼60 CFU/50 µL, which is converted to 1.2 X 103 CFU/mL. The undiluted culture that was used to spike the stool samples is estimated to have a 1.2X109CFU/mL. The CFU/mL was estimated to be 1.2X108 CFU/mL for the 10⁻¹ dilution, 1.2X107 CFU/mL for the 10⁻² dilution, 1.2X106 CFU/mL for the 10⁻³ dilution, 1.2X105 CFU/mL for the 10⁻⁴ dilution, which converted to 3.3X108 CFU/gram spiked stool sample for the undiluted culture (100), 3.3X107 CFU/gram spiked stool sample for the 10⁻¹ dilution, 3.3X106 CFU/gram spiked stool sample for the 10⁻² dilution, 3.3X105 CFU/gram spiked stool sample for the 10⁻³ dilution, and 3.3X104 CFU/gram spiked stool sample for the 10⁻⁴ dilution.

Two microliters of each of the DNAs extracted from the spiked stool samples, the no-spike stool sample and the spkied PBS buffer sample were analyzed on an agarose gel (see Figure 7) and these DNAs were used for PCR amplification for M17p detection.

The total DNA extracted from the spiked stool samples and controls were used as templates in PCR reactions using the three M17 strain detection PCR assays developed in Example 2.

The primer sets for each of the three assays are CP11 and CP12, CP13 and CP14, and CP19 and CP20, details of which are provided in Tables 8 and 9, herein above.

All PCR reactions were set up as shown below with a total reaction volume of 20 µL:

| | |
|---|---|
| 1 µL DNA was assembled with 2X Amplitaq Gold PCR Master Mix | 10 µL |
| 10 X BSA | 2 µL |
| Primer 1 at 20 µM | 1 µL |
| Primer 2 at 20 µM | 1 µL |
| H₂O | 5 µL |

PCR reactions include a no-template negative control (H2O) and positive control (1 µL of undiluted cell culture (used to spike the stool samples) mixed with 50 µL H2O and incubate at 98 °C for 6 minutes. 1 µL of the cracked cell sample was used as the positive control in PCR).

### Cycling conditions:

95 °C for 5 minutes followed by 30 cycles of 95 °C for 30 seconds, 50 °C for 30 seconds, and 72 °C 1 minute. Then hold at 72 °C for 7 minutes and stopped by holding at 4 °C. After PCR, 5 µL of each PCR product, resulting from the DNA extracted from the M17p spiked stool samples, was analyzed on an agarose gel.

### RESULTS

### The results are presented in Figures 8-13.

PCR amplicons of expected size were detected in all three sets of primers used, in two replicate PCR assays on DNAs extracted from the 3.3X10⁸ CFU/gram spiked stool sample (or the undiluted culture (10₀) spike), the 3.3X10⁷ CFU/gram spiked stool sample (or the 10⁻¹dilution spike), and the 3.3X10⁶ CFU/gram spiked stool sample (or the 10⁻² dilution spike). There was no amplification observed in the negative control (no-template control). The positive control generated an amplicon of expected size. There was no amplification observed in the no-spike stool DNA control. The PBS buffer control spiked with a 10⁻³ dilution, generated an amplicon with similar intensity as the amplicon generated from 10⁻³ spiked stool DNA sample, indicating that PCR reactions are not inhibited from the stool DNA samples.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims.

All publications, patents and patent applications mentioned in this specification are herein incorporated in their entirety by reference into the specification, to the same extent as if each individual publication, patent or patent application was specifically and individually indicated to be incorporated herein by reference. In addition, citation or identification of any reference in this application shall not be construed as an admission that such reference is available as prior art to the present invention. To the extent that section headings are used, they should not be construed as necessarily limiting.

## Claims

1. A method of identifying an M17 strain *of E. coli* in a human sample, the method comprising analyzing DNA extracted from the human sample for a presence or absence of at least one M 17 specific nucleic acid sequence of an M 17 nucleic acid sequence being selected from the group consisting of SEQ ID NOs: 33, 34, 31, 3, 30, 35 and 36 under experimental conditions, said at least one M17 specific nucleic acid sequence being distinguishable from non M17 nucleic acid sequences in said DNA under said experimental conditions, wherein a presence of said at least one M17 specific nucleic acid sequence is indicative of M 17 in the human sample.

2. A method of identifying an M17 strain of *E. coli* in a human biological sample, the method comprising analyzing products of an amplification reaction using DNA extracted from the human biological sample and a primer pair which amplifies an M17 specific nucleic acid sequence of an M17 nucleic acid sequence, wherein said primer pair is selected from the group consisting of SEQ ID NOs: 37 and 38; SEQ ID NO: 39 and 40; and SEQ ID NOs: 45 and 46, wherein a product of said amplification reaction is indicative of an M17 strain of *E.coli.*

3. The method of claims 1 or 2, further comprising quantifying an amount of M 17 in the sample.

4. The method of claim 1, wherein said analyzing is effected using at least one oligonucleotide being at least 13 bases which hybridizes to said M17 specific nucleic acid sequence to provide a detectable signal under said experimental conditions and which does not hybridize to said non M17 nucleic acid sequences to provide a detectable signal under said experimental conditions.

5. The method of any of claims 1 or 2, wherein said M17 strain of E.coli is *E.coli* M17p (M17 parent) - ATCC Deposit No. 202226 or *E.coli* M17SNAR - ATCC Deposit No. 7295.

6. The method of claims 1 or 2, wherein said biological sample comprises a fecal sample.

7. The method of claim 1, wherein said analyzing is effected using two oligonucleotides, each of said two oligonucleotides being at least 13 bases.

8. The method of claim 7, wherein said determining is effected by PCR analysis.

9. A primer pair which amplifies an M17 specific nucleic acid sequence of an M17 nucleic acid sequence being selected from the group consisting of SEQ ID NOs: 1-36 under experimental conditions and does not amplify a non-M17 specific nucleic acid sequence under said experimental conditions, each primer of the pair being at least 13 bases.

10. The primer pair of claim 9, wherein said M17 nucleic acid sequence is selected from the group consisting of SEQ ID NOs: 3, 30, 31, 33, 34, 35 and 36.

11. The primer pair of claim 9, wherein at least one of the primers of the pair hybridizes to a polynucleotide sequence which is unique to M17.

12. The primer pair of claim 9, wherein said at least one of the primers has a nucleotide sequence as set forth in SEQ ID NO: 37-40, 45, 46 and 62-573.

13. The primer pair of claim 9, wherein a first primer of the pair is as set forth in SEQ ID NO: 37 and a second primer of the pair is as set forth in SEQ ID NO: 38.

14. The primer pair of claim 9, wherein a first primer of the pair is as set forth in SEQ ID NO: 39 and a second primer of the pair is as set forth in SEQ ID NO: 40.

15. The primer pair of claim 9, wherein a first primer of the pair is as set forth in SEQ ID NO: 45 and a second primer of the pair is as set forth in SEQ ID NO: 46.
